(19) Europäisches Patentamt
European Patent Office
Office européen des brevets

(11) EP 4 252 781 A1

(12) **EUROPEAN PATENT APPLICATION**
published in accordance with Art. 153(4) EPC

(43) Date of publication:
**04.10.2023 Bulletin 2023/40**

(21) Application number: **21898733.7**

(22) Date of filing: **29.11.2021**

(51) International Patent Classification (IPC):
***A61K 47/54*** *(2017.01)* ***A61K 47/28*** *(2006.01)*
***A61K 47/14*** *(2017.01)* ***A61K 38/16*** *(2006.01)*
***A61K 9/00*** *(2006.01)* ***A61P 3/10*** *(2006.01)*

(52) Cooperative Patent Classification (CPC):
**A61K 9/00; A61K 38/16; A61K 47/14; A61K 47/28; A61K 47/54; A61P 3/10**

(86) International application number:
**PCT/KR2021/017802**

(87) International publication number:
**WO 2022/114908 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2020 KR 20200163362**

(71) Applicant: **D&D Pharmatech Inc.**
**Seongnam-si, Gyeonggi-do 13486 (KR)**

(72) Inventors:
- **JEON, Ok Cheol**
  **Seoul 08785 (KR)**
- **LIM, Sung Mook**
  **Seoul 06378 (KR)**
- **PARK, Eun Ji**
  **Seoul 06279 (KR)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

# (54) ORAL FORMULATION OF BIOLOGICALLY ACTIVE MATERIAL CONJUGATE HAVING BIOTIN MOIETY, FATTY ACID MOIETY, OR COMBINATION THEREOF COUPLED THERETO

(57) The present invention relates to an oral pharmaceutical composition comprising (i) a biologically active material conjugate in which a biological active material is conjugated with a biotin moiety, a fatty acid moiety, or a combination thereof, and (ii) an excipient, wherein the absorption rate of the biologically active material is remarkably increased, whereby conventional drugs difficult to orally administer, such as proteins or peptides, can be administered orally.

[FIG. 1]

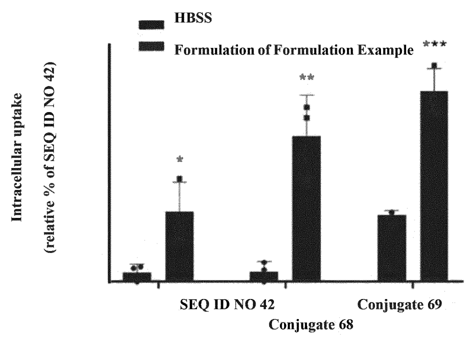


EP 4 252 781 A1

## Description

FIELD OF THE INVENTION

The present invention relates to an oral formulation of a physiologically active substance conjugate to which a biotin moiety, a fatty acid moiety, or a combination thereof is bound. More specifically, the present invention relates to an oral pharmaceutical formulation comprising (i) a physiologically active substance conjugate bound to a biotin moiety, a fatty acid moiety, or a combination thereof, and (ii) an excipient.

BACKGROUND OF THE INVENTION

In order for a drug to act effectively, high bioavailability must be ensured. Bioavailability refers to the degree of a drug used at a target site after drug administration, and the degree is different depending on the administration method, target environment, and the like. A drug may be lost or degraded in the course of delivery from the site of administration to the target, depending on the mode of administration.

Typically, drug delivery of therapeutic agents including proteins and polypeptides, etc. is divided into parenteral administration and oral administration. Parenteral administration methods include intravenous injection, intramuscular injection, subcutaneous injection, sublingual administration, etc., where oral administration method means ingestion of the drug orally. Most therapeutic agents, such as proteins and polypeptides, are administered by a parenteral method due to considerations of bioavailability, target environment and delivery process, etc. and it is known that parenteral administration method exhibits a direct and rapid effect. However, parenteral administration may cause pain or discomfort to the patient, and side effects such as infection by injection and air embolism may appear depending on the route. On the other hand, in the case of the oral administration method, there is an advantage in that it is convenient and the effect can be continuously displayed by the method of direct administration by mouth. Accordingly, many pharmaceutical companies have attempted to administer therapeutic agents by oral administration, but there is a problem in that [a drug administered by] oral administration passes through the digestive tract, so resistance to an acidic environment and enzymatic degradation, etc. is required. In particular, it is known that proteins and peptides have a low bioavailability of about 0.1% when administered orally.

In order to solve the problems of oral administration, attempts have been made to prepare separate [oral] formulations using surfactants and absorption enhancers, etc. together, or to increase the delivery of the drug by micronizing drug particles and adjusting the number of administrations. Such oral insulin and oral GLP-1 analogs are being developed by large pharmaceutical companies, and in addition, various research and development activities for oral administration of interferon alpha and the like are in progress. However, peptides and protein drugs are substances that are difficult to administer orally, and various attempts have been made to solve this problem, but it has not been clearly resolved so far. In particular, peptides and protein drugs have a problem in that the oral absorption rate is not high when administered orally, and this results in the problem of low pharmaceutical effect if not properly formulated.

SUMMARY OF THE INVENTION

FIELD OF THE INVENTION

The object of the present invention is to provide an oral pharmaceutical preparation by mixing a physiologically active substance conjugate, to which a biotin moiety, a fatty acid moiety, or a combination thereof is bound, and which has an outstanding oral absorption rate, with an excipient. More specifically, the object of the present invention is to efficiently increase the absorption rate of a physiologically active substance in the body through an oral formulation comprising: a physiologically active substance conjugate to which a biotin moiety and a fatty acid moiety are bonded; and an excipient.

[Technical Solution]

To achieve the object stated above, the present invention provides an oral pharmaceutical formulation comprising (i) a physiologically active substance conjugate bound to a biotin moiety, a fatty acid moiety, or a combination thereof, and (ii) an excipient.

Another aspect of the present invention provides an oral pharmaceutical formulation comprising: a physiologically active substance conjugate bound to a biotin moiety and a fatty acid moiety; and an excipient.

In one embodiment of the present invention, examples of the excipient may include bile acid, a derivative thereof, or a pharmaceutically acceptable salt thereof.

Further, in one embodiment of the present invention, the bile acid is at least one selected from the group

comprising glycocholic acid, glycochenodeoxycholic acid, taurochenodeoxycholic acid, taurocholic acid, deoxycholic acid, cholic acid, chenodeoxycholic acid, urso deoxycholic acid and lithocholic acid.

Further, one embodiment of the present invention may further comprise at least one selected from the group comprising alpha-tocopherol, malic acid, fumaric acid, ascorbic acid, butylated hydroxyanisole, butylated hydroxy toluene, sodium phosphate, calcium phosphate, potassium phosphate, galactose, glucose, maltose, gallic acid, propyl gallate, and pharmaceutically acceptable salts thereof.

EFFECTS OF THE INVENTION

The present invention, by comprising a physiologically active substance conjugate bound to a biotin moiety, a fatty acid moiety, or a combination thereof, and an excipient, provides the benefit of substantially increased absorption rates in the body. Specifically, the present invention, by comprising an excipient to improve enzyme stability, provides the benefit of substantially increased absorption rates in the body.

[Brief Description of the Drawings]

FIG. 1 is a diagram showing the results of measuring the accumulation, in Caco-2 cells, of hGH and oral formulations comprising conjugates 68 and 69 according to an embodiment of the present invention.
FIG. 2 is a diagram showing the results of measuring the blood glucose regulating ability of insulin and an oral formulation comprising conjugate 65 according to an embodiment of the present invention.
FIG. 3 is a diagram showing the results of measuring the blood glucose regulating ability of insulin and oral formulations comprising conjugates 65 and 66 according to an embodiment of the present invention.
FIG. 4 is a diagram showing the results of measuring the weight loss and feed intake reduction effects of amylin and oral formulations comprising conjugates 33, 36, 39, 42, 61, 62, 63 or 64 according to an embodiment of the present invention.
FIG. 5 is a diagram showing the results of measuring the blood glucose regulating ability of an oral formulation comprising conjugate 52 according to an embodiment of the present invention.

[Best Mode for Carrying Out the Invention]

The present invention relates to an oral pharmaceutical formulation comprising (i) a physiologically active substance conjugate to which a biotin moiety and a fatty acid moiety are bound, and (ii) bile acid, propyl gallate or a combination thereof.

[Mode for Carrying Out the Invention]

Hereinafter, embodiments and examples of the present invention will be described in detail so that those skilled in the art to which the present invention belongs can readily carry out the present invention.

However, the present invention may be embodied in many different forms and is not limited to the embodiments and examples described herein. Throughout the specification of the present invention, when a part "includes" a certain component, it means that other components may be further included, rather than excluding other components, unless otherwise stated.

The terms "about", "substantially", etc., to the extent used throughout the specification of the present invention, are used to refer to values equal to or close to the numerical values inherent to the manufacturing and material tolerances stated, and are used to aid in understanding the present invention or prevent an unconscionable infringer from unfair use of the disclosure. The term "step of -(doing) " or "step of" as used throughout the specification of the present invention does not mean "step for ~".

Throughout the specification of the present invention, the term "combination thereof' included in Markush type expressions refers to a mixture or combination of at least one selected from a group comprising the component elements stated in the Markush type expression, and means that at least one selected from a group comprising the components elements is included. Throughout the specification of the present invention, the statement "and/or B" means "and B, or A or B."

The present invention relates to an oral pharmaceutical formulation comprising (i) a physiologically active substance conjugate bound to a biotin moiety, a fatty acid moiety, or a combination thereof, and (ii) an excipient.

In one aspect of the present invention, (i) is a bioactive substance conjugate in which a biotin moiety and a fatty acid moiety are linked.

In one embodiment of the present invention, the excipient is bile acid, a derivative thereof, or a pharmaceutically acceptable salt thereof.

Further, in one embodiment of the present invention, the bile acid is at least one selected from the group comprising glycocholic acid, glycochenodeoxycholic acid, taurochenodeoxycholic acid, taurocholic acid, deoxycholic acid, cholic acid, chenodeoxycholic acid, urso deoxycholic acid and lithocholic acid.

Further, one embodiment of the present invention may further comprise at least one selected from the group comprising alpha-tocopherol, malic acid, fumaric acid, ascorbic acid, butylated hydroxyanisole, butylated hydroxy toluene, sodium phosphate, calcium phosphate, potassium phosphate, galactose, glucose, maltose, gallic acid, propyl gallate, and pharmaceutically acceptable salts thereof.

Typically, peptide and protein drugs correspond to Class 3 of the Biopharmaceutical Classification System (BCS), being highly water soluble and having restrictions on absorption sites in the gastrointestinal tract. Peptide and protein drugs have high hydrophilicity and large molecular weight, can be degraded by gastric acid of low pH, and have low intestinal absorption rate due to attack by enzymes such as trypsin. Typically, the oral bioavailability (BA) of peptide and protein drugs is about 0.1%, making it difficult to use them as pharmaceutical formulations. In order to address this problem, a technique of passing through the stomach using an enteric capsule is used, but this method is limited in that the absorption rate of peptides and proteins cannot be fundamentally improved.

In contrast, the physiologically active substance conjugate bonded to a biotin moiety, fatty acid moiety or combination thereof according to one embodiment of the present invention is able to promote absorption in the intestines by increasing intestinal membrane permeation.

Further, the physiologically active substance conjugate bonded to a biotin moiety, fatty acid moiety or combination thereof according to one embodiment of the present invention is able to exhibit outstanding pharmacokinetic effects.

Further, the physiologically active substance conjugate bonded to a biotin moiety, fatty acid moiety or combination thereof according to one embodiment of the present invention is able to protect against degradation of a physiologically active substance such as a peptide by enzymes, and is able to ultimately promote the permeation of the intestinal membrane by a physiologically active substance and its absorption in the intestine.

Further, the physiologically active substance conjugate bonded to a biotin moiety, fatty acid moiety or combination thereof according to one embodiment of the present invention, by being bonded to biotin, which is a type of water soluble vitamin, can be absorbed by active transport through a sodium-dependent multivitamin transporter.

Further, the biotin moiety, fatty acid moiety or combination thereof according to one embodiment of the present invention may be bonded to an active site or an inactive site of the physiologically active substance, and thus does not inhibit the activity of the physiologically active substance.

Further, more specifically, by preparing an oral formulation by mixing the physiologically active substance conjugate bonded to a biotin moiety, fatty acid moiety or combination thereof with an excipient, the absorption rate of a peptide or protein can be further improved.

In the present invention, "unsubstituted or substituted" refers to unsubstituted or substituted. "Substituted" refers to having one or more substituents, and a substituent refers to a chemical moiety that is covalently bonded or fused to any atom of a main group such as alkylene or heteroalkylene.

In the present invention, "halo" refers to fluorine, chlorine, bromine, iodine, and the like.

In the present invention, "alkyl" refers to a monovalent moiety obtained by removing a hydrogen atom from a carbon atom of an aliphatic or alicyclic, saturated or unsaturated hydrocarbon compound, for example, methyl, ethyl, propyl, butyl, pentyl, hexyl, n-propyl, n-butyl, n-pentyl, isopropyl, isobutyl, sec-butyl, tert-butyl, isopentyl, neopentyl and the like.

In the present invention, "heteroalkyl" is an alkyl containing one or more heteroatoms, and the heteroatom is a heteroatom positioned at any one carbon atom of the alkyl to replace C, CH, $CH_2$ or $CH_3$.

In the present invention, "alkylene" refers to a divalent moiety obtained by removing a hydrogen atom from a carbon atom of an aliphatic or alicyclic, saturated or unsaturated hydrocarbon compound.

In the present invention, "heteroalkylene" refers to an alkylene containing one or more hetero atoms.

In the present invention, "aryl" refers to a monovalent moiety obtained by removing a hydrogen atom from an aromatic ring atom of an aromatic compound having a ring atom. For example, "$C_{5-10}$aryl" refers to a monovalent moiety obtained by removing a hydrogen atom from an aromatic ring atom of an aromatic compound having 5 to 10 ring atoms of carbon. Examples of aryl include groups derived from benzene, acenaphthene, fluorene, phenalene, acephenanthrene and aceanthrene.

In the present invention, "heteroaryl" is an aryl containing one or more heteroatoms, for example, pyridine, pyrimidine, benzothiophene, furyl, dioxalanyl, pyrrolyl, oxazolyl, pyridyl, pyridazinyl, pyrimidinyl, isobenzofuran, indole, isoindole, indolizine, indoline, isoindoline, purine, benzodioxane, quinoline, isoquinoline, quinolizine, benzoxazine, benzodiazine, pyridopyridine, quinoxaline, quinazoline, cinnoline, phthalazine, naphthyridine, pteridine, perimidine, pyridoindole, oxantrene, phenoxatiin, phenazine, phenoxazine, and the like.

In the present invention, "arylene" refers to a divalent moiety obtained by removing a hydrogen atom from an

aromatic ring atom of an aromatic compound having a ring atom.

In the present invention, "heteroarylene" refers to arylene containing one or more heteroatoms.

In the present invention, "alkenyl" is an alkyl having one or more carbon-carbon double bonds, for example, vinyl ($-CH=CH_2$), 1-propenyl ($-CH=CHCH_3$), isopropenyl, tenyl, pentenyl, and hexenyl.

In the present invention, "alkynyl" is an alkyl group having one or more carbon-carbon triple bonds, and examples thereof include ethynyl and 2-propynyl.

In the present invention, when a part of the general formula is defined as a specific compound, it includes forms in which the compound is combined with other components.

According to one embodiment of the present invention, the biotin moiety may be represented by General Formula A below.

[General Formula A]

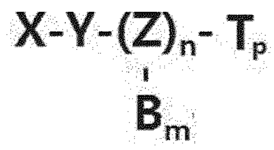

Where, in General Formula A

X is a functional group capable of binding to a physiologically active substance;
Y is a spacer;
Z is a binding unit;
B may be represented by the following Chemical Formula A-1;

[Chemical Formula A-1]

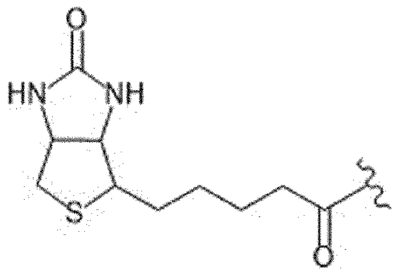

Z is connected to 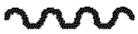 of Chemical Formula A-1,
T is a terminal group,
m is an integer of 1 to 10;
n is an integer of 0 or 1 to 10, where, when n=0, Y bonds directly with B or T; and,
p is an integer of 0 or 1.

According to one embodiment of the present invention, in General Formula A, X is a functional group capable of binding to a physiologically active substance. Although not limited thereto, the functional group is a functional group capable of reacting with a thiol group, a carboxyl group and/or an amine group, for example, maleimide, succinimide, N-hydroxysuccinimide, aldehyde, carboxyl group, carboxyl ester, succinimidyl ester, tetrafluorophenyl, -O-tetrafluorophenyl (TFP,2,3,5,6-tetrafluorophenyl), tetrafluorophenyl ester, pentafluorophenyl (PFP), pentafluorophenyl ester, -O-benzotriazole, benzotriazole, sulfotetrafluorophenyl (STP), sulfodichlorophenyl (SDP), nitrophenol, and nitrophenyl carbonate (NPC).

In one embodiment of the present invention, the functional group X may retain its structure or may be eliminated or modified when bound to a physiologically active substance.

Y is a spacer and may have a structure having cleavability in the body. Without being limited thereto, for example, Y may be a direct bond, or may include a substituted or unsubstituted alkylene, -O-, -C(O)NR-, -C(O)O- or -C(O) -, -NR-, -NOR-, or the like. More specifically, Y may be a direct bond, or the structure of Y may include at least one of a group

comprising substituted or unsubstituted $C_{1-50}$ linear alkylene, substituted or unsubstituted $C_{1-50}$ nonlinear alkylene, substituted or unsubstituted $C_{1-50}$ Linear heteroalkylene, substituted or unsubstituted $C_{1-50}$ Non-linear heteroalkylene, substituted or unsubstituted $C_{1-50}$ arylene, substituted or unsubstituted $C_{1-50}$ heteroarylene, -O-, -C(O), -C(O)NR-, -C(O)O-, -S-, -NR- or - NOR-, wherein R is hydrogen, or substituted or unsubstituted $C_{1-50}$ alkyl, substituted or unsubstituted $C_{1-50}$ aryl, or an ethylene glycol repeating unit ($-(CH_2CH_2O)_n-$, where n is an integer of at least 1 but not more than 20).

Z is a binding unit capable of bonding with B, and may include, for example, but is not limited to, an amino acid, polypeptide, alkylene, amine, or polyamidoamine structure.

Non-limiting examples of the amino acid may include lysine, 5-hydroxylysine, 4-oxalicine, 4-thialysine, 4-selenalysine, 4-thiahomolysine, 5,5-dimethyllysine, 5,5-difluorolysine, trans-4-dihydrolysine (trans-4-dehydrolysine), 2,6-diamino-4-hexinoic acid, cis-4-dihydrolysine (cis-4-dehydrolysine), 6-N-methyllysine, diaminopimelic acid, ornithine, 3-methylomithine, $\alpha$-methylornithine, citrulline, homocitrulline, arginine, aspartate, asparagine, glutamate, glutamine, histidine, ornithine, proline, serine, threonine, and the like.

In the present invention, when n is 0, B or T may be bonded directly with X or Y (spacer).

In the present invention, T is a terminal group, and may be hydrogen or $NH_2$, but is not limited hereto.

In the present invention, when p is 0, B may be a terminal.

In the present invention, X-Y may together form a physiologically active substance binding site.

According to an embodiment of the present invention, in General Formula A, m may be an integer of 1 to 10, and specifically may be an integer or 1 to 8, 1 to 5, or 1 to 4.

In one embodiment of the present invention, X may be selected from the group comprising maleimide, succinimide, N-hydroxysuccinimide, succinimidyl succinate, succinimidyl glutarate, succinimidyl methyl ester, succinimidyl pentyl ester, Succinimidyl carbonate, p-nitrophenyl carbonate, aldehyde, amine, thiol, oxyamine, iodoacetamide, aminooxyl, hydrazide, hydroxy, propionate, pyridyl, alkyl halide, vinyl sulfone, carboxyl, hydrazide, halogen acetamide, $C_{2-5}$ alkynyls, $C_{6-20}$ aryldisulfides, $C_{5-20}$ heteroaryldisulfide, isocyanate, thioester, iminoester, and derivatives thereof.

In a specific embodiment of the present invention, X is maleimide, N-hydroxysuccinimide, succinimidyl carbonate, p-nitrophenyl carbonate, thiol, aminooxyl, aldehyde or amine.

In a specific embodiment of the present invention, X is maleimide, N-hydroxysuccinimide, aldehyde or amine.

In one embodiment of the invention, Y is absent, or is a substituted or unsubstituted, linear or branched $C_{1-50}$ alkylene, substituted or unsubstituted, linear or branched $C_{1-50}$ heteroalkylene, substituted or unsubstituted, $C_{6-50}$ arylene, or substituted or unsubstituted $C_{6-50}$ heteroarylene, where if substituted, comprises at least one selected from the group comprising =O, $-C(O)NH_2$, -OH, -COOH, -SH, =NH and $-NH_2$.

In one embodiment of the present invention, Y comprises -C(O)-.

In one embodiment of the present invention, Y comprises -C(O)NH-.

In one embodiment of the present invention, Y is a substituted linear or branched $C_{1-50}$ heteroalkylene, and comprises at least one -C(O)-.

In one embodiment of the present invention, Y is $-(C(O))_q-(CH_2)_r-(C(O)NH)_s-(CH_2)_r-(OCH_2CH_2)_t-(C (O))_q-$, wherein q, r, s, t are independently selected, q and s are 0 or 1, r is an integer of 1 to 20, and t is an integer of 0 to 20.

In one embodiment of the present invention, Y is $-(CH_2)_rC(O)NHNH-$, where r is an integer of 1 to 20.

In one embodiment of the present invention, Y comprises $-C(O)-(OCH_2CH_2)_u-NH-$ as a repeating unit, where u is an integer of 1 to 20.

In one embodiment of the present invention, Y comprises $-C(O)-(OCH_2CH_2)_u-NH-$ as a repeating unit, where u is an integer of 2 to 4.

In one embodiment of the present invention, Y comprises an amino acid as a component.

In a specific embodiment of the present invention, Y comprises glutamic acid, glutamine, glycine, isoleucine, or lysine as a component, where each amino acid may exist in bonded form.

In a specific embodiment of the present invention, Y comprises glutamic acid or lysine as a component.

In an embodiment of the present invention, Y comprises a fatty acid as a component.

In a specific embodiment of the present invention, Y comprises a $C_{12-24}$ fatty acid, and the fatty acid exists in a bonded form.

In one embodiment of the present invention, Y is a direct bond.

In one embodiment of the present invention, Z is any one of the following, each of which may be independently selected.

A) forms an amino acid or a derivative thereof together with X or separately from X;
B) is a substituted or unsubstituted linear or branched $C_{1-50}$ heteroalkylene;

where, if substituted, comprises at least one selected from the group comprising =O, $-C(O)NH_2$, -OH, -COOH, -SH, =NH, and $-NH_2$.

In one embodiment of the present invention, Z is linked to B through -NH-.
In one embodiment of the present invention, Z is a hydrophilic amino acid or a derivative thereof.
In a specific embodiment of the present invention, Z may be selected from the group comprising lysine, arginine, histidine, glutamine, asparagine, threonine, cysteine, serine and derivatives thereof.
In one embodiment of the present invention, Z comprises at least one glycerol, at least one polyethylene glycol, or a combination thereof.
It comprises

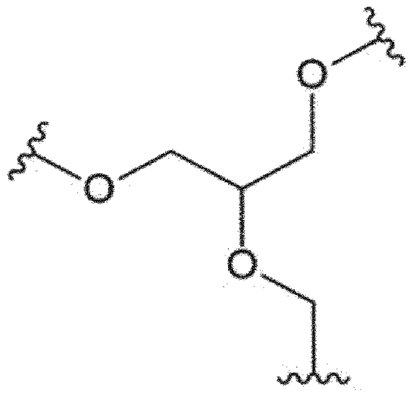
;

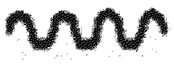 represents a binding site; and at least one

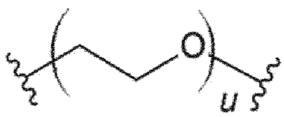

binds to at least one of the binding sites, where u is an integer of 1 to 20.
In one embodiment of the present invention, Z comprises

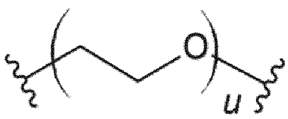
,

and $-(CH_2)_3NH-$ is further bonded to

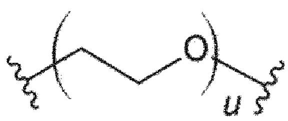
.

In one embodiment of the present invention, T may be selected from the group comprising amine, $C_{1-8}$ alkyl, $C_{1-8}$ alkenyl, halo, hydroxy, thiol, sulfonic acid, carboxyl, phenyl, benzyl, aldehyde, azide, cyanate, isocyanate, thiocyanate, isothiocyanate, nitrile and phosphonic acid.
In one specific aspect of the invention, T is an amine.
In one embodiment of the present invention, the biotin moiety is selected from the group comprising:

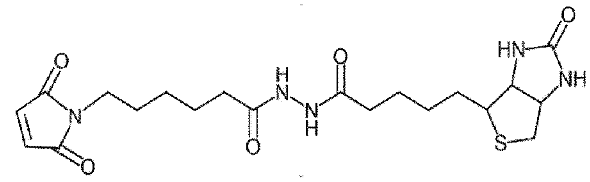

,

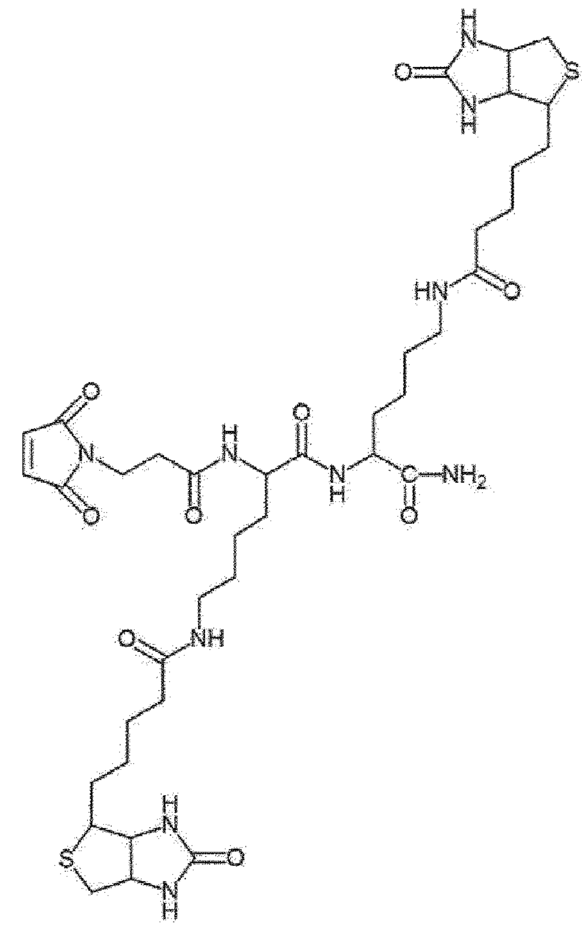

,

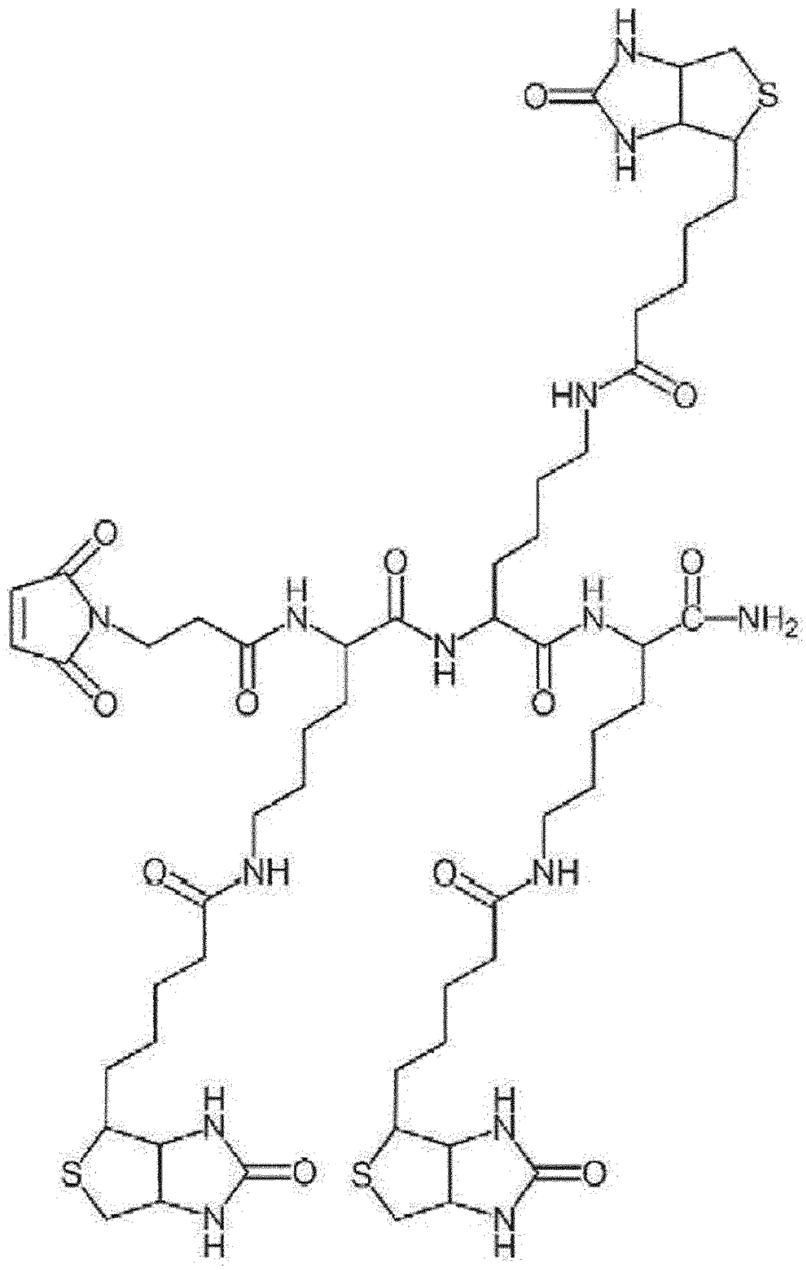

,

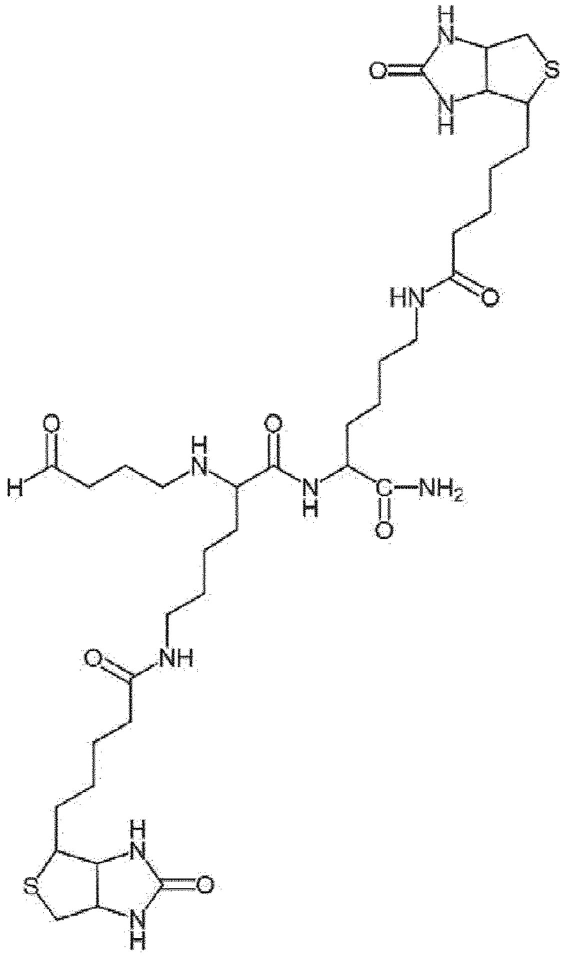

,

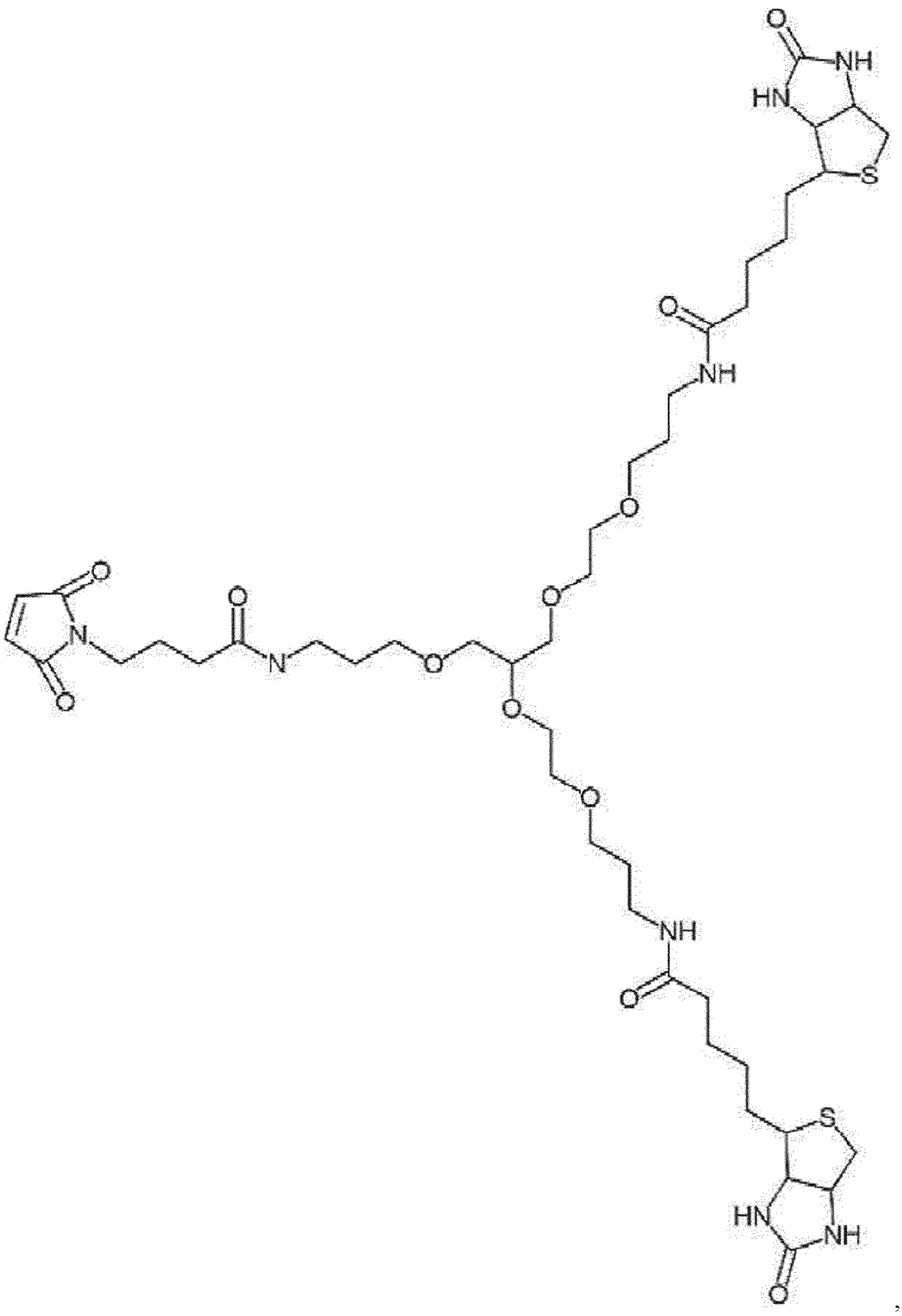

,

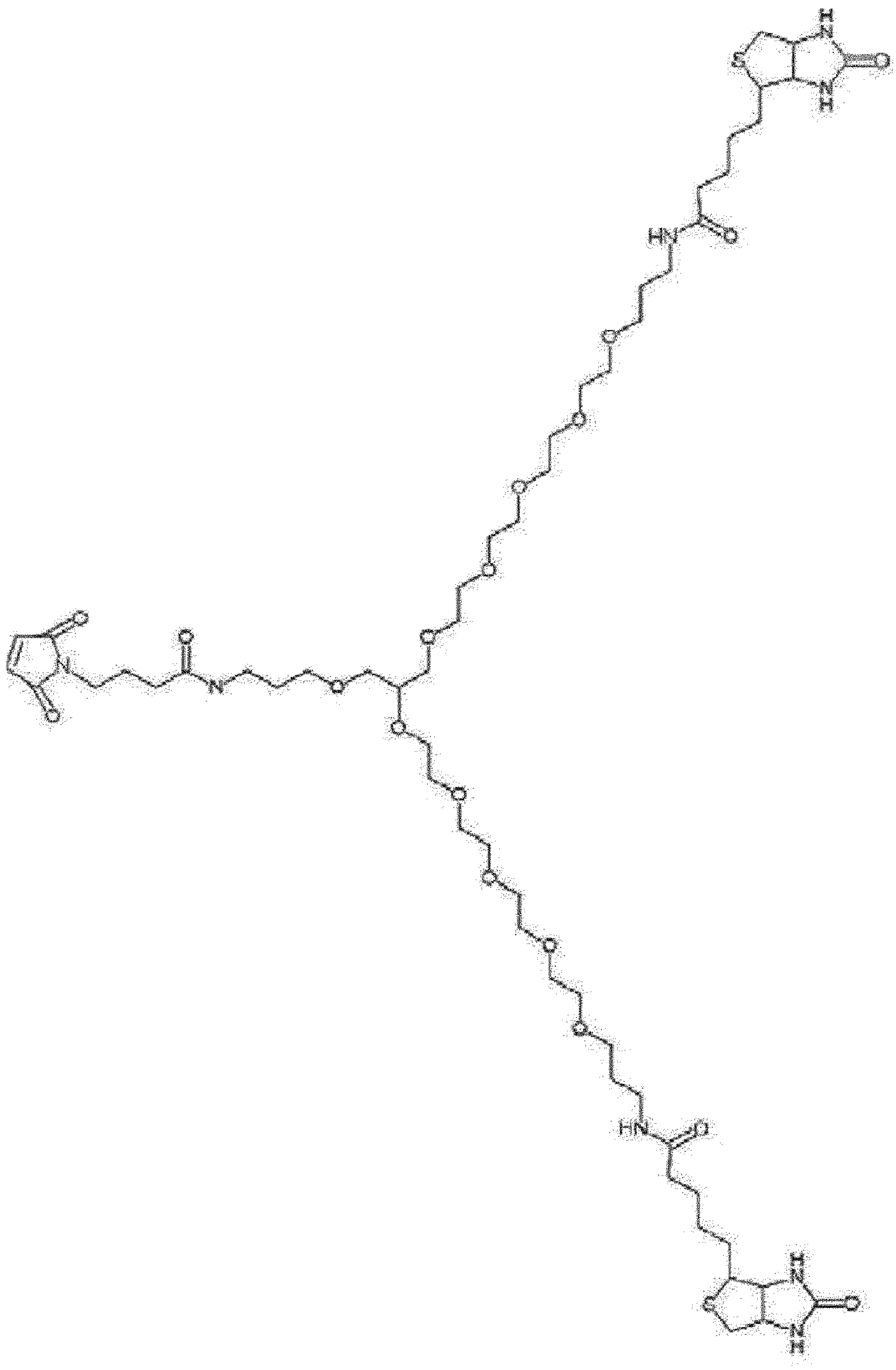

,

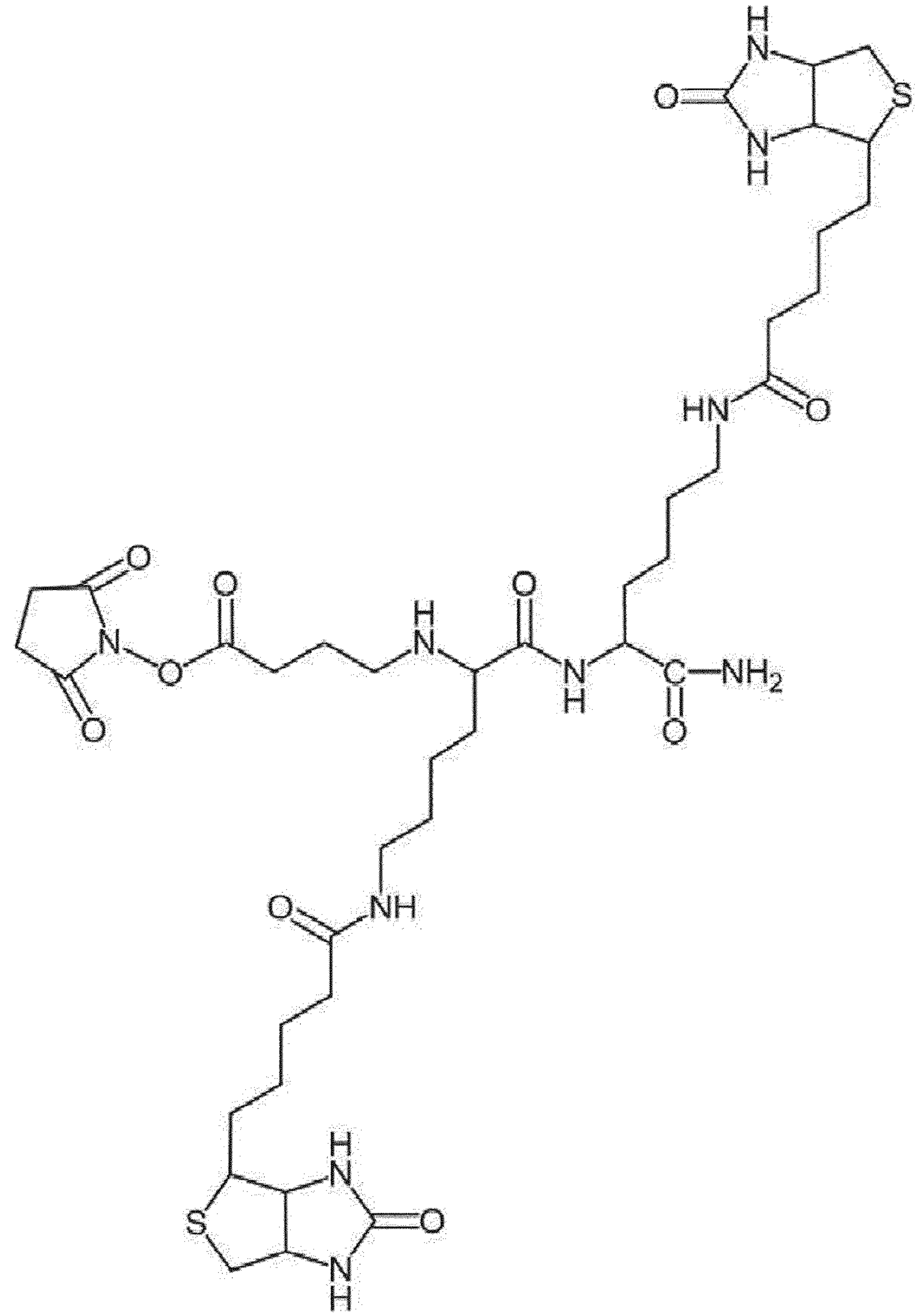

,

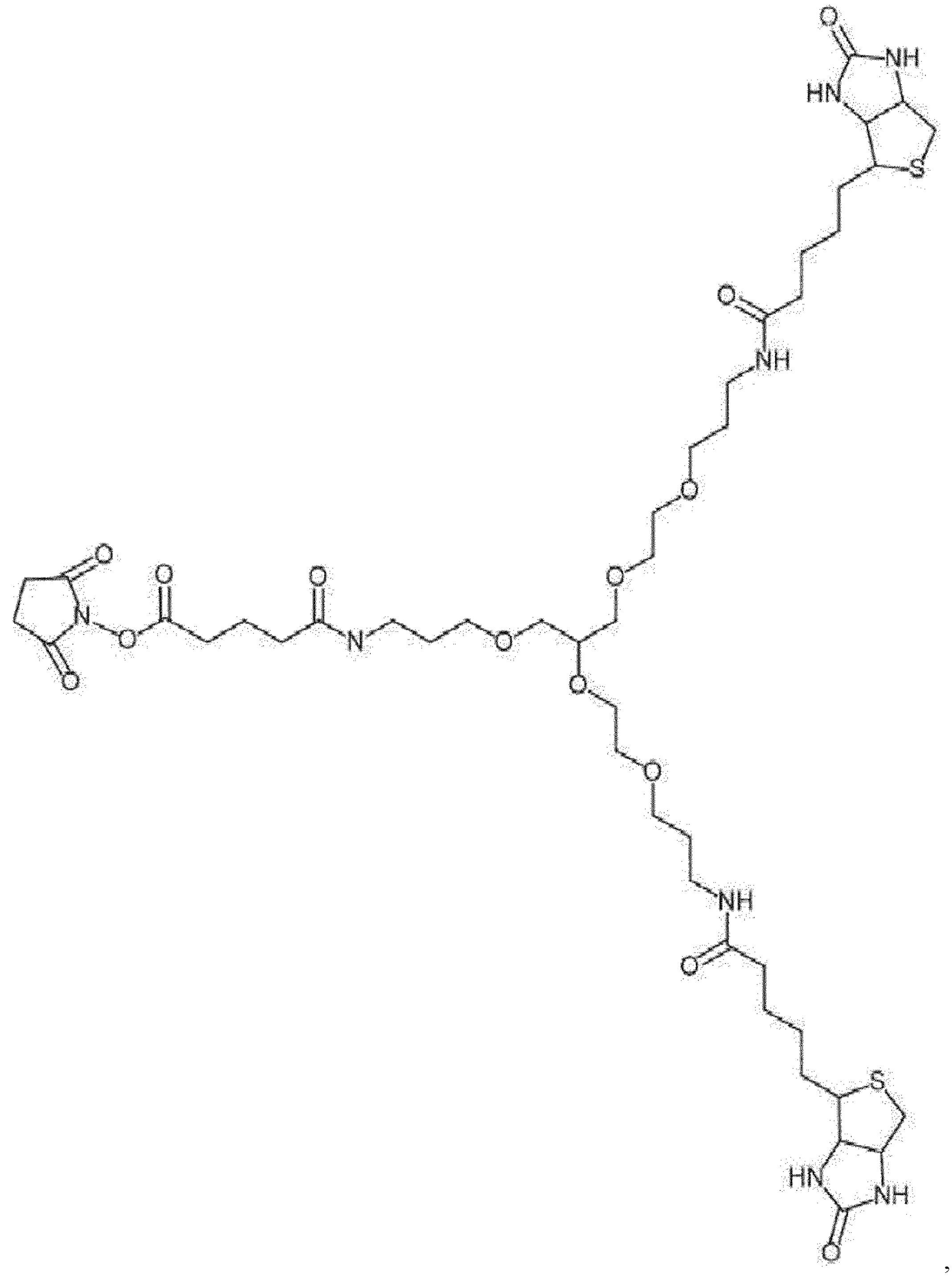

,

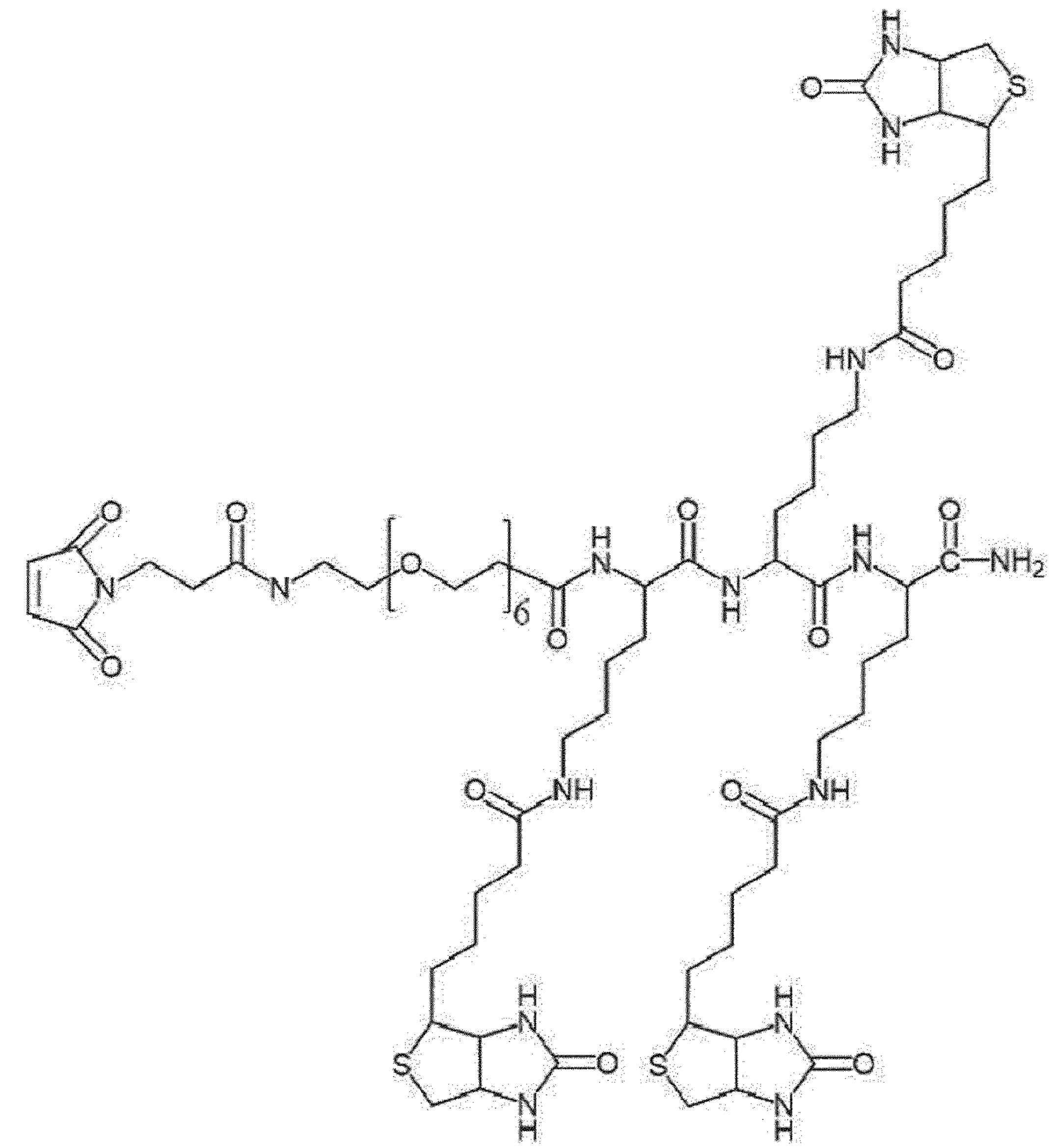

,

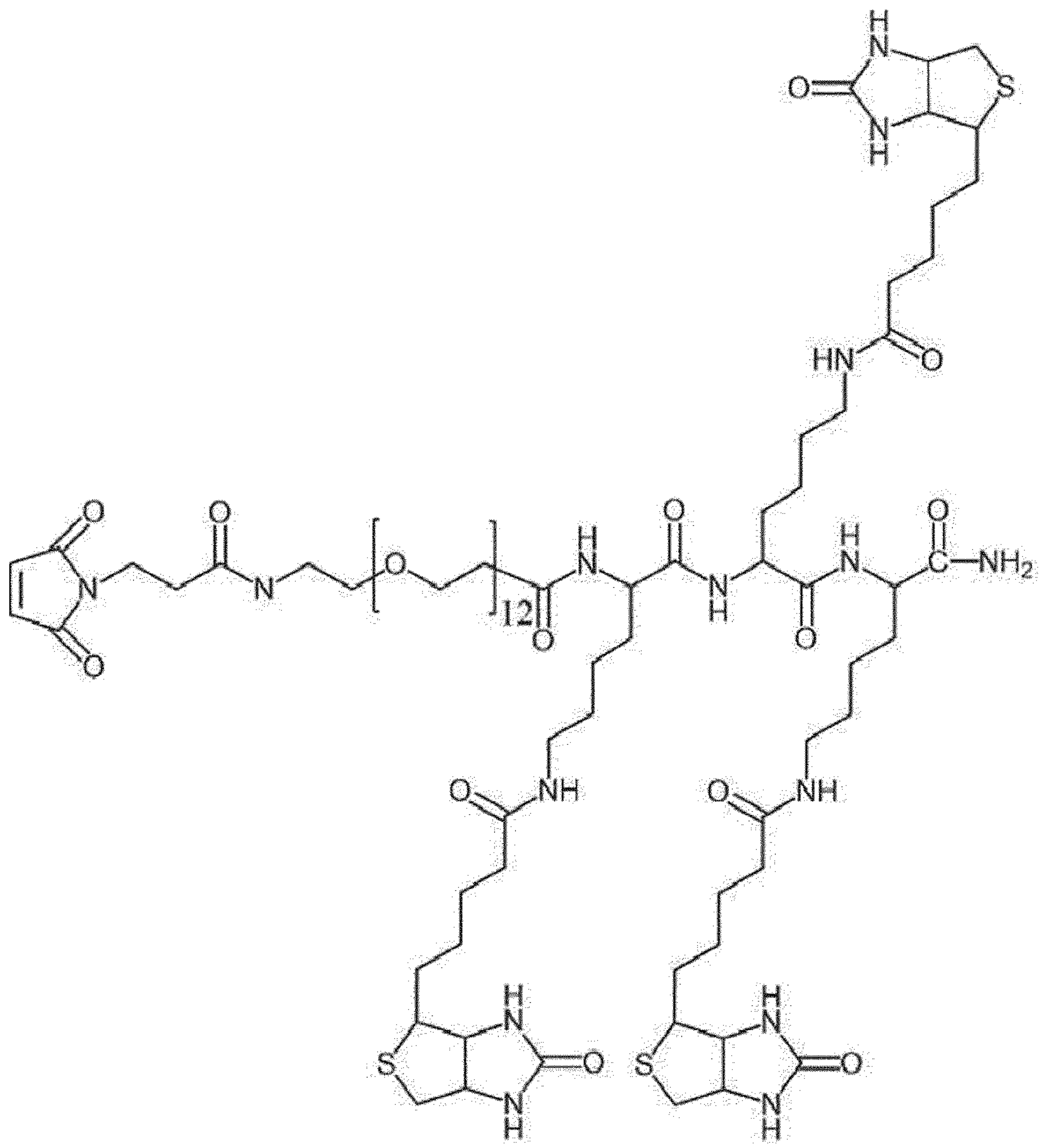

,

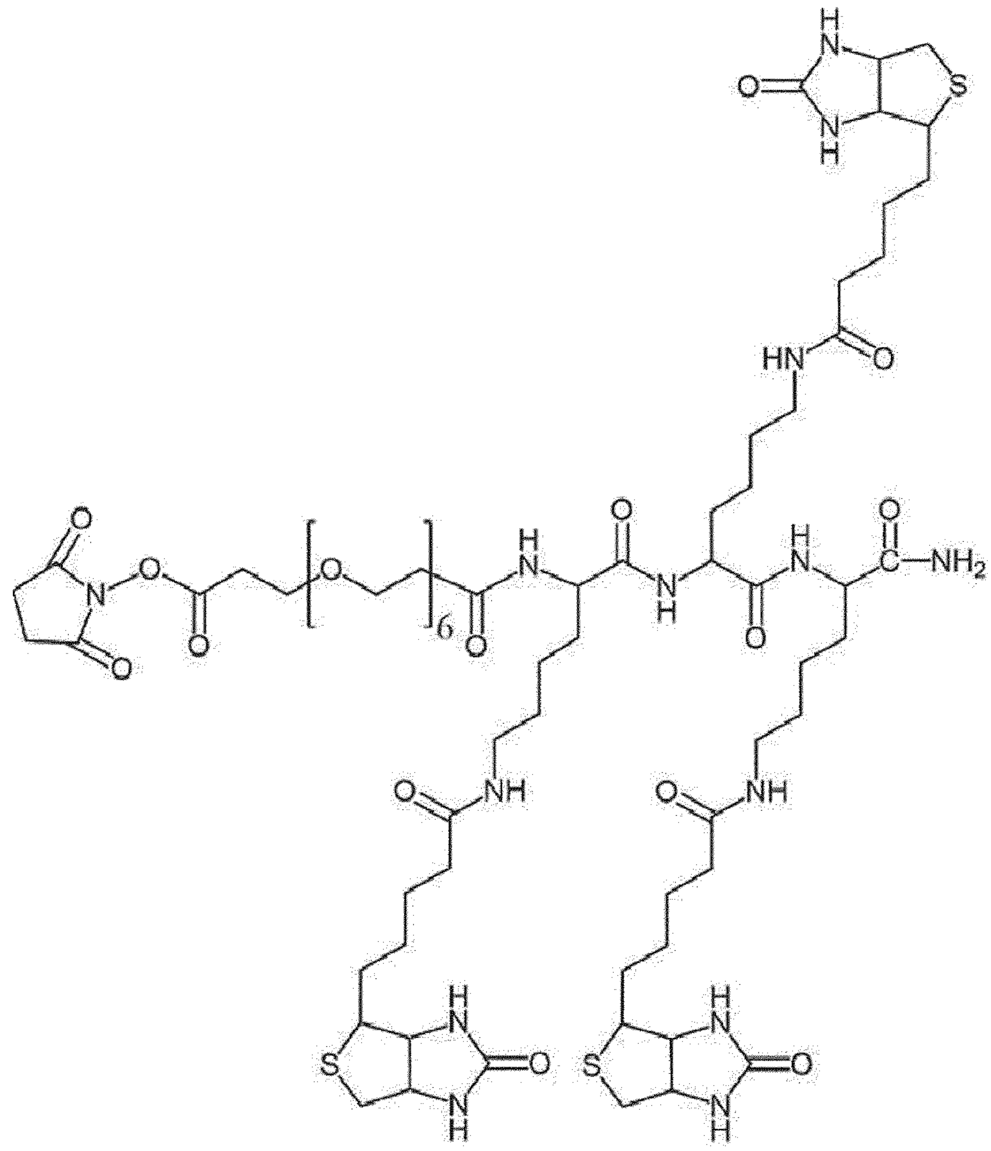

,

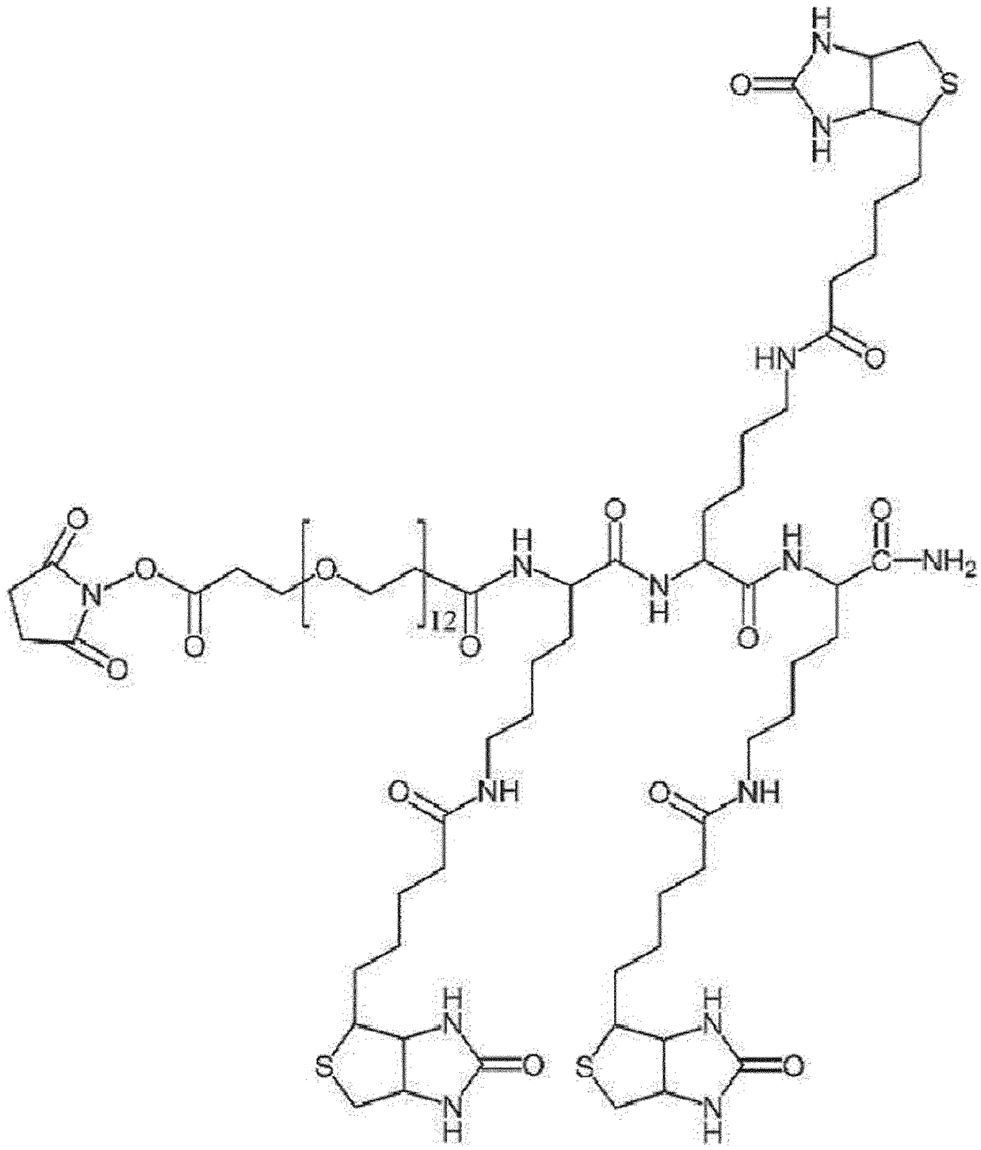

,

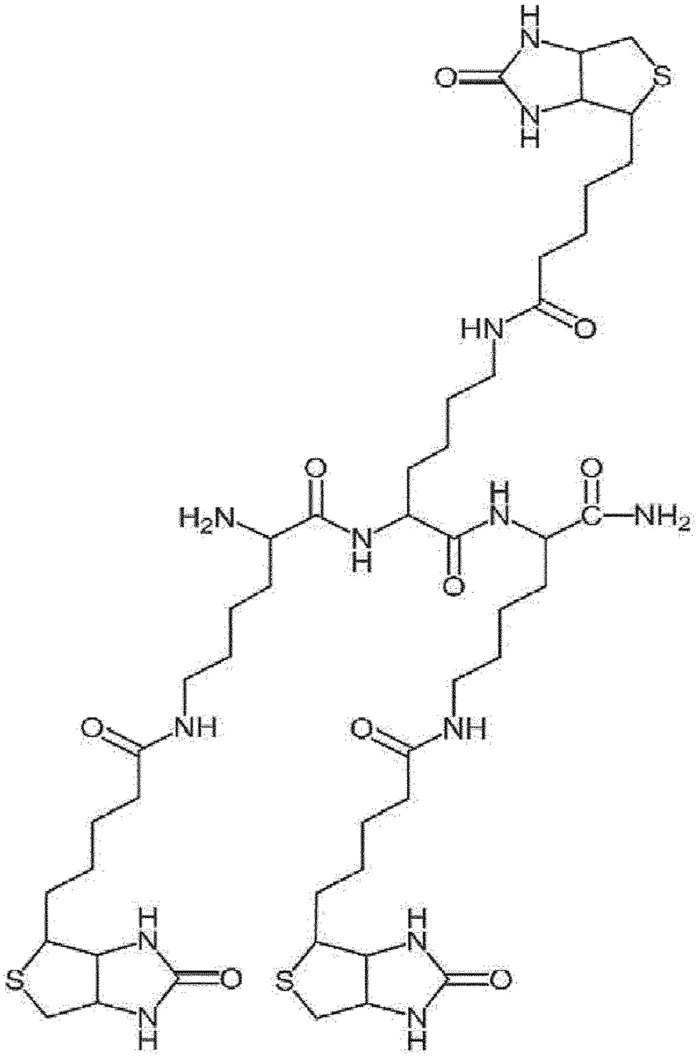

,

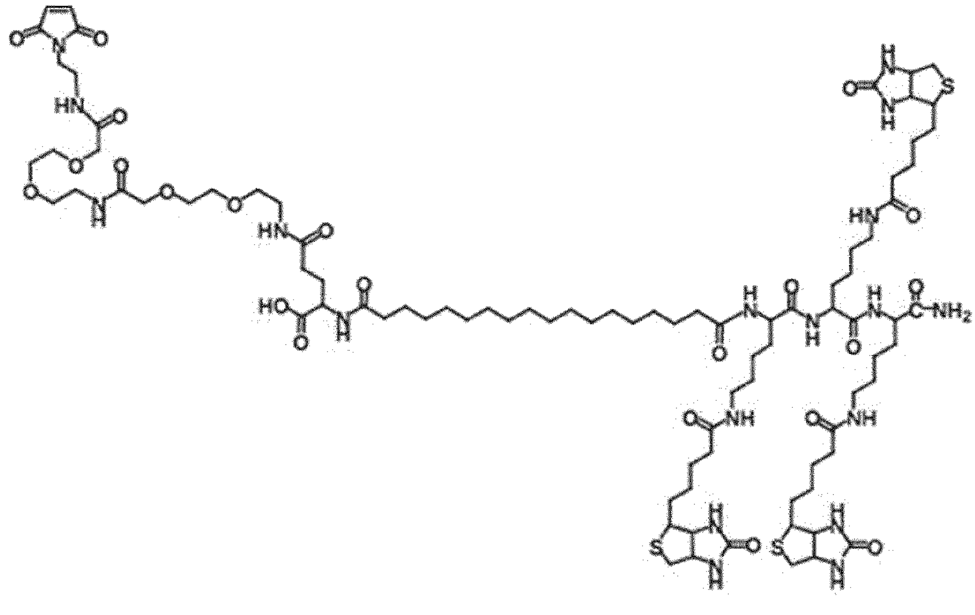

,

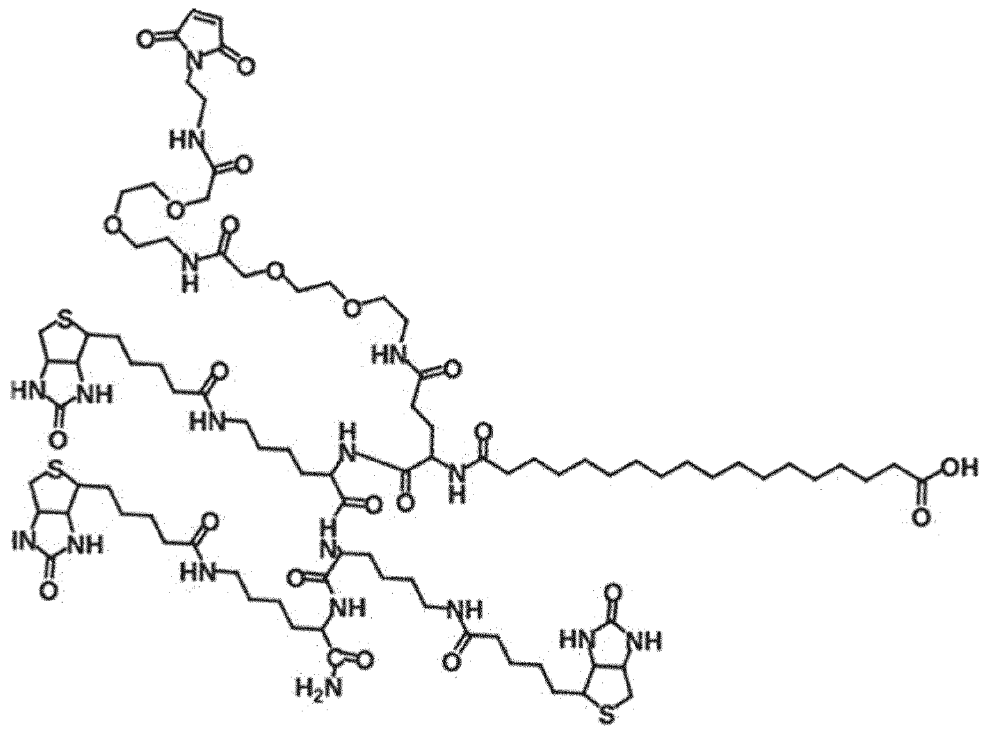

,

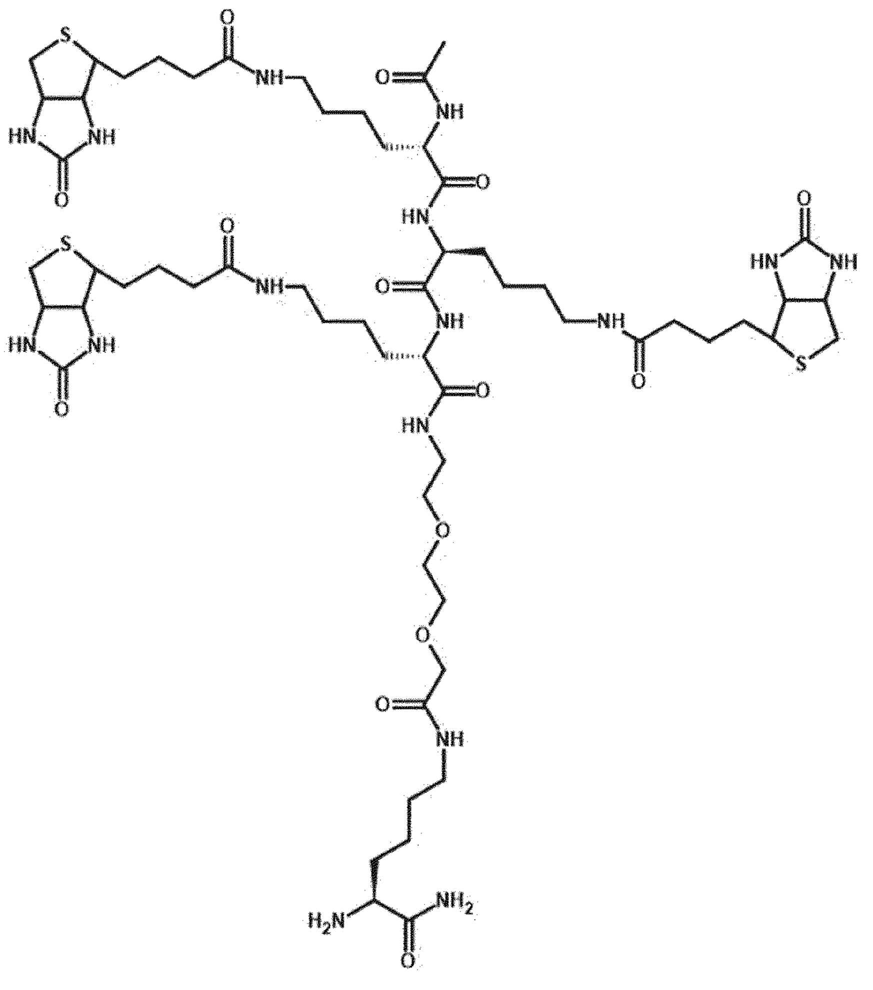

and

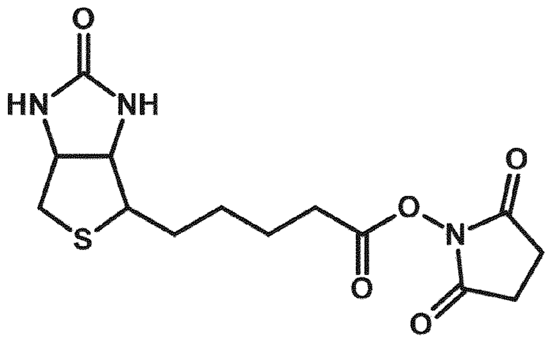

According to one embodiment of the present invention, the fatty acid moiety may be represented by General Formula B below:

[General Formula B] X'-Y'-W

Where, in the above formula,

X' is a functional group capable of binding to a the physiologically active substance;
Y' is a spacer; and
W is a fatty acid.

In the present specification, the fatty acid includes, carboxylic acid having a long saturated or unsaturated aliphatic chain, including, for example, but not limited to, caprylic acid, lauric acid, which is a type of saturated fatty acid, Palmitic acid, Stearic acid, Arachidic acid, Cerotic acid, Myristoleic acid, which is a kind of unsaturated fatty acid, Palmitoleic acid, oleic acid, linoleic acid, alpha-linolenic acid, and the like.

According to one embodiment of the present invention, in General Formula B, X' is a functional group capable of binding to a physiologically active substance. Here, X' is the same as X in the General Formula A. Accordingly, in one embodiment of the present invention, the functional group X' may retain its structure or may be eliminated or modified when bound to a physiologically active substance.

According to one embodiment of the present invention, in General Formula B, W may correspond to a fatty acid. Here, the fatty acid includes all types of fatty acids, including simple, modified, added, deleted and the like.

In one aspect of the present invention, Y' is the same as Y in the General Formula A above. Accordingly, in one embodiment of the present invention, the spacer Y' may be a direct bond, or may include at least one of the group comprising substituted or unsubstituted $C_{1-50}$ linear alkylene in the structure of Y, substituted or unsubstituted $C_{1-50}$ non-linear alkylene, substituted or unsubstituted $C_{1-50}$ linear heteroalkylene, substituted or unsubstituted $C_{1-50}$ nonlinear heteroalkylene, substituted or unsubstituted $C_{1-50}$ arylene, substituted or unsubstituted $C_{1-50}$ heteroarylene, -O-, -C(O), -C(O)NR-, -C(O)O-, -S-, -NR- or -NOR-, wherein R is hydrogen, or unsubstituted $C_{1-50}$ alkyl, substituted or unsubstituted $C_{1-50}$ aryl, or an ethylene glycol repeating unit ($-(CH_2CH_2O)_n-$, where n is an integer of at least 1 but not more than 20).

In one embodiment of the present invention, W is a substituted or unsubstituted linear or branched $C_{1-60}$ alkylene, substituted or unsubstituted linear or branched $C_{1-60}$ alkenylene, substituted or unsubstituted linear or branched $C_{1-60}$ heteroalkylene, or substituted or unsubstituted linear or branched $C_{1-60}$ heteroalkenylene, and if substituted, may be substituted by at least one selected from the group comprising =O, $-C(O)NH_2$, -OH, -COOH, -SH, =NH, $-NH_2$, and halo.

In one aspect of the present invention, W is one or more substituted $C_{12-24}$ alkylene or one or more substituted $C_{36-48}$ heteroalkylene, and when substituted, may include =O or -COOH.

In one embodiment of the present invention, W is a $C_{12-24}$ alkylene wherein at least one is substituted or a C36-48 heteroalkylene wherein at least one is substituted, and if substituted, may comprise =O or -COOH.

In one aspect of the present invention, W in which one or more is substituted is a substituted or unsubstituted C12-24 saturated fatty acid, and when substituted, includes -COOH.

In one embodiment of the present invention, the fatty acid moiety may have the chemical formula of General Formula B1 below:

[General Formula B1] X'i-Y'-C(O)-Fi

Where, in the above formula,

$X'_1$ is maleimide, N-hydroxysuccinimide, aldehyde, amine, tetrafluorophenyl ester or nitrophenol;
Y' is a spacer; and
$F_1$ is a $C_{6-28}$ substituted or unsubstituted linear or branched alkylene, or substituted or unsubstituted linear or branched heteroalkylene.

According to one embodiment of the present invention, in General Formula B-1, $X_1$ may be the same as X in General Formula A. Accordingly, in one embodiment of the present invention, the functional group $X_1$ may retain its structure or may be eliminated or modified when bound to a physiologically active substance.
Further, in General Formula B1, Y' may be the same as Y in General Formulae A and B.
In one embodiment of the present invention, Y' is a substituted or unsubstituted $C_{6-50}$ linear or branched heteroalkylene, and if substituted, comprises at least one selected from the group comprising =O, $-C(O)NH_2$, -OH, -COOH, -SH, =NH and $-NH_2$.
In one embodiment of the present invention, Y' may comprise $-(CH_2CH_2O)-$ as a repeating unit.
In one embodiment of the present invention, Y' comprises $-C(O)-(OCH_2CH_2)_u-NH-$ as a repeating unit, where u is an integer of 1 to 20.
In one specific embodiment of the present invention, Y' comprises $-C(O)-(OCH_2CH_2)_u-NH-$ as a repeating unit, where u is an integer of 2 to 4.
In one embodiment of the present invention, Y' comprises an amino acid or a derivative thereof as a component.
In a specific embodiment of the present invention, Y' comprises glutamic acid, glutamine, glycine, isoleucine, or lysine as a component, where each amino acid may exist in bonded form.
In a specific embodiment of the present invention, Y' comprises glutamic acid or lysine as a component.
In one embodiment of the present invention, $F_1$ may be a substituted or unsubstituted $C_{10-28}$ linear or branched alkylene.
In a specific embodiment of the present invention,, the W wherein at least one is substituted is a substituted or unsubstituted $C_{12-24}$ saturated fatty acid, and if substituted, comprises -COOH.
In a specific embodiment of the present invention, $F_1$ is $-(CH_2)_v-COOH$, where v is an integer of 10 to 20.
In a specific aspect of the present invention, $F_1$ is $-C(O)-(CH_2)_v-COOH$, and v is an integer from 10 to 20.
In a specific embodiment of the present invention, the fatty acid moiety may be selected from the group comprising:

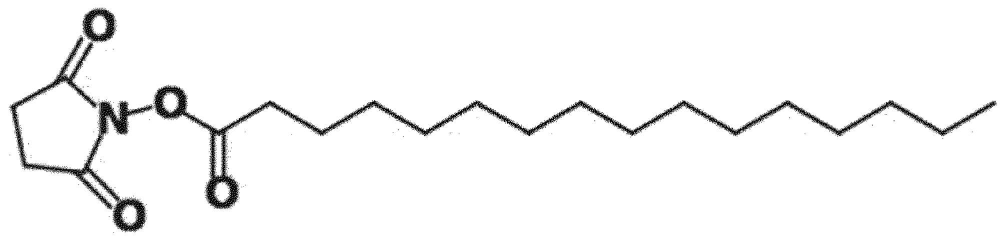

,

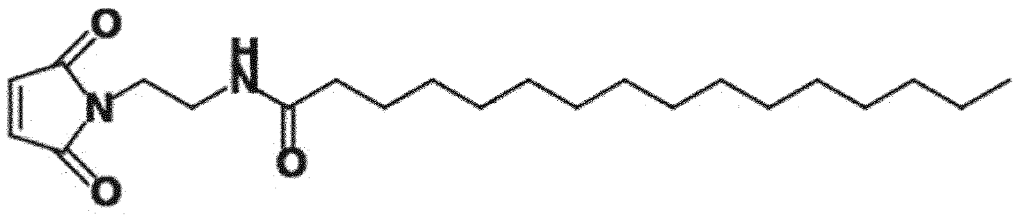

,

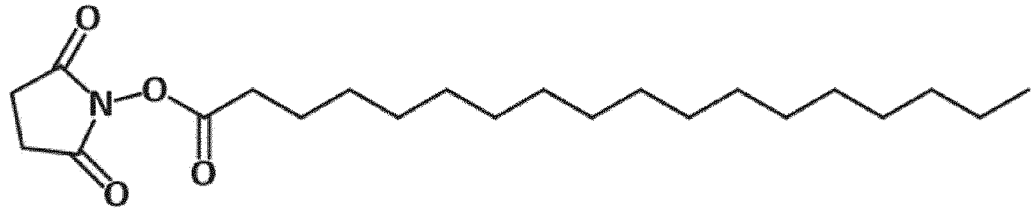

,

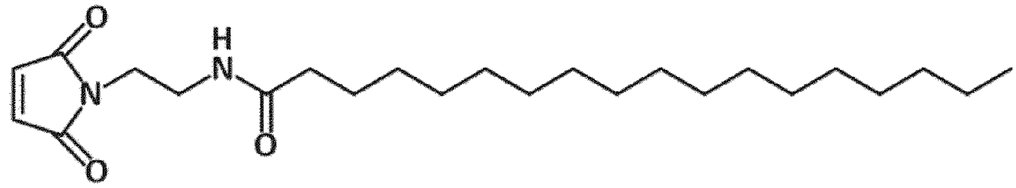

,

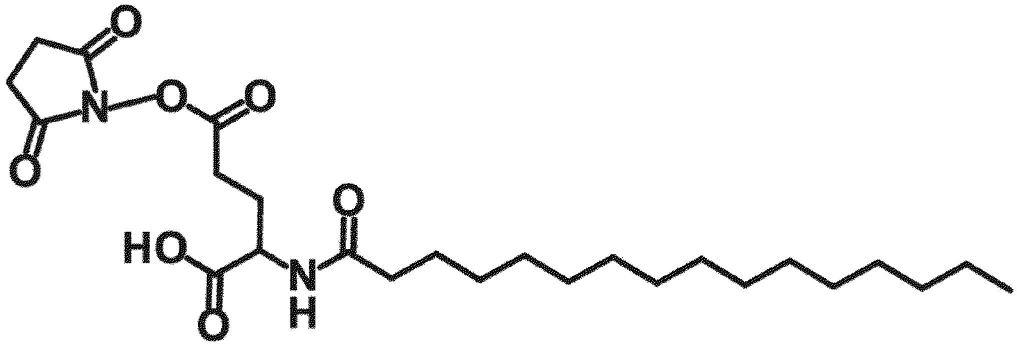

,

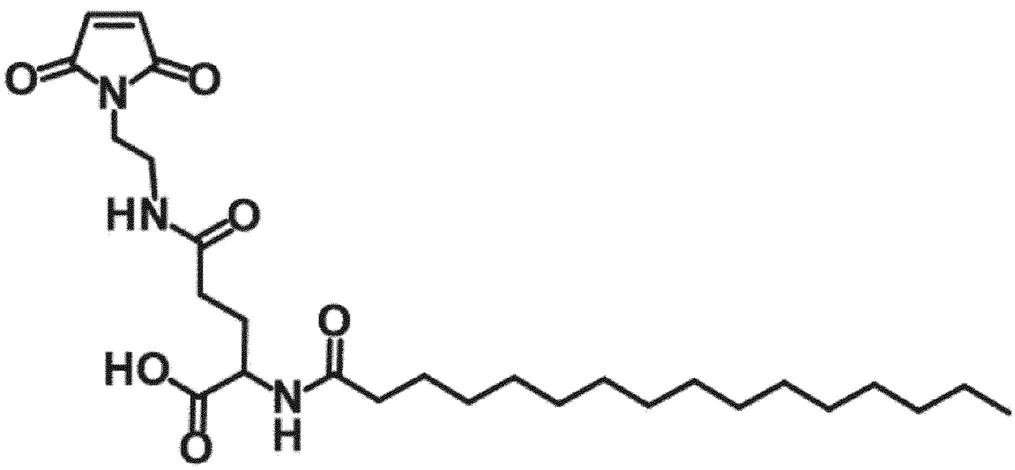

,

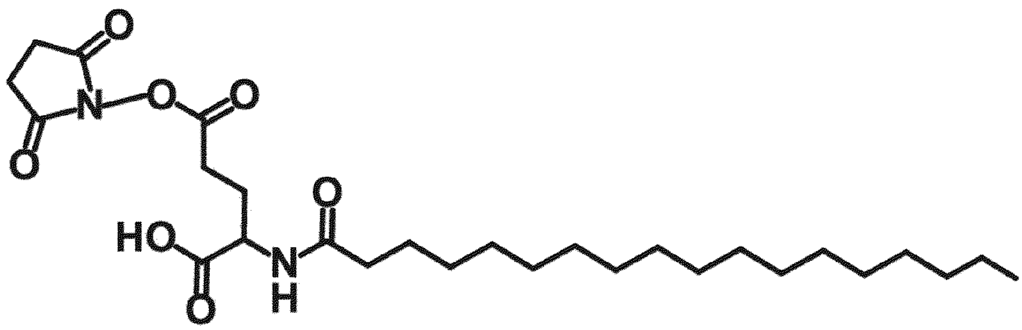

,

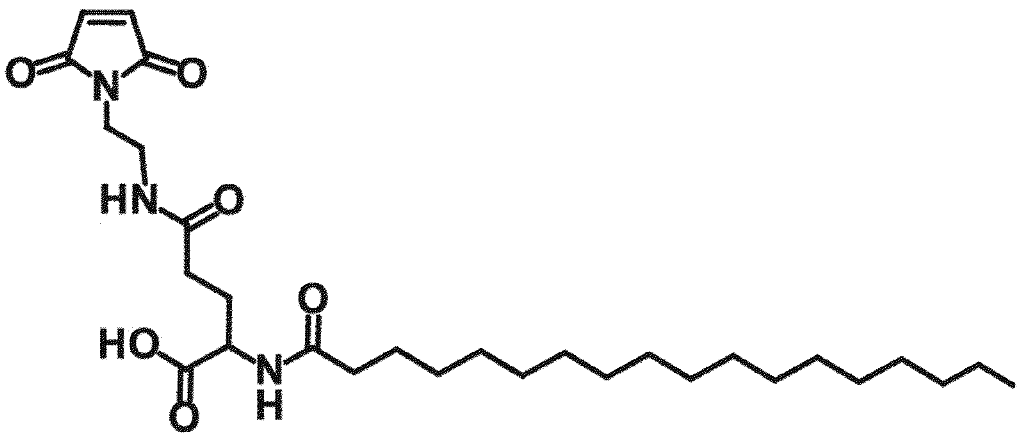

,

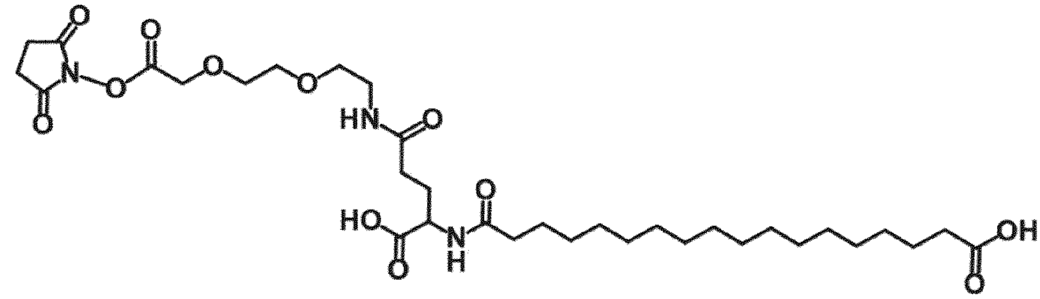

,

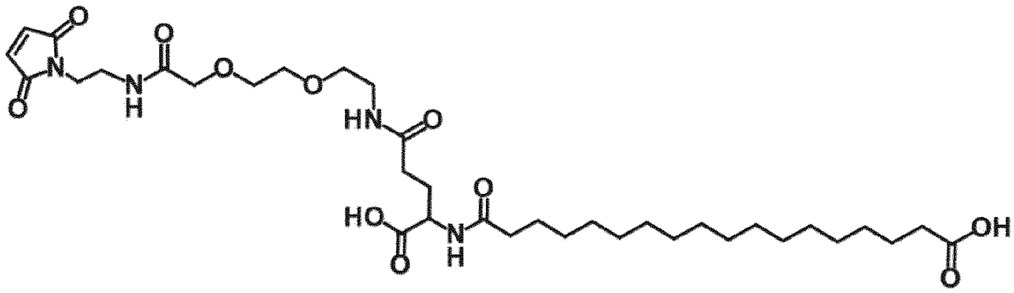

,

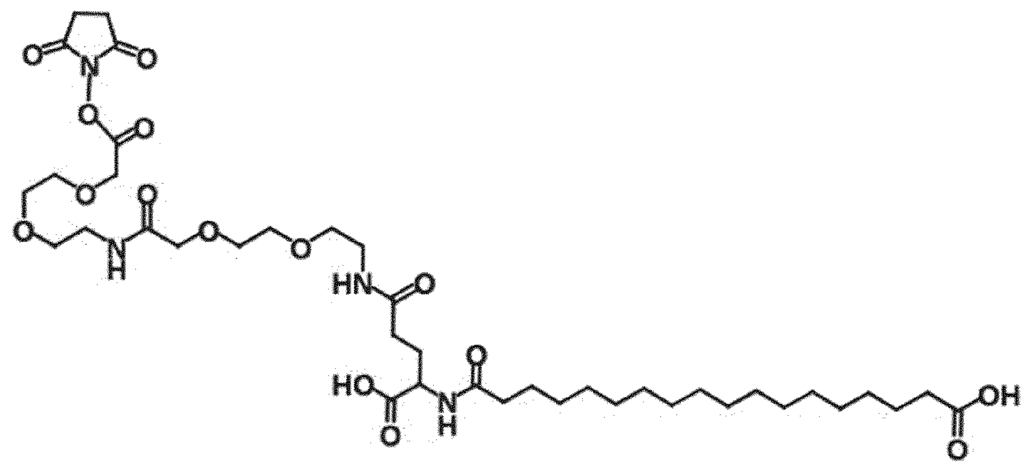

,

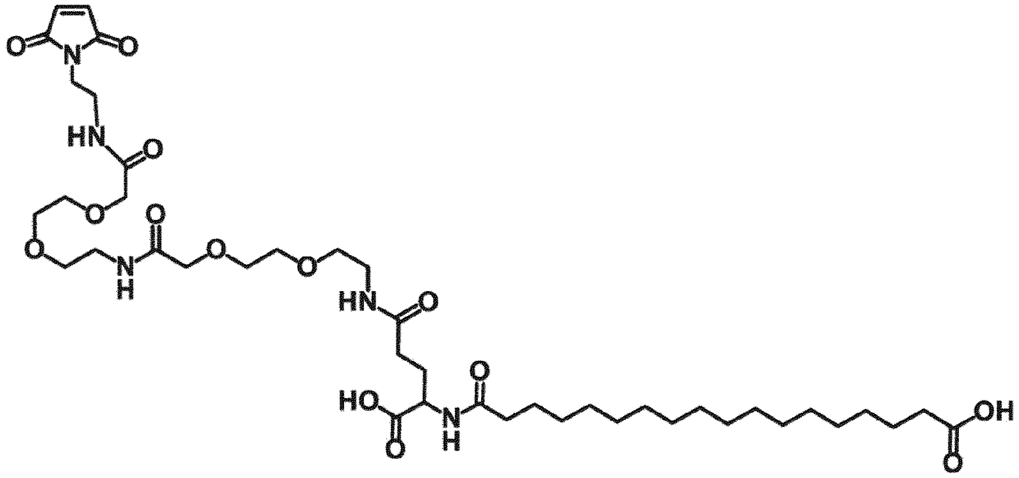

,

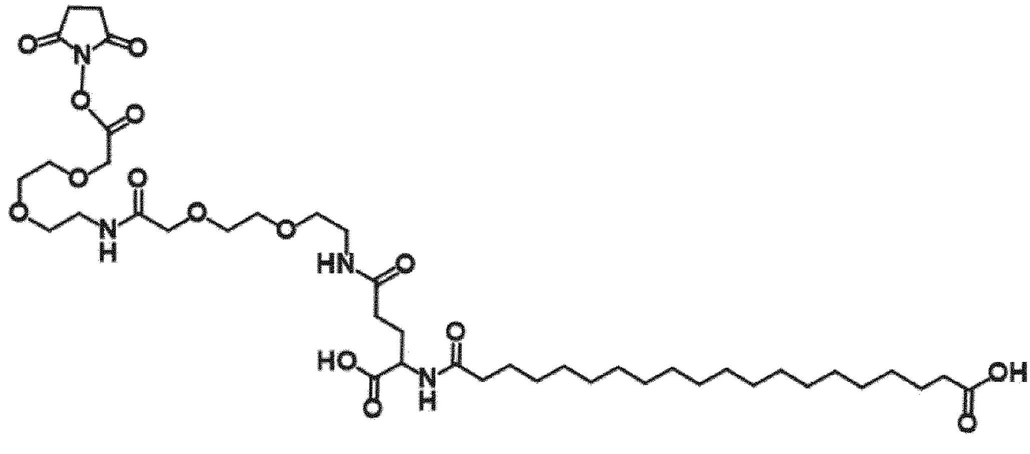

,

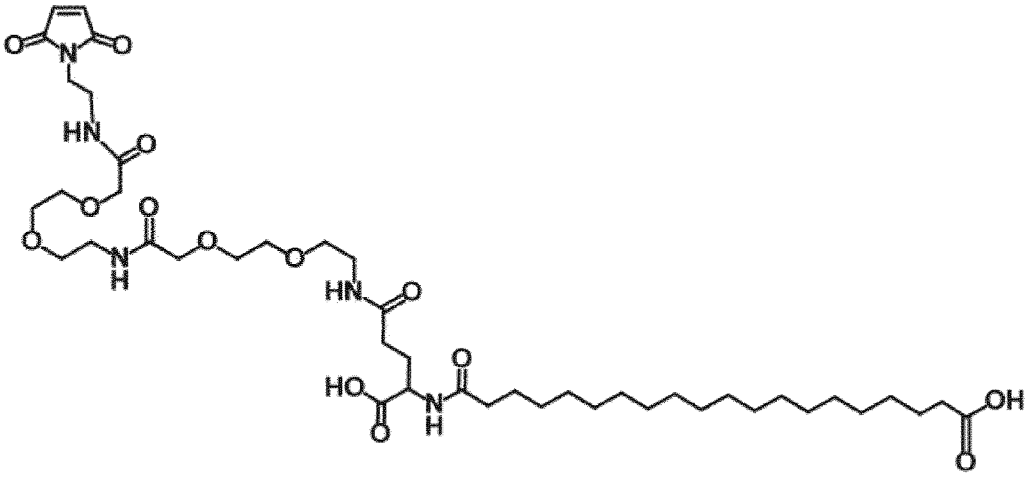

,

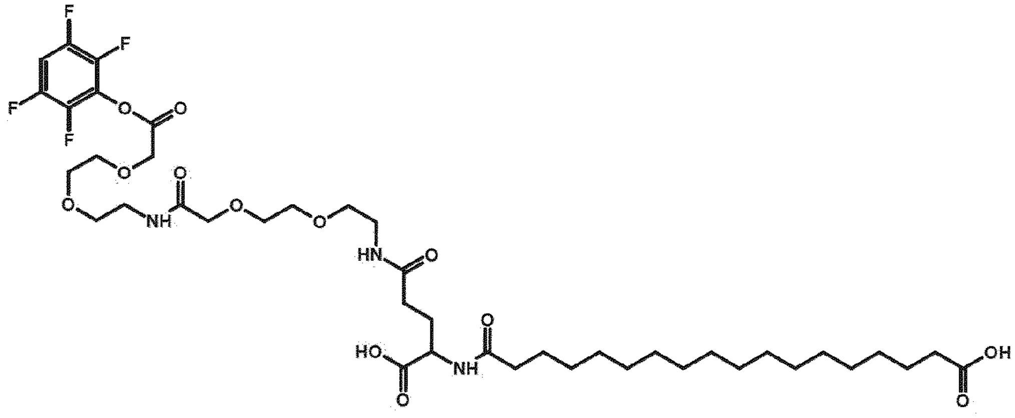

,

and

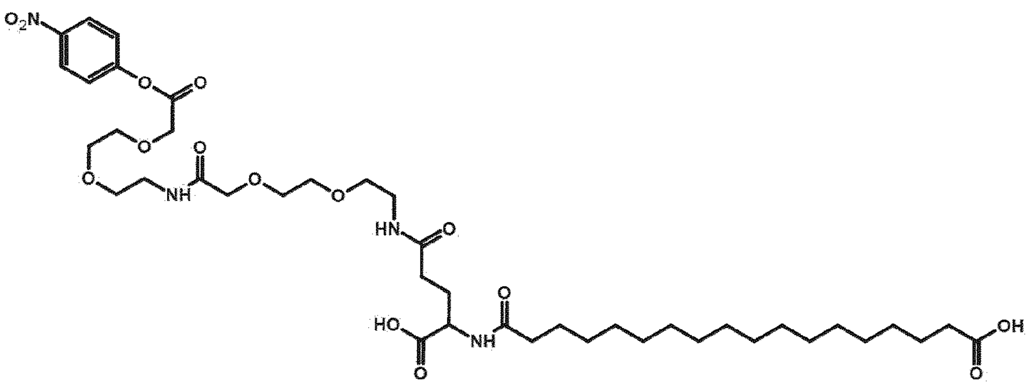

.

According to one embodiment of the present invention, the bond between biotin moiety and the physiologically active substance may be formed by various bonds. It may be formed by bonding a functional group of a biotin moiety with a functional group of a physiologically active material, and may be formed as, for example, but is not limited to, a thiol-ether bond or an amide bond.

In one example, the bond between the biotin moiety and the physiologically active substance may be formed by the method of Reaction Formula 1 below. In Reaction Formula 1

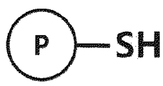

represents a physiologically active substance comprising a thiol group, and represents a reaction between a biotin moiety comprising maleimide according to an embodiment of the present invention and a thiol group (-SH) of a cysteine residue present in the physiologically active substance.

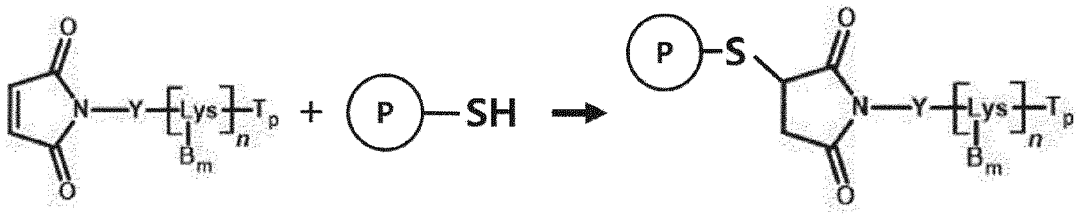

[Reaction Formula 1]

In one specific example, the bond between the biotin moiety and the physiologically active substance may be formed by the method of Reaction Formula 2 below. In Reaction Formula 2,

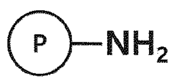

represents a physiologically active substance comprising an amine group, and represents a reaction between a biotin moiety comprising N-hydroxy succinimide according to an embodiment of the present invention and an amine group ($-NH_2$) present in the physiologically active substance.

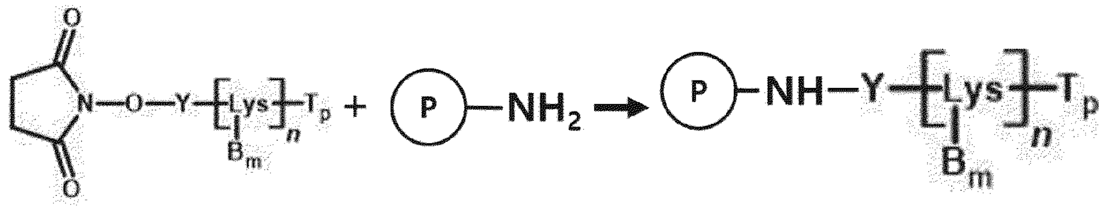

[Reaction Formula 2]

According to one embodiment of the present invention, there may be no particular limitation on the physiologically active substance.

In the present invention, a physiologically active substance refers to a substance which may be administered to the body for a specific purpose, and which causes a physiological or biochemical reaction in the body.

According to an embodiment of the present invention, the physiologically active substance may be a substance used in a pharmaceutical formulation. For example, it may be a substance used for the prevention or treatment of diabetes, obesity, fatty liver disease, irritable bowel syndrome, neurodegenerative disease, bone disease, osteoporosis, human growth hormone deficiency, anticancer or non-alcoholic fatty liver disease. These are non-limiting examples, as the indications may vary depending on the type of the physiologically active substance.

According to one embodiment of the present invention, the physiologically active substance may be, but is not limited to, a polypeptide or a non-peptidic polymer. Non-limiting examples include polypeptide, protein, polysaccharide, or a derivative thereof. Non-limiting examples of the physiologically active substance include glucagon (Glugacon), GLP-1 (Glucagon-like peptide-1), GLP-2 (Glucagon-like peptide-2), GIP (glucose-dependent insulinotropic polypeptide), exendin-4, insulin, parathyroid hormone, interferon, erythropoietin, calcitonin, amylin, serotonin, rituximab, trastuzumab, uricase, tissue plasminogen activator, thymoglobin, vaccine, heparin or heparin analog, antithrombin III, filgrastim, pramlintide acetate, exenatide, eptifibatide, antivenin, IgG, IgM, HGH, thyroxine, blood clotting factors VII and VIII, glycolipids acting as therapeutic agents, and derivatives thereof.

According to one embodiment of the present invention, it may be bonded to a biotin moiety.

By bonding a biotin moiety to the physiologically active substance, it is possible to not inhibit the biological activity of the physiologically active substance, and thereby it is possible to have the same biological activity as the physiologically active substance or an improved biological activity.

Although not limited hereto, the physiologically active substance may comprise an exposed -SH group, so that a biotin moiety may be bonded to the -SH group. In addition, the physiologically active substance may comprise an exposed $-NH_3^+$ group or a $-NH_2$ group, so that a biotin moiety may be bonded to the exposed $-NH_3^+$ group or $-NH_2$ group.

According to one embodiment of the present invention, the binding site of the biotin moiety with the physiologically active substance may be adjusted so as to bond while avoiding sites which exhibit activity.

Further, according to one embodiment of the present invention, the fatty acid moiety may be bonded directly to the physiologically active substance. Further, part of the fatty acid moiety may be shared with the biotin moiety. For example, in one aspect of the following embodiments, biotin moieties B35 and B36 share the fatty acid portion which is part of a fatty acid moiety. However, this corresponds to one example and is not limited thereto.

According to one embodiment of the present invention, the fatty acid moiety may be bonded directly to the physiologically active substance, with the terminal of the fatty acid moiety not bonded to the physiologically active substance bonded to the biotin moiety.

Further, according to one embodiment of the present invention, the fatty acid moiety may be bonded to the physiologically active substance, at a site of the physiologically active substance other than the site at which the biotin moiety is bonded.

In addition, the fatty acid moiety may also bind to the active site or the inactive site of the biotin moiety, and may exhibit the same properties as the above properties.

According to one embodiment of the present invention, both the biotin moiety and the fatty acid moiety may be bonded to the physiologically active substance, and a physiologically active substance conjugate to which both a biotin moiety and fatty acid moiety are bonded, when compared to a conjugate to which only a biotin moiety or only a fatty acid moiety is bonded, may exhibit superior oral absorption rate, pharmacokinetics, enzyme degradation inhibition, intestinal membrane permeation, and the like.

According to one embodiment of the present invention, the physiologically active substance may be glucagon, calcitonin, GLP-1, GLP-2, GIP, exendin-4, parathyroid hormone, insulin, amylin, human growth hormone or a derivative thereof.

According to an embodiment of the present invention, the physiologically active substance may be a polypeptide having any one of the following amino acid sequences of SEQ ID NOs 1 to 7 or derivatives thereof. Specifically, the physiologically active substances of SEQ ID Nos: 1 to 7 are, respectively glucagon derivatives (SEQ ID NO: 1), GLP-1 (SEQ ID NO: 2), GLP-2 (SEQ ID NO: 3), GIP (SEQ ID NO: 4), exendin-4 (SEQ ID NO: 5), parathyroid hormone (SEQ ID NO: 6), and glucagon (SEQ ID NO: 7).

SEQ ID NO: 1: H(Aib)QGTFTSDYSKYLDEQAAKEFVQWLMNT
SEQ ID NO: 2: HAEGTFTSDVSSYLEGQAAKEFIAWLVKGR
SEQ ID NO: 3: HADGSFSDEMNTILDNLAARDFINWLIQTKITD
SEQ ID NO: 4: YAEGTFISDYSIAMDKIHQQDFVNWLLAQKGKKNDWKHNITQ
SEQ ID NO: 5: HGEGTFTSDLSKQMEEEAVRLFIEWLKNGGPSSGAPPPS
SEQ ID NO: 6: SVSEIQLMHNLGKHLNSMERVEWLRKKLQDVHNF
SEQ ID NO: 7: HSQGTFTSDYSKYLDSRRAQDFVQWLMNT

In addition, the physiologically active substance may be proteins having the amino acid sequence of SEQ ID NOs: 15 and 16 or proteins having the amino acid sequence of SEQ ID NOs: 17 and 16, wherein the proteins are joined through disulfide bonds between the 6th and 11th cysteine of SEQ ID NOs: 15 or 17; the 7th cysteine of SEQ ID NOs: 15 or 17 and the 7th cysteine of SEQ ID NO 16; and the 20th cysteine of SEQ ID NOs: 15 or 17 and the 19th cysteine of SEQ ID NO 16. Specifically, the proteins having the amino acid sequences of SEQ ID NOs: 15 and 16 or physiologically active substance having the amino acid sequences of SEQ ID NOs: 17 and 16 represent insulin (SEQ ID NO 15 (Insulin A chain derivative) and 16 (Insulin B chain) / SEQ ID NO 17 (Insulin A chain) and 16 (Insulin B chain))

SEQ ID NO: 15 GIVEQCCTSICSLEQLENYCN
SEQ ID NO: 16: FVNQHLCGSHLVEALYLVCGERGFFYTPKT
SEQ ID NO: 17: GIVEQCCTSICSLYQLENYCN

According to one embodiment of the present invention, cysteine may be substituted or inserted into the polypeptide to adjust the site of binding with the biotin moiety.

In a non-limiting example, any at least one of the amino acids of a polypeptide selected from the group comprising the amino acid sequences represented by SEQ ID NOs: 1 through 7 may be substituted or inserted with a cysteine amino acid. At this time, the biotin moiety bonds to the -SH group of the cysteine amino acid.

Further, any at least one of the amino acids of a polypeptide selected from the group comprising the above amino acid sequences may be substituted or inserted with a lysine amino acid. At this time, the biotin moiety is bound

to the $-NH_2$ group of the lysine amino acid.

Further, the polypeptide into which the cysteine amino acid is inserted may be a polypeptide having any one of the amino acid sequences of SEQ ID NOs: 8 through 14 below. Specifically, the physiologically active substances of SEQ ID NOs: 8 through 14 below refer to the physiologically active substances of SEQ ID NOs: 1 through 7, wherein a cysteine amino acid has been substituted or inserted (for example, in the physiologically active substance of SEQ ID NO 8, at least any one of the amino acids of the physiologically active substance of SEQ ID NO 1 has been substituted with cysteine)

SEQ ID NO: 8: H(Aib)QGTFTSDYSKYLDEQAAKEFVQWLMNTC
SEQ ID NO: 9: HAEGTFTSDVSSYLEGQAAKEFIAWLVKGRC
SEQ ID NO: 10: HADGSFSDEMNTILDNLAARDFINWLIQTKITDC
SEQ ID NO: 11: YAEGTFISDYSIAMDKIHQQDFVNWLLAQKGKKNDWKHNITQC
SEQ ID NO: 12: HGEGTFTSDLSKQMEEEAVRLFIEWLKNGGPSSGAPPPSC
SEQ ID NO: 13: SVSEIQLMHNLGKHLNSMERVEWLRKKLQDVHNFC
SEQ ID NO: 14: HSQGTFTSDYSKYLDSRRAQDFVQWLMNTC

According to one embodiment of the present invention, a portion of the polypeptide may be substituted to adjust the site of binding with the biotin moiety.

Further, according to one embodiment of the present invention, the amino acid lysine may be substituted or inserted into the polypeptide to adjust the site of binding with the biotin moiety.

In a non-limiting example, any at least one of the amino acids of the amino acid sequence represented by SEQ ID NO 5 may be substituted or inserted with the amino acid lysine.

In another non-limiting example, any at least one of the amino acids of the amino acid sequence represented by SEQ ID NO 5 may be substituted with 2-aminoisobutyric acid (Aib), with the insertion of a lysine amino acid. At this time, the biotin moiety is bound to the $-NH_2$ group of the lysine amino acid.

Further, the polypeptide wherein a portion has been substituted, or wherein a lysine amino acid has been substituted or inserted may be a polypeptide having the amino acid sequence of any one of SEQ ID NOs: 18 through 21 below. Specifically, the physiologically active substances of SEQ ID NOs: 18 through 21 below refer to exendin-4 derivatives, wherein a portion of the amino acids of the physiologically active substance of SEQ ID NO 5 has been substituted or inserted.

SEQ ID NO: 18: HGEGTFTSDLSKQMEEEAVRLFIEWLKNGGPSSGAPPPSK
SEQ ID NO: 19: HGEGTFTSDLSKQMEEEAVRLFIEWLKNGGPSSGAPPPSKKK
SEQ ID NO: 20: H(Aib)EGTFTSDLSKQMEEEAVRLFIEWLKNGGPSSGAPPPSK
SEQ ID NO: 21: H(Aib)EGTFTSDLSKQMEEEAVRLFIEWLKNGGPSSGAPPPSKKK

According to one embodiment of the present invention, a cysteine may be substituted or inserted into the polypeptide to adjust the site of binding with the biotin moiety.

In a non-limited example, any at least one of the amino acids of a polypeptide selected from the amino acid sequences of SEQ ID NOs: 1 through 7 may be substituted or inserted with a cysteine amino acid. Here, the biotin moiety bonds to the -SH group of the cysteine amino acid.

Further, any at least one of the amino acids of a polypeptide selected from the group comprising the above amino acid sequences may be substituted or inserted with a lysine amino acid. At this time, the biotin moiety is bound to the $-NH_2$ group of the lysine amino acid.

Further, the polypeptide into which the cysteine amino acid is inserted may be a polypeptide having any one of the amino acid sequences of SEQ ID NOs: 8 through 14 below. Specifically, the physiologically active substances of SEQ ID NOs: 8 through 14 below refer to the physiologically active substances of SEQ ID NOs: 1 through 7, wherein a cysteine amino acid has been substituted or inserted (for example, in the physiologically active substance of SEQ ID NO 8, at least any one of the amino acids of the physiologically active substance of SEQ ID NO 1 has been substituted with cysteine)

SEQ ID NO: 8: H(Aib)QGTFTSDYSKYLDEQAAKEFVQWLMNTC
SEQ ID NO: 9: HAEGTFTSDVSSYLEGQAAKEFIAWLVKGRC
SEQ ID NO: 10: HADGSFSDEMNTILDNLAARDFINWLIQTKITDC
SEQ ID NO: 11: YAEGTFISDYSIAMDKIHQQDFVNWLLAQKGKKNDWKHNITQC
SEQ ID NO: 12: HGEGTFTSDLSKQMEEEAVRLFIEWLKNGGPSSGAPPPSC
SEQ ID NO: 13: SVSEIQLMHNLGKHLNSMERVEWLRKKLQDVHNFC
SEQ ID NO: 14: HSQGTFTSDYSKYLDSRRAQDFVQWLMNTC

According to one embodiment of the present invention, a portion of the polypeptide may be substituted to adjust the site of binding with the biotin moiety.

Further, according to one embodiment of the present invention, the amino acid lysine may be substituted or inserted into the polypeptide to adjust the site of binding with the biotin moiety.

In a non-limiting example, any at least one of the amino acids of the amino acid sequence represented by SEQ ID NO 5 may be substituted or inserted with the amino acid lysine.

In another non-limiting example, any at least one of the amino acids of the amino acid sequence represented by SEQ ID NO 5 may be substituted with 2-aminoisobutyric acid (Aib), with the insertion of a lysine amino acid. Here, the biotin moiety bonds to the $-NH_2$ group of the lysine amino acid.

Further, the polypeptide wherein a portion has been substituted, or wherein a lysine amino acid has been substituted or inserted may be a polypeptide having the amino acid sequence of any one of SEQ ID NOs: 18 through 21 below. Specifically, the physiologically active substances of SEQ ID NOs: 18 through 21 below refer to exendin-4 derivatives, wherein a portion of the amino acids of the physiologically active substance of SEQ ID NO 5 has been substituted or inserted.

SEQ ID NO: 18: HGEGTFTSDLSKQMEEEAVRLFIEWLKNGGPSSGAPPPSK
SEQ ID NO: 19: HGEGTFTSDLSKQMEEEAVRLFIEWLKNGGPSSGAPPPSKKK
SEQ ID NO: 20: H(Aib)EGTFTSDLSKQMEEEAVRLFIEWLKNGGPSSGAPPPSK
SEQ ID NO: 21: H(Aib)EGTFTSDLSKQMEEEAVRLFIEWLKNGGPSSGAPPPSKKK

According to one embodiment of the present invention, the physiologically active substance may be a polypeptide having the amino acid sequence of SEQ ID NO 22 below, or a derivative thereof. The physiologically active substance of SEQ ID NO 22 below refers to amylin. SEQ ID NO: 22: KCNTATCATQRLANFLVHSSNNFGAILSSTNVGSNTY

According to one embodiment of the present invention, a portion of the amino acid sequence represented by SEQ ID NO 22 may be substituted or inserted to adjust the site of binding with the biotin moiety.

In a non-limiting example, any at least one of the amino acid having the amino acid sequence represented by SEQ ID NO 22 may be substituted with the amino acid proline, aspartic acid, or arginine. In another non-limiting example, any at least one of the amino acid having the amino acid sequence represented by SEQ ID NO 22 may be substituted with the amino acid lysine.

Further, the polypeptide wherein a portion of the amino acids represented by SEQ ID NO 22 have been substituted or inserted may be a polypeptide having the amino acid sequence of any one of SEQ ID NOs: 23 through 31 below. Specifically, the physiologically active substances of SEQ ID NOs: 23 through 31 below represent amylin derivatives wherein a portion of the amino acids of the physiologically active substance of SEQ ID NO 22 has been substituted or inserted.

SEQ ID NO: 23: KCNTATCATQRLANFLVHSSNNFGAILSSTNVGSNTYK
SEQ ID NO: 24: KCNTATCATQRLANFLVHSSNNFGPILPPTNVGSNTY
SEQ ID NO: 25: KCNTATCATQRLANFLVHSSNNFGPILPPTNVGSNTYK
SEQ ID NO: 26: KCNTATCATQRLADFLRHSSPNFGAIPSSTNVGSRTY
SEQ ID NO: 27: KCNTATCATQRLADFLRHSSPNFGAIPSSTNVGSRTYK
SEQ ID NO: 28: KCNTATCATQRLADFLRHSSNNFGAIPSSTNVGSRTY
SEQ ID NO: 29: KCNTATCATQRLADFLRHSSNNFGAIPSSTNVGSRTYK
SEQ ID NO: 30: RCNTATCATQRLADFLRHSSNNFGAIPSSTNVGSKTY
SEQ ID NO: 31: RCNTATCATQRLADFLRHSSNNFGAIPSSTNVGSKTYK

According to one embodiment of the present invention, the physiologically active substance may be a polypeptide having the amino acid sequence of SEQ ID NO 32 below, or a derivative thereof. The physiologically active substance of SEQ ID NO 32 below represents exendin-4 derivatives.
SEQ ID NO: 32: H(Aib)QGTFTSDKSKYLDERAAQDFVQWLLDGGPSSGAPPPS

According to one embodiment of the present invention, a portion of the amino acid sequence of SEQ ID NO 32 may be deleted, substituted or inserted to adjust the site of binding with the biotin moiety.

In a non-limiting example, any at least one of the amino acids of the amino acid sequence represented by SEQ ID NO 32 may be substituted with the amino acid methionine, lysine, isoleucine, tryptophan or glycine. In another non-limiting example, any at least one of the amino acids of the amino acid sequence represented by SEQ ID NO 32 may be deleted.

Further, the polypeptide in which a portion of the amino acids represented by SEQ ID NO 32 has been deleted, substituted or inserted may be a polypeptide having the amino acid sequence of any one of SEQ ID NOs: 32 through 37 below. Specifically, the physiologically active substances of SEQ ID NOs: 33 through 37 below wherein a portion of

amino acids of the physiologically active substance of SEQ ID NO 32 has been deleted, substituted or inserted represent exendin-4 derivatives.

SEQ ID NO: 33: H(Aib)QGTFTSDKSKYLDERAAQDFVQWLMDGGPSSGAPPPS
SEQ ID NO: 34: H(Aib)QGTFTSDKSKYLDKIAAQDFVQWLIDGGPSSGAPPPS
SEQ ID NO: 35: H(Aib)QGTFTSDKSWYLDKIAAQDFVQWLLGGGPSSGAPPPS
SEQ ID NO: 36: H(Aib)QGTFTSDKSWYLDERAAQDFVQWLMGGGPSSGAPPPS
SEQ ID NO: 37: H(Aib)QGTFTSDKSKWLDKIAAQDFVQWLIGGGPSSGAPPPS

According to one embodiment of the present invention, any at least one of the amino acids of the amino acid sequence represented by SEQ ID NO 12 may be substituted with 2-aminoisobutyric acid (Aib).

According to one embodiment of the present invention, a polypeptide wherein any at least one of the amino acids of the amino acid sequence represented by SEQ ID NO 12 has been substituted with 2-aminoisobutyric acid (Aib) and any at least one has been substituted with Des-amino-His(h) may be the polypeptide having the amino acid sequence of SEQ ID NOs: 38 through 39 below. Specifically, the physiologically active substances of SEQ ID NOs: 38 or 39 below, wherein amino acids of the physiologically active substance of SEQ ID NO 12 have been substituted, represent exendin-4 derivatives. More specifically, the physiologically active substance having the amino acid sequence of SEQ ID NO 39 is a physiologically active substance wherein at least one of the amino acids has been substituted with Des-amino-His(h).

SEQ ID NO: 38: H(Aib)HGEGTFTSDLSKQMEEEAVRLFIEWLKNGGPSSGAPPPSC
SEQ ID NO: 39: H(Aib)HGEGTFTSDLSKQMEEEAVRLFIEWLKNGGPSSGAPPPSC

According to one embodiment of the present invention, any at least one of the amino acids of the amino acid sequence represented by SEQ ID NO 8 may be substituted with lysine (Lys) or arginine (Arg).

A polypeptide wherein any at least one of the amino acids of the amino acid sequence represented by SEQ ID NO 8 has been substituted with lysine or arginine may be the polypeptide having the amino acid sequence of SEQ ID NOs: 40 through 41 below. Specifically, the physiologically active substances of SEQ ID NOs: 40 or 41 below, wherein amino acids of the physiologically active substance of SEQ ID NO 8 have been substituted, represent glucagon derivatives.

SEQ ID NO: 40: H(Aib)QGTFTSDYSKYLDEQAAKEFVQWLMNTK
SEQ ID NO: 41: H(Aib)QGTFTSDYSKYLDEKRAKEFVQWLMNTC

According to one embodiment of the present invention, the physiologically active substance may be the polypeptide having the amino acid sequence of SEQ ID NO 42 or a derivative thereof. Specifically, the physiologically active substance of SEQ ID NO 42 represents a human growth hormone derivative.

SEQ ID NO: 42:

MFPTIPLSRLFDNAMLRAHRLHQLAFDTYQEFEEAYIPKEQKISFLQNPQTSLCFSESIP
TPSNREETQQKSNLELLRISLLLIQSWLEPVQFLRSVFANSLVYGASDSNVYDLLKDLEEG
IQTLMGRLEDGSPRTGQIFKQTYSKFDTNSHNDDALLKNYGLLYCFRKDMDKVETFLRI
VQCRSVEGSCGF

According to one embodiment of the present invention, the physiologically active material to which the biotin moiety, fatty acid moiety or a combination thereof is bonded may be covalently bonded with, or form an inclusion body (microsphere) with, any at least one selected from the group comprising peptide and non-peptidic polymer, fatty acid, cholesterol, antibody, antibody fragment, albumin and fragments thereof, nucleotide, fibronectin, transferrin, FcRn binding material, saccharide, elastin, heparin, and derivatives thereof.

The non-peptidic polymer may be selected from the group comprising polyethylene glycol (PEG), polypropylene glycol, copolymers of ethylene glycol and propylene glycol, polyoxyethylated polyols, polyvinyl alcohol (PVA), polysaccharides, dextran, polyvinylethyl ether, PLA (polylactic acid, polylactic acid), PLGA (polylactic-glycolic acid), lipid polymer, chitin, hyaluronic acid, and combinations thereof.

In the present invention, "derivative" means that a portion of the chemical structure has been modified by deletion, substitution, addition, or the like.

In the present invention, "pharmaceutically acceptable" means that the substances comprised do not substantially irritate the organism and do not inhibit biological activity and properties.

In the present invention, "pharmaceutically acceptable salt" refers to a salt having desirable biological activity that does not inhibit biological activity and properties in humans or animals, and includes, but is not limited to, inorganic acid salts (hydrochloric acid, sulfuric acid, phosphoric acid, nitric acid), organic Acids (acetic acid, oxalic acid, maleic acid, fumaric acid, succinic acid, benzoic acid, ascorbic acid, tannic acid, pamoic acid, alginic acid, triethylamine, cyclohexylamine, pyridine), alkali metal salts (sodium salt, potassium salt), alkaline earth metal salts (calcium salts), ammonium salts, addition salts thereof, and the like.

In the present invention, a bile acid is an amphiphilic molecule and can promote drug permeation through a biological membrane. A bile acid may be absorbed in a form bonded with the physiologically active substance bound to a biotin moiety, a fatty acid moiety, or a combination thereof of the present invention, thereby minimizing the loss of the physiologically active substance upon oral administration and thereby improving the absorption rate in the body.

Further, a bile acid derivative in which a part of a bile acid is substituted, deleted, or added may be appropriately selected in consideration of cell stability, cytotoxicity, absorption rate in the body, and the like.

In one embodiment of the present invention, the excipient comprises a bile acid, a derivative thereof, or a pharmaceutically acceptable salt thereof.

In a specific embodiment of the present invention, the bile acid is at least one selected from the group comprising glycocholic acid, glycochenodeoxycholic acid, taurochenodeoxycholic acid, taurocholic acid, deoxycholic acid, cholic acid, chenodeoxycholic acid, ursodeoxycholic acid, and lithocholic acid.

In a specific embodiment of the present invention, the bile acid is one selected from the group comprising chenodeoxycholic acid, deoxycholic acid, cholic acid, glycocholic acid, taurocholic acid and ursodioxycholic acid.

In one embodiment of the present invention, the excipient may further comprise at least one selected from the group comprising alpha-tocopherol, malic acid, fumaric acid, ascorbic acid, butylated hydroxyanisole, butylated hydroxy toluene, sodium phosphate, calcium phosphate, potassium phosphate, galactose, glucose, maltose, gallic acid, propyl gallate, and pharmaceutically acceptable salts thereof.

In a specific embodiment of the present invention, the excipient may further comprise gallic acid, propyl gallate or a pharmaceutically acceptable salt thereof.

In one embodiment of the present invention, the excipient may comprise two or more bile acids or pharmaceutically acceptable salts thereof. In a specific embodiment of the present invention, the excipient may be kenodioxycholic acid, deoxycholic acid, ursodioxycholic acid, or a pharmaceutically acceptable salt thereof.

In one embodiment of the present invention, the weight ratio of (i) the physiologically active substance bound to the biotin moiety and (ii) the excipient is 1: 0.01 to 1000. If the excipient comprises two or more different excipients, the above excipient weight means the weight of all excipients included.

More specifically, the weight ratio may be 1: 0.01 to 900, 1: 0.015 to 850, 1: 0.018 to 800, 1: 0.02 to 750, 1: 0.025 to 700, 1: 0.028 to 650, 1: 0.03 to 600, 1: 0.035 to 550, 1: 0.038 to 500, 1: 0.04 to 500, 1: 0.042 to 500, 1: 0.045 to 500, 1: 0.048 to 500, 1: 0.05 to 500, 1: 0.05 to 450, 1: 0.05 to 400, 1: 0.05 to 350, 1: 0.05 to 300, or 1: 0.05 to 250.

In a more specific embodiment of the present invention, the excipient includes bile acid or a pharmaceutically acceptable salt thereof, and propyl gallate or a pharmaceutically acceptable salt thereof.

Specifically, the weight ratio of bile acid or a pharmaceutically acceptable salt thereof to propyl gallate or a pharmaceutically acceptable salt thereof may be 1: 0.01 to 8. More specifically, the weight ratio may be 1: 0.015 to 7.5, 1: 0.018 to 7.5, 1: 0.02 to 7, 1: 0.025 to 7, 1: 0.028 to 6.5, 1: 0.03 to 6.5, 1: 0.035 to 6, 1: 0.038 to 6, 1: 0.04 to 5.5, 1: 0.042 to 5.5, 1: 0.045 to 5, 1: 0.046 to 5, 1: 0.047 to 5, 1: 0.048 to 5, 1: 0.049 to 5, or 1: 0.05 to 5.

Specifically, the weight of the bile acid or a pharmaceutically acceptable salt thereof may be 1mg to 1,000mg. More specifically, the weight may be at least 25mg, at least 50mg, at least 100mg, at least 150mg, at least 180mg, at least 200mg, at least 210mg, at least 220mg, at least 230mg, at least 240mg, or at least 250mg. However, weight of the bile acid or a pharmaceutically acceptable salt thereof may be appropriately adjusted according to the patient's body weight, administration dose, number of administrations, and the like.

Specifically, the weight of propyl gallate or a pharmaceutically acceptable salt thereof may be 1mg to 1,000mg. More specifically, the weight may be at least 25mg, at least 50mg, at least 100mg, at least 150mg, at least 200mg, at least 250mg, at least 300mg, at least 310mg, at least 320mg, at least 330mg, at least 340mg, or at least 350mg. However, the weight of propyl gallate or a pharmaceutically acceptable salt thereof may be appropriately adjusted according to the patient's body weight, administration dose, number of administrations, and the like.

In one embodiment of the present invention, the oral absorption rate of the oral pharmaceutical formulation may be improved through binding of a biotin moiety and/or fatty acid moiety, even if [the oral pharmaceutical formulation] does not comprise an excipient.

In a specific embodiment of the present invention, the oral pharmaceutical formulation may have an oral absorption rate at least 1.5 times improved over a case wherein an excipient is not used.

Further, by using the above bile acids, derivatives thereof or pharmaceutically acceptable salts thereof as an excipient in the oral pharmaceutical formulation, the oral absorption rate may be improved by 1.5 times or more.

In a more specific embodiment of the present invention, by using propyl gallate or a pharmaceutically acceptable salt thereof as an excipient in the oral pharmaceutical formulation, the oral absorption rate may be improved by 1.5 times or more. Here, in a case where [the formulation] comprises propyl gallate or a pharmaceutically acceptable salt thereof, the oral absorption rate may be improved by 1.7 times or more when compared to a case wherein only the physiologically active substance is present; the oral absorption rate may be improved by 1.8 times or more when compared to a case wherein [the physiologically active substance] is bound to a biotin moiety and/or a fatty acid moiety; and the oral absorption rate may be improved by 2 times or more when compared to a case wherein [the physiologically active substance] is bound to a biotin moiety and/or a fatty acid moiety and comprises a bile acid as an excipient.

In the present specification, the excipient may further comprise a conventionally pharmaceutically acceptable excipient. The physiologically active material bound to the biotin moiety of the present invention may be formulated using an excipient, non-limiting examples of which include stabilizer, surfactant, plasticizer, lubricant, solubilizer, buffer, sweetener, base, adsorbent, flavoring agent, binder, suspending agent, antioxidant, brightening agent, coating agent, flavoring agent, flavoring agent, wetting agent, wetting agent, defoaming agent, chewing agent, refreshing agent, colorant, sugar coating agent, isotonic agent, pH adjusting agent, emollient, emulsifier, adhesive, adhesion enhancer, thickening agent, thickening agent, foaming agent, excipient, dispersing agent, propellant, disintegrating agent, disintegrating aid, fragrance, desiccant, preservative, preservative, softening agent, solvent, solubilizer, solubilizing agent, fluidizing agent, and the like.

In the present invention, the oral pharmaceutical formulation may further comprise include starch, calcium carbonate, sucrose or lactose, gelatin and the like for solid preparations, and suspensions, internal solutions, emulsions, syrups and the like for liquid preparations, and may further comprise a lubricant, a wetting agent, a sweetener, a fragrance, a preservative, and the like. In addition, calcium or Vitamin D3 may be further added to enhance efficacy as a therapeutic agent for proliferative diseases or autoimmune diseases.

The object of another embodiment of the present invention is to provide a use of the oral pharmaceutical formulation described in the above. The use of the pharmaceutical formulation may be determined according to the type of physiologically active substance.

Here, the use of the pharmaceutical formulation may be determined according to the type of physiologically active substance.

According to one embodiment of the present invention, the formulation may be used for the prevention or treatment of diabetes, obesity, fatty liver disease, irritable bowel syndrome, neurodegenerative disease, bone disease, osteoporosis, human growth hormone deficiency, anticancer or non-alcoholic fatty liver disease.

According to one embodiment of the present invention, when the physiologically active substance is GLP-1, GLP-2, GIP, insulin, amylin or a derivative thereof, the conjugate can be used for the prevention or treatment of diabetes. Specifically, the conjugate comprising the physiologically active substance of SEQ ID NO 12 can be used for preventing or treating diabetes. However, this example is illustrative and the present invention is not limited hereto.

Further, according to one embodiment of the present invention, when the physiologically active substance is parathyroid hormone or a derivative thereof, the conjugate can be used for the prevention or treatment of bone diseases. Specifically, the conjugate comprising the physiologically active substance of SEQ ID NO 6 can be used for the prevention or treatment of bone diseases. However, this example is illustrative and the present invention is not limited hereto.

According to one embodiment of the present invention, when the physiologically active substance is hGH or a derivative thereof, the conjugate can be used for preventing or treating human growth hormone deficiency. Specifically, the conjugate comprising the physiologically active substance of SEQ ID NO 42 can be used for preventing or treating human growth hormone deficiency, be used for preventing or treating human growth hormone deficiency. However, this example is illustrative and the present invention is not limited hereto.

In the following, the present invention is described in detail by means of embodiments and experimental examples. However, the following embodiments and experimental examples are intended to exemplify the present invention, and the present invention is not limited thereto.

[Embodiments]

Preparation of biotin moiety>

List of abbreviations

HBTU: 3-[Bis(dimethylamino)methyliumyl]-3H-benzotriazole-1-oxide hexafluorophosphate (: 3-[Bis(dimethylamino)methyliumyl]-3H-benzotriazol-1-oxide hexafluorophosphate)
DIEA: Ethyldiisopropylamine
HATU: 1-[bis(dimethylamino)methylene]-1H-1,2,3-triacolo[4,5-b]pyridinium-3oxide hexafluorophosphate (1-[Bis(dimethylamino)methylene]- 1H-1,2,3-triazolo[4,5-b]pyridinium 3-oxide hexafluorophosphate)
DIC: Diisopropylcarbodiimide
HOBt:: 1-Hydroxybenzotriazole
MBHA: 4-Methylbenzhydrylamine hydrochloride
Fmoc: 9-Fluorenylmethoxycarbonyl
DMF: dimethylformamide
SPPS: Solid Phase Peptide Synthesis
HPLC: High Performance Liquid Chromatography
LCMS: Liquid Chromatography Mass Spectrometry

Common SPPS method

In some cases, solid phase synthesis of a peptide can be improved through use a di-peptide protected from di-peptide amide bonds having groups that can be cleaved under acidic conditions, for example, 2-Fmoc-oxy-4-methoxybenzyl, or 2,4,6-trimethoxybenzyl. The Fmoc-protected amino acid derivative used was the recommended standard, for example: Fmoc-Ala-OH, Fmoc-Arg(Pbf)-OH, Fmoc-Asn(Trt)-OH, Fmoc-Asp(OtBu)-OH, Fmoc-Cys(Trt)-OH, Fmoc-Gln(Trt)-OH, Fmoc-Glu(OtBu)-OH, Fmoc-Gly-OH, Fmoc-His(Trt)-OH, Fmoc-Ile-OH, Fmoc-Leu-OH, Fmoc-Lys(Boc)-OH, Fmoc-Met-OH, Fmoc-Phe-OH, Fmoc-Pro-OH, Fmoc-Ser(tBu)-OH, Fmoc-Thr(tBu)-OH, Fmoc-Trp(Boc)-OH, Fmoc-Tyr(tBu)-OH, or Fmoc-Val-OH and the like supplied by Anaspec, Bachem, Iris Biotech, or Novabiochem. The N-terminal amino acid was Boc protected at the alpha amino group. For example: Fmoc-8-amino-3,6-dioxaoctanoic acid, Fmoc-tranexamic acid, Fmoc-isonipecotic acid, Fmoc-Glu-OtBu, Fmoc-Lys(Fmoc)-OH supplied by Anaspec, Bachem, Iris Biotech, or Novabiochem was used.

Peptide synthesis using SPPS

Peptides can be synthesized using general Fmoc chemistry in link amide MBHA resins using HBTU/DIEA, HATU/DIEA, or DIC/HOBt as the coupling reagents. The combinations of reactants and coupling reagents used in synthesis include the following.

[Table 1]

| # | Reactant | Coupling Reagent |
|---|---|---|
| 1 | Fmoc-Lys (Biotin)-OH (1.5 *eq*) | HBTU (1.42 eq) and DIEA (3.0 eq) |
| 2 | Fmoc-Lys (Biotin)-OH (2.0 *eq*) | HBTU (1.9 eq) and DIEA (4.0 eq) |
| 3 | 3-(2,5-dioxo-2,5-dihydro-1H-pyrrol-1-yl)propanoic acid (3.0 *eq*) | DIC (3.0 eq) and HOBt (6.0 eq) |
| 4 | 2,2-dimethyl-4-oxo-3,7,10,13,16,19,22-heptaoxapentacosan-25-oic acid (2.0 *eq*) | HATU (1.9 eq) and DIEA (4.0 eq) |
| 5 | Fmoc-21-amino-4,7,10,13,16,19-hexaoxaheneicosanoic acid (2.0 *eq*) | HATU (1.9 eq) and DIEA (4.0 eq) |

An exemplary protocol for the peptide synthesis process using SPPS comprises the following. 1) Add DMF to a vessel containing link amide MBHA resin and expand for 2 hours (sub: 0.68 mmol/g, 1.0 mmol, 1.47 g or 5 mmol, 7.35 g, sub: 0.68 mmol/g). 2) After adding 20% piperidine/DMF, mix for 30 minutes. 3) After removing the solvent of 1)-2), wash using DMF (30 seconds x 5 times). 4) Add the reactant (one of the reactants #1 to #5) and mix for 30 seconds, then add the coupling reagent (one of the coupling reagents #1 to #5) corresponding to the reactant, and carry out nitrogen bubbling for 1 hour. 5) After adding 20% piperidine/DMF, mix for 30 minutes. In the exemplary protocol of 1) to 5) above, iterative synthesis can be performed using combinations of the reactants and coupling reactants #1 to #5 more than once. To remove Fmoc, treatment with 20% piperidine/DMF solution for 30 minutes was used.

General Procedure for Peptide Purification and Analysis

Unpurified peptide was dissolved in an appropriate mixture of water, TFA and ACN, purified using preparative HPLC, dried and quantified. The conditions for purification using preparative HPLC include those shown in Table 2 below.

[Table 2]

| Purification Conditions | |
|---|---|
| Solvent | $ACN/H_2O$ |
| Equipment | SHIMADZU LC-8A, or Gilson GX-281 |
| Mobile Phase | A: $H_2O$ (0.075% TFA in $H_2O$) |
| | B: $CH_3CN$ |
| Gradient | 15-35%-60 min. Retention time: 42min, or<br>20-50%-60 min. Retention time: 45min, or<br>5-35%-60 min. Retention time: 50min |
| Column | Luna25*200mm, C18, 10um, 110A+Gemin150*30mm, C18, 5um, 110A, or<br>Luna50*25mm, C18, 10um, 100A+Gemini(R)250*50mm, C8, 5um, 110 |
| Flow Rate | 80mL/Min or 20mL/Min |
| Wavelength | 220/254 nm |
| Oven Tern. | Room Temperature |

After purification using preparative HPLC, the final product was characterized using analytical HPLC or LCMS. As a result of the analysis, the biotin moieties of Table 3 below were obtained.

[Table 3]

| Biotin Moiety | Designation |
|---|---|
| B1 | N-Biotinoyl-N'-(6-maleiidohexanoyl)hydrazide |
| B2 | 3-Maleimidopropionate-Lys(Biotin)-Lys(Biotin)-$CONH_2$ |
| B3 | 3-Maleimidopropionate-Lys(Biotin)-Lys(Biotin)-Lys(Biotin)-$CONH_2$ |
| B4 | propionate-N-hydroxysuccinimide ester-PEG-Lys(Biotin)-Lys(Biotin)-Lys(Biotin)-$CONH_2$ |
| B5 | 3-Maleimidopropionate-PEG-Lys(Biotin)-Lys(Biotin)-Lys(Biotin)-$CONH_2$ |

B1:

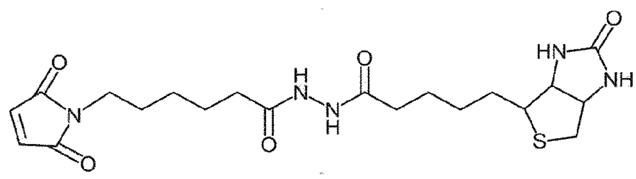

,

B2:

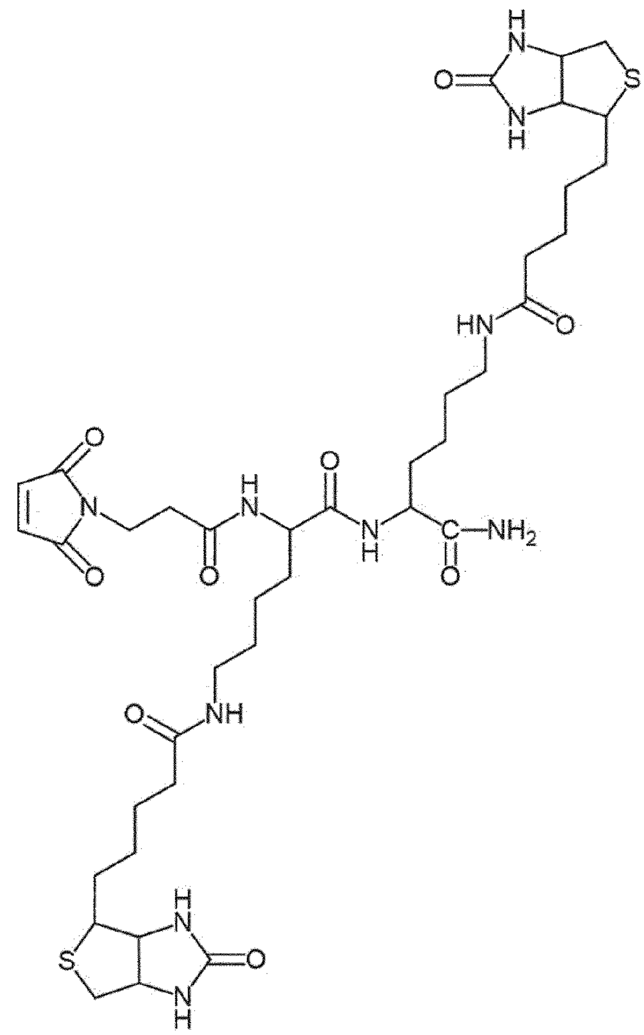

,

B3:

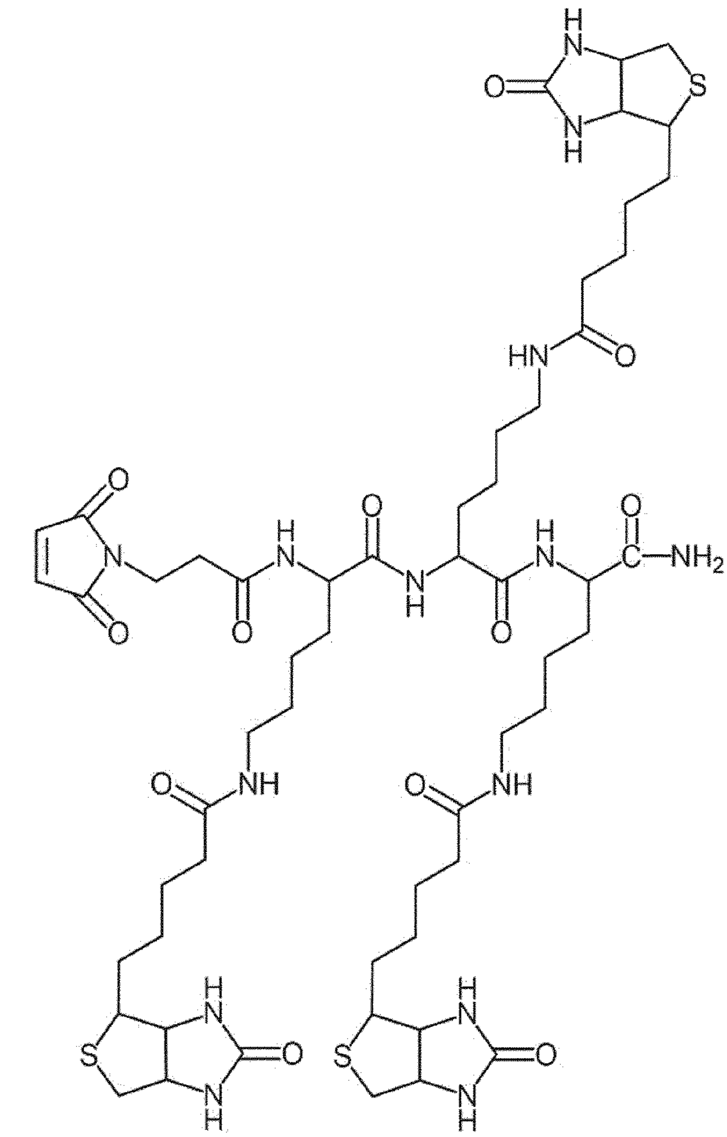

,

B4:

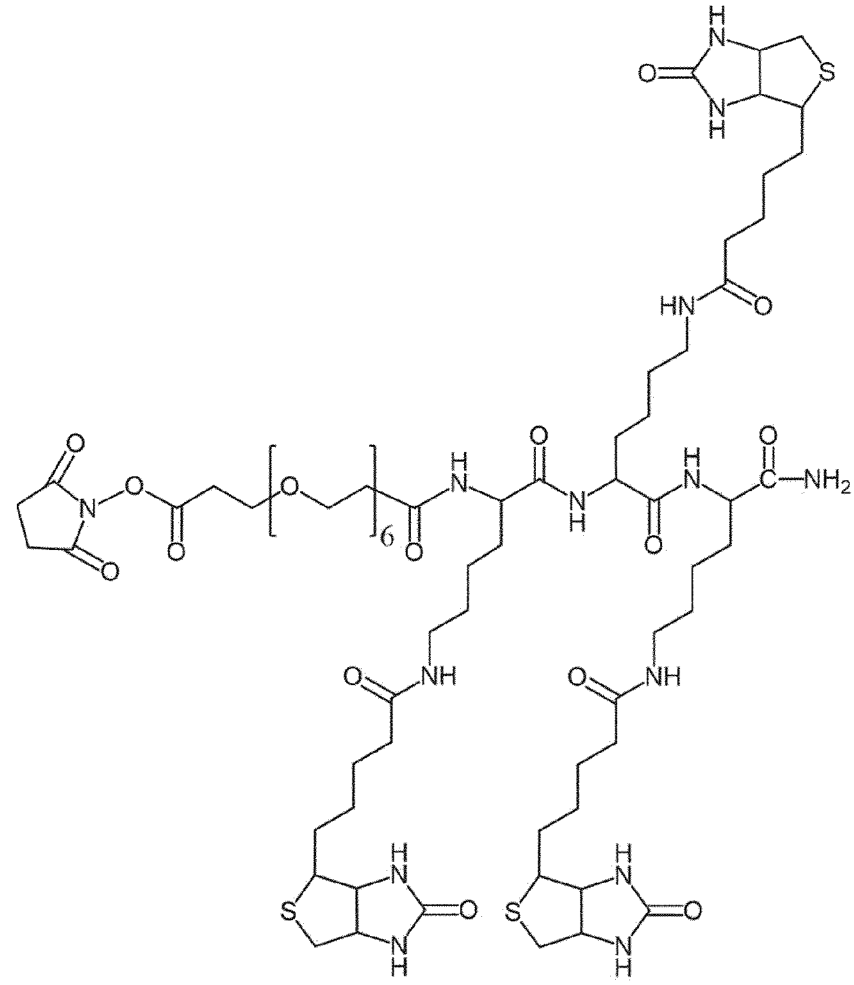

,

B5:

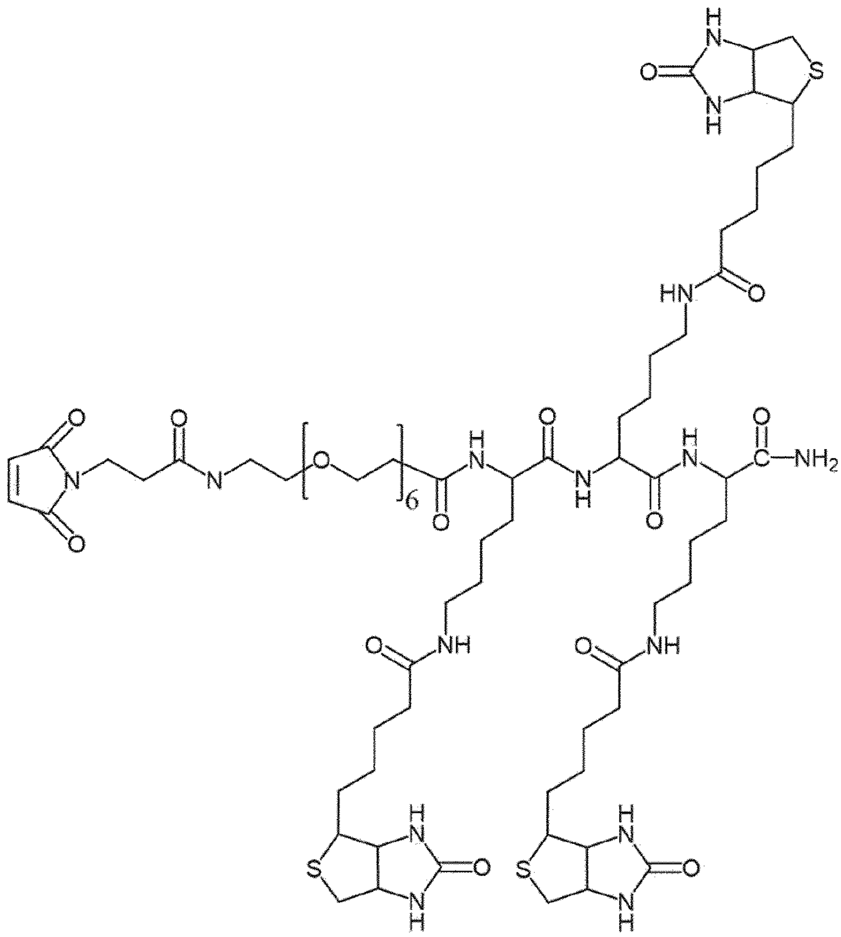

Further, through the protocol 1)~5) for peptide synthesis using SPPS, purification and analysis, the following biotin moieties were obtained. In Table 4 below, X, Y, Z and B are included in the definition of General Formula A of the present specification.

[Table 4]

| Biotin Moiety | X | Y | Z | Number of B (Biotin) |
|---|---|---|---|---|
| B6 | Aldehyde | propane | Lysine | 2 |
| B7 | Maleimide | butyrate | Glycerol and PEG | 2 |
| B8 | Maleimide | butyrate | Glycerol and PEG | 2 |
| B9 | N-hydroxysuccinimide | butyrate | Lysine | 2 |
| B10 | N-hydroxysuccinimide | glutarate | Glycerol and PEG | 2 |
| B11 | Maleimide | PEG12 | Lysine | 3 |
| B12 | N-hydroxysuccinimide | PEG12 | Lysine | 3 |
| B13 | amine | - | Lysine | 3 |
| B14 | Aldehyde | pentane | Lysine | 2 |
| B15 | Maleimide | adipate | Glycerol and PEG | 2 |
| B16 | Maleimide | suberate | Glycerol and PEG | 2 |
| B17 | Maleimide | sebacate | Glycerol and PEG | 2 |
| B18 | N-hydroxysuccinimide | adipate | Glycerol and PEG | 2 |
| B19 | N-hydroxysuccinimide | suberate | Lysine | 4 |

(continued)

| Biotin Moiety | X | Y | Z | Number of B (Biotin) |
|---|---|---|---|---|
| B20 | N-hydroxysuccinimide | sebacate | Lysine | 4 |
| B21 | N-hydroxysuccinimide | PEG6 | Glycerol and PEG | 2 |
| B22 | Succinimidyl carbonate | PEG6 | Lysine | 2 |
| B23 | Succinimidyl carbonate | PEG12 | Lysine | 3 |
| B24 | Succinimidyl carbonate | pentane | Lysine | 3 |
| B25 | Succinimidyl carbonate | hexane | Lysine | 3 |
| B26 | p-nitrophenyl carbonate | PEG6 | Lysine | 3 |
| B27 | p-nitrophenyl carbonate | PEG12 | Lysine | 4 |
| B28 | p-nitrophenyl carbonate | propane | Glycerol and PEG | 2 |
| B29 | p-nitrophenyl carbonate | pentane | Glycerol and PEG | 2 |
| B30 | amine | - | Glycerol and PEG | 2 |
| B31 | thiol | butyrate | Lysine | 2 |
| B32 | thiol | glutarate | Lysine | 3 |
| B33 | aminoxy | PEG6 | Lysine | 3 |
| B34 | iodoacetamide | PEG6 | Lysine | 3 |
| B35 | Maleimide | EG2-EG2-Glu-C18 | Lysine | 3 |
| B36 | Maleimide | EG2-EG2-Glu-C18 | Lysine | 3 |
| B37 | Amine | Lys-EG2 | Lysine | 3 |
| B38 | N-hydroxysuccinimide | - | - | 1 |

B35:

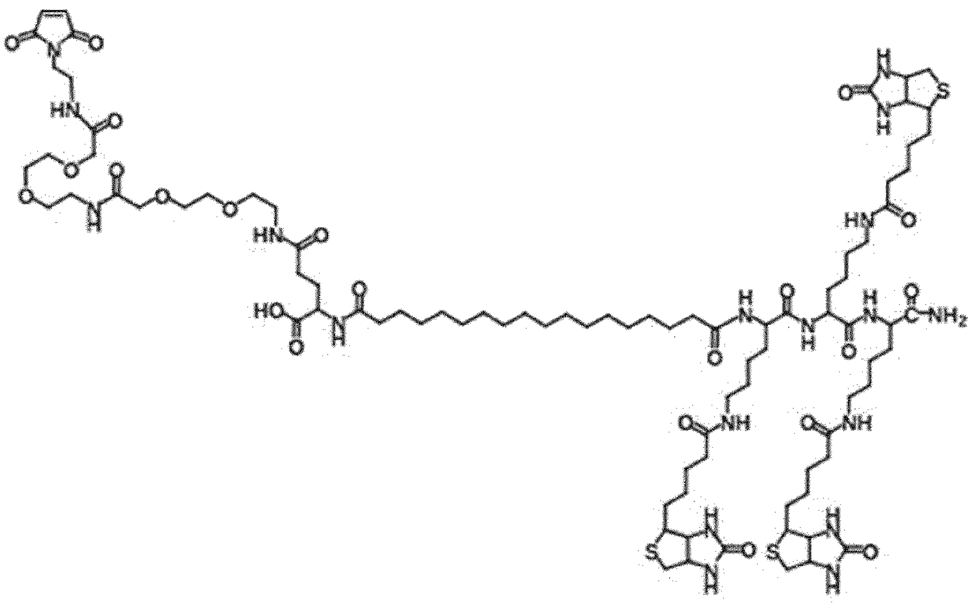

B36:

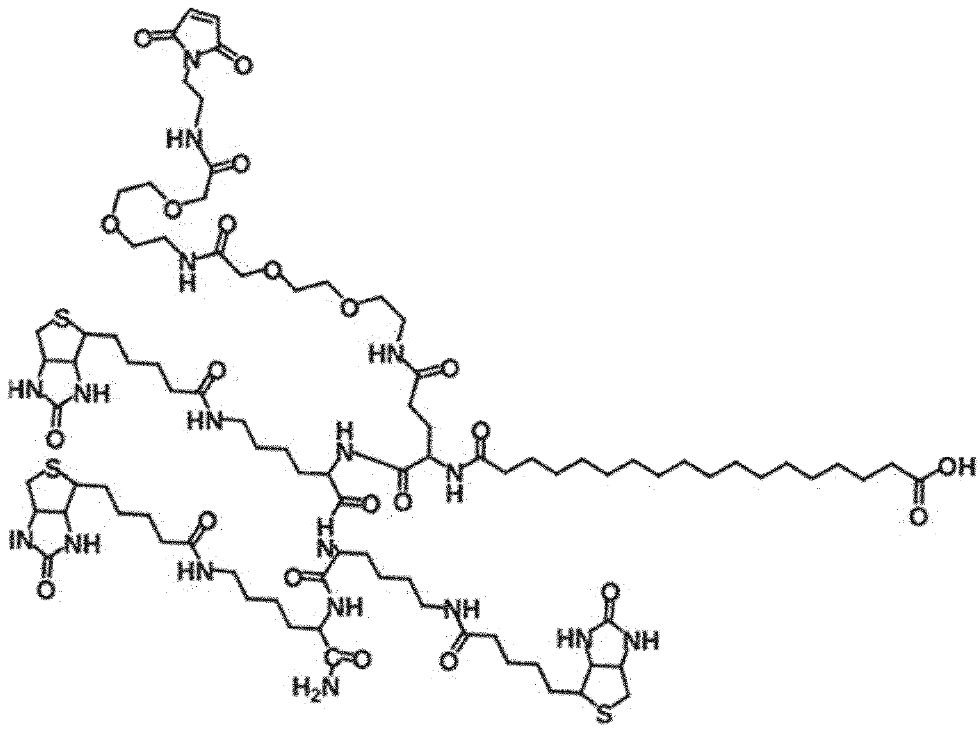

B37:

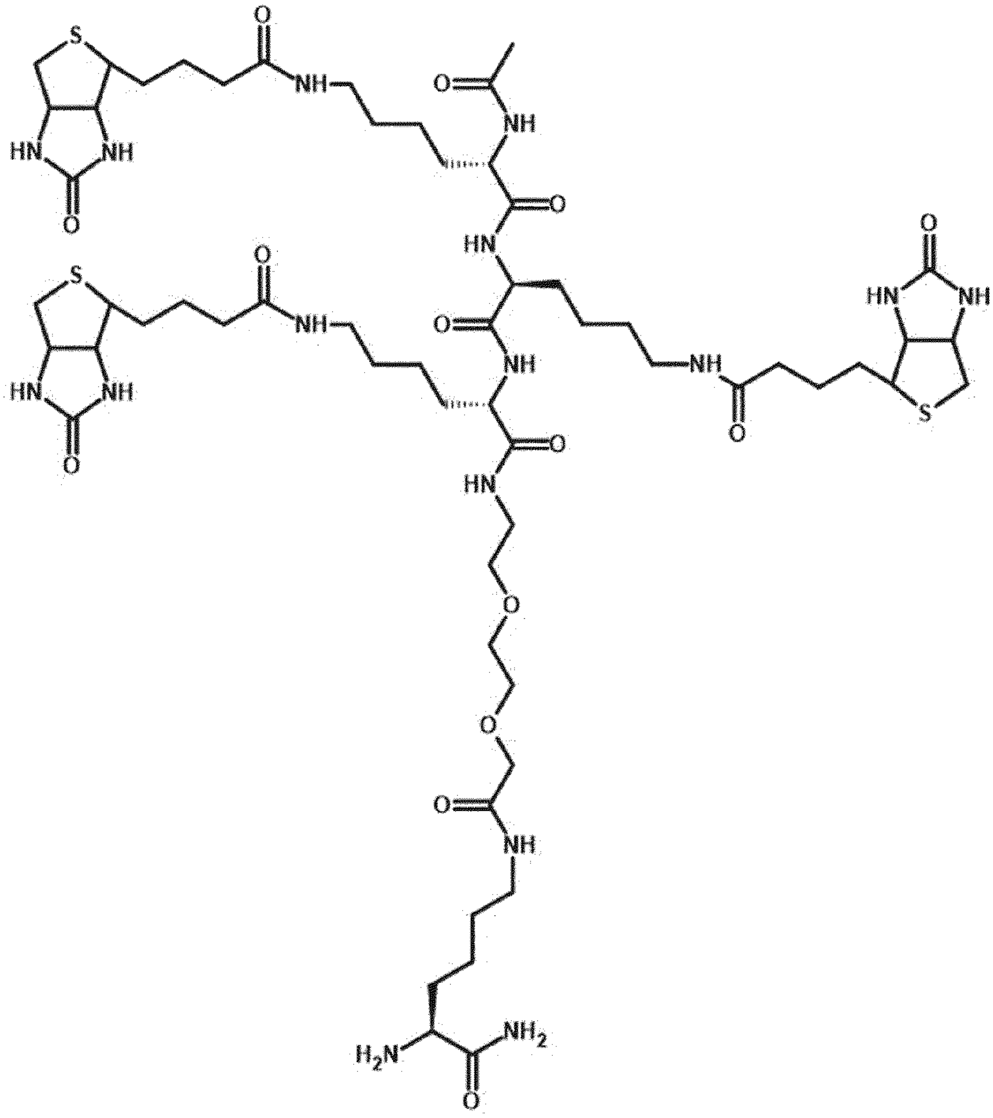

B38:

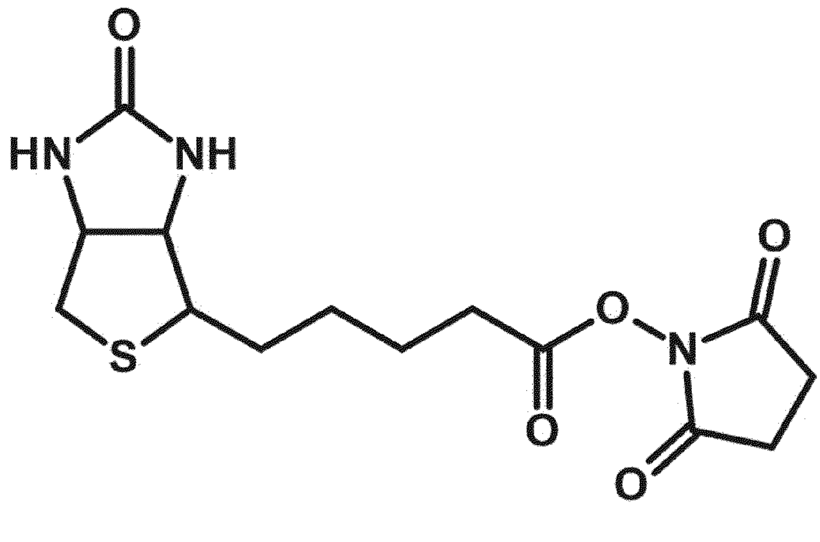

<Fatty Acid Moiety>

The fatty acid moiety may be prepared using methods known to the art, or a commercially obtained substance may be used.

As the fatty acid moiety, the fatty acid moieties of Table 5 below were used.

[Table 5]

| Fatty Acid Moiety | Designation |
|---|---|
| F1 | C16-NHS |
| F2 | C16-MAL |
| F3 | C18-NHS |
| F4 | C18-MAL |
| F5 | C16-Glu-NHS |
| F6 | C16-Glu-MAL |
| F7 | C18-Glu-NHS |
| F8 | C18-Glu-MAL |
| F9 | C18-Glu-EG2-NHS |
| F10 | C18-Glu-EG2-MAL |
| F11 | C18-Glu-EG2-EG2-NHS |
| F12 | C18-Glu-EG2-EG2-MAL |
| F13 | C20-Glu-EG2-EG2-NHS |
| F14 | C20-Glu-EG2-EG2-MAL |
| F15 | C18-Glu-EG2-EG2-TFP |
| F16 | C18-Glu-EG2-EG2-NPC |

F1:

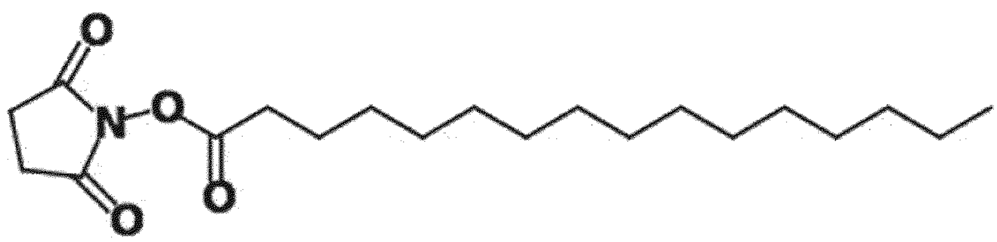

F2:

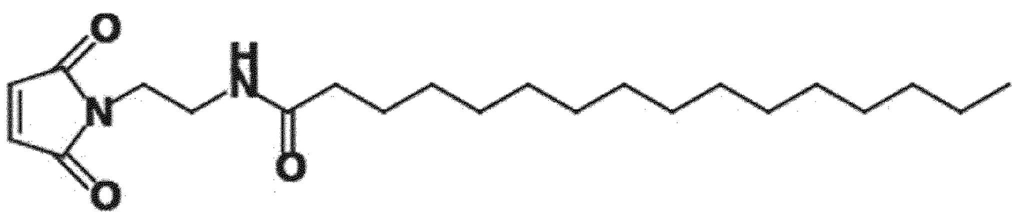

F3:

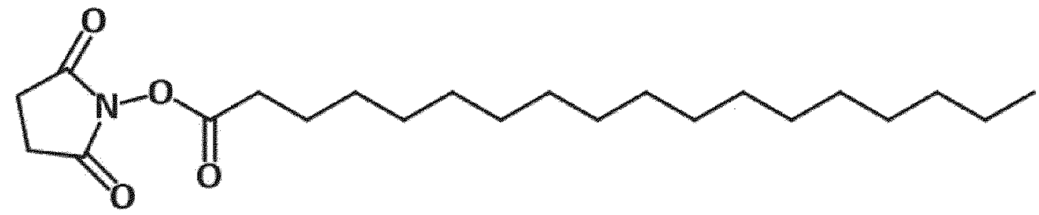

F4:

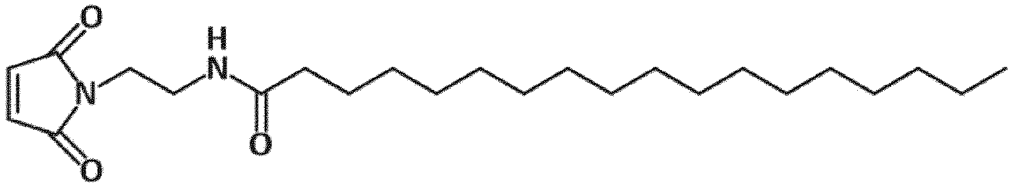

F5:

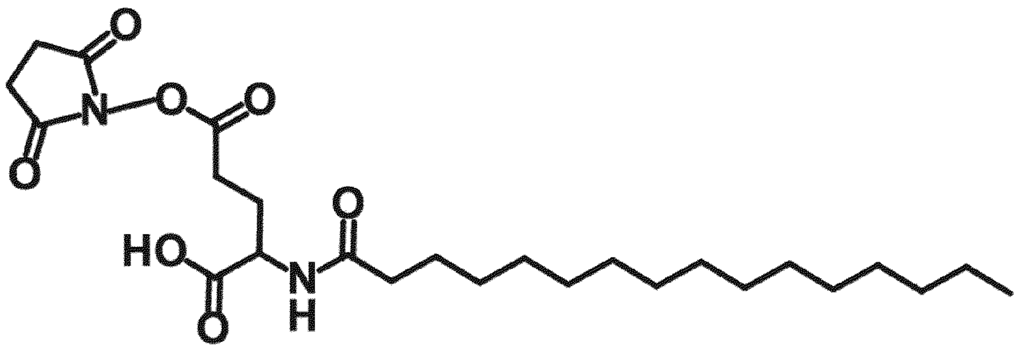

F6:

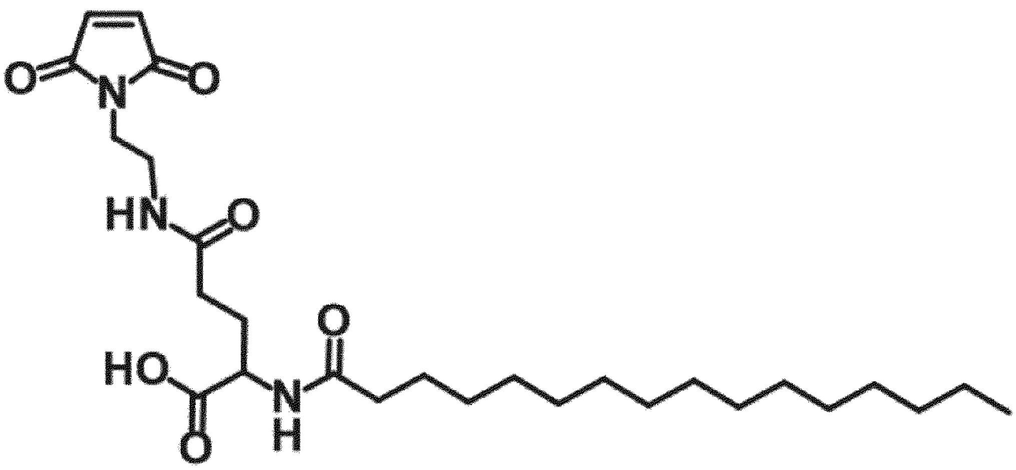

F7:

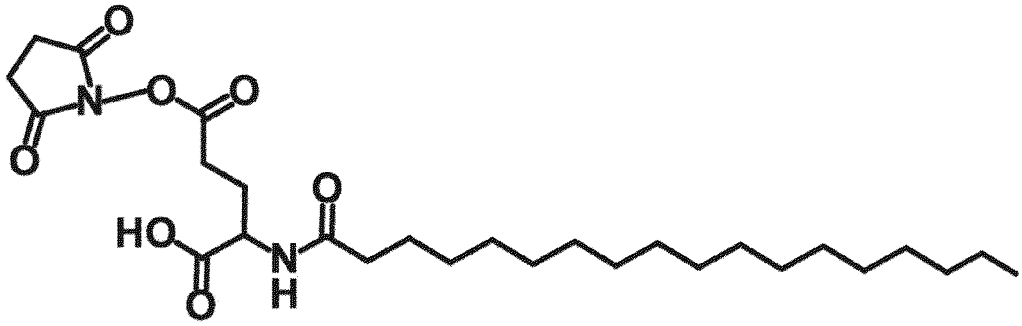

F8:

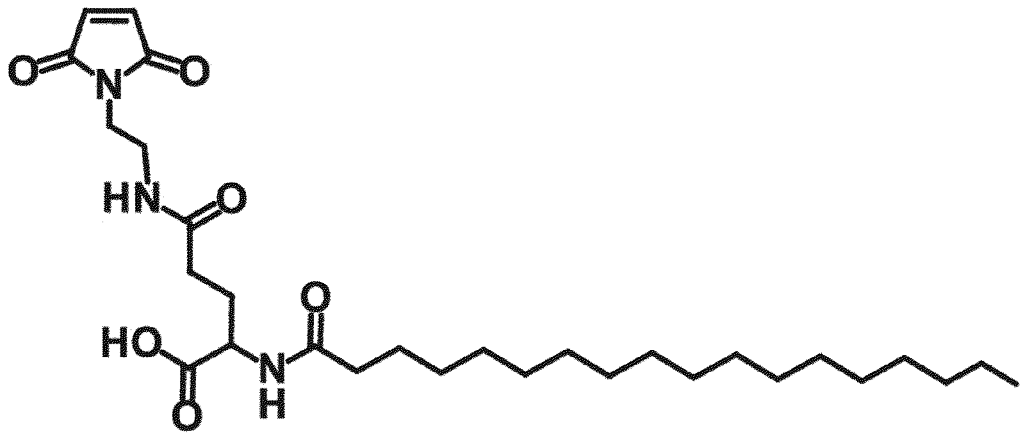

F9:

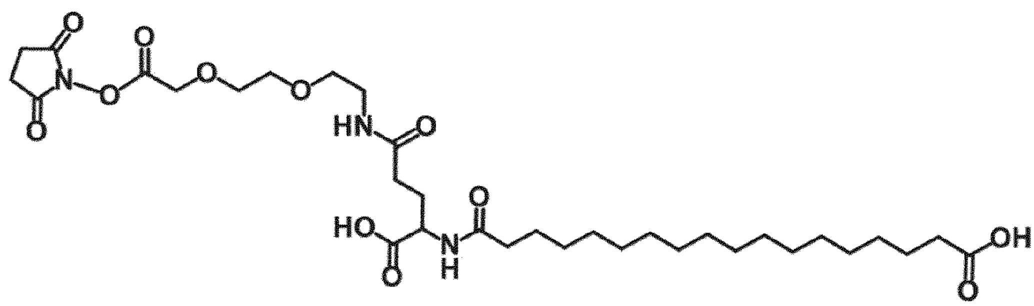

F10:

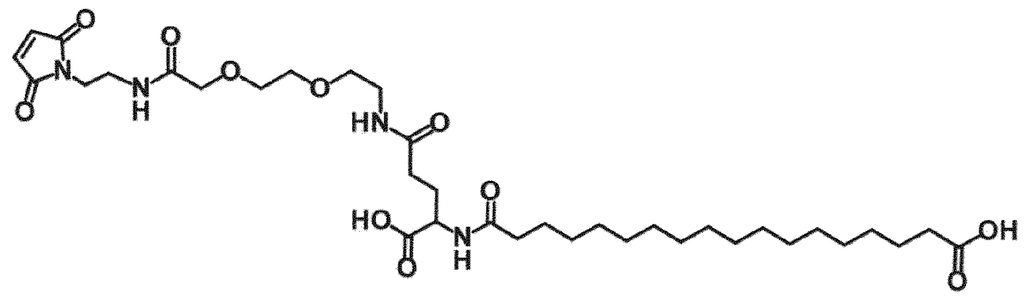

F11:

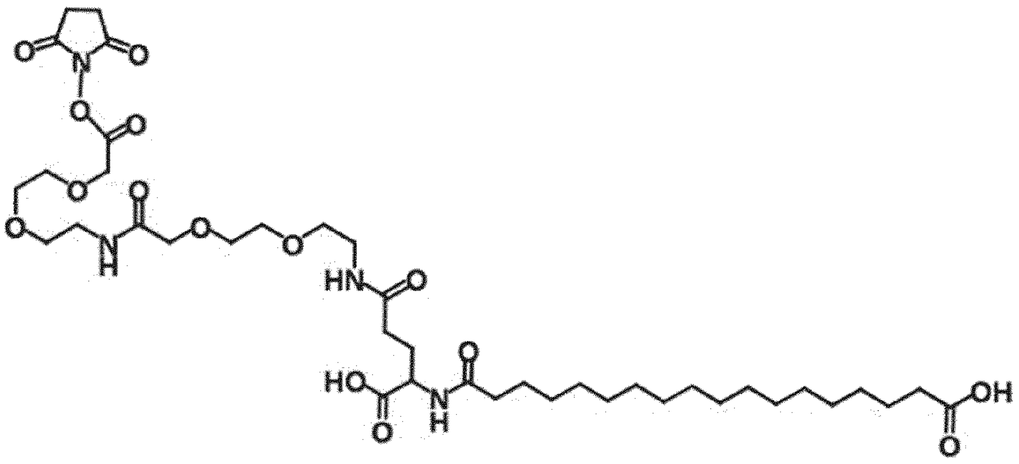

F12:

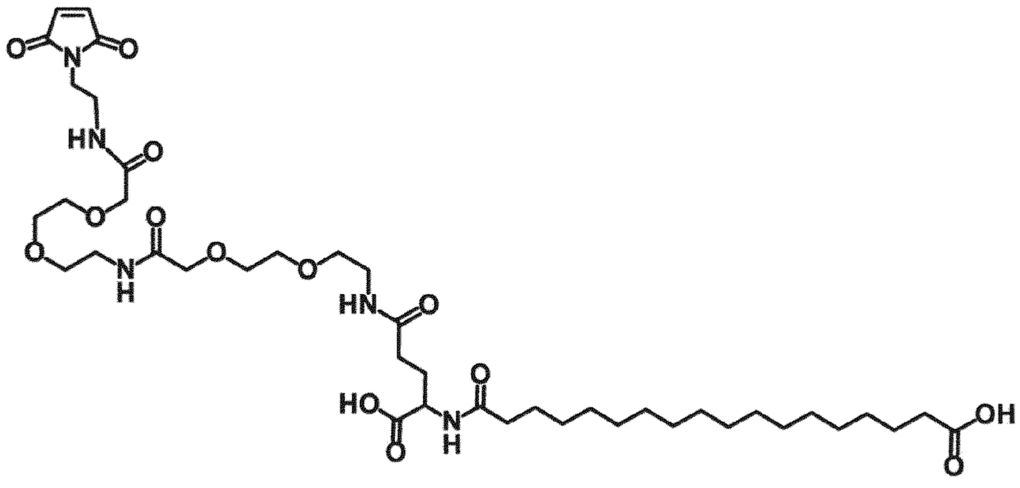

F13:

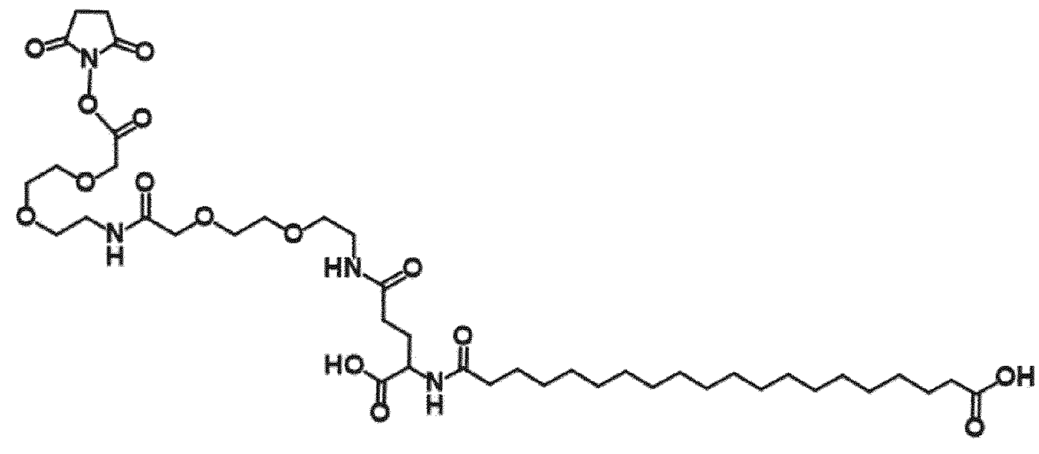

F14:

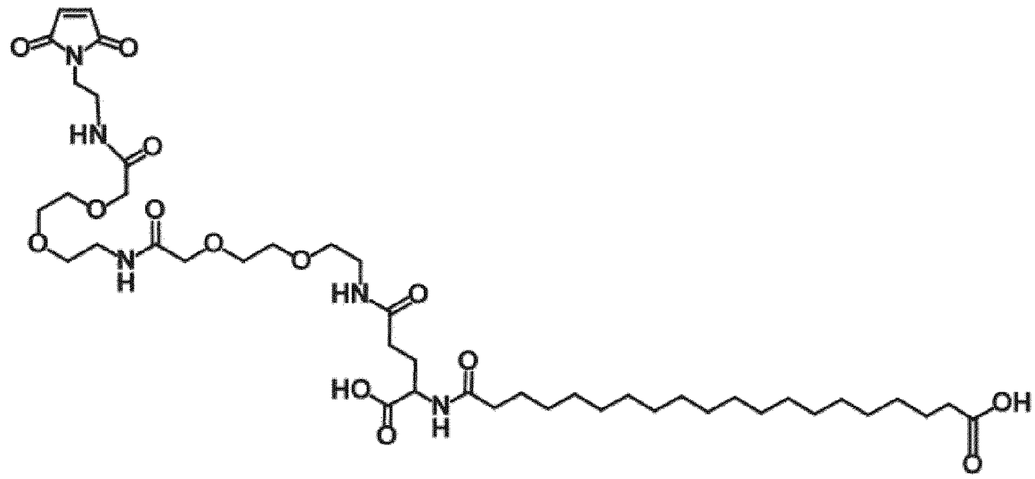

F15:

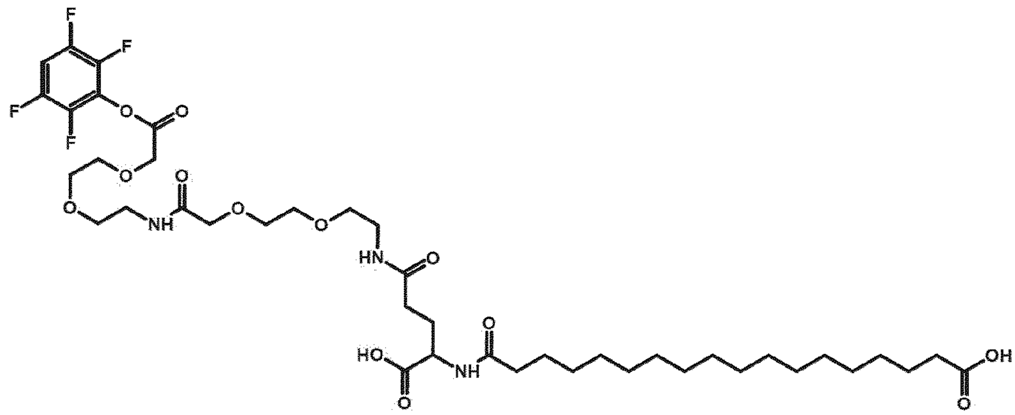

F16:

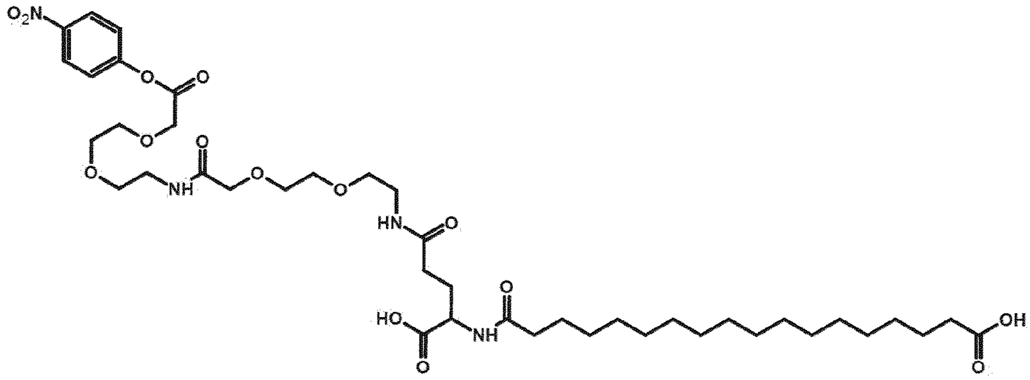

<Physiologically Active Substance (Polypeptide)>

The physiologically active substance (polypeptide) may be prepared using methods known to the art, or commercially obtained substances may be used. In the present invention, the sequences of the physiologically active substances bound to a biotin moiety, a fatty acid moiety, or a combination thereof are shown in Table 6 below.

[Table 6]

| Physiologically Active Substance (Polypeptide) | SEQ ID NO | Amino Acid Sequence |
|---|---|---|
| P1 | 1 | H(Aib)QGTFTSDYSKYLDEQAAKEFVQWLMNT |
| P2 | 2 | HAEGTFTSDVSSYLEGQAAKEFIAWLVKGR |
| P3 | 3 | HADGSFSDEMNTILDNLAARDFINWLIQTKITD |
| P4 | 4 | YAEGTFISDYSIAMDKIHQQDFVNWLLAQKGKKNDWKHNITQ |
| P5 | 5 | HGEGTFTSDLSKQMEEEAVRLFIEWLKNGGPSSGAPPPS |
| P6 | 6 | SVSEIQLMHNLGKHLNSMERVEWLRKKLQDVHNF |
| P7 | 7 | HSQGTFTSDYSKYLDSRRAQDFVQWLMNT |
| P8 | 8 | H(Aib)QGTFTSDYSKYLDEQAAKEFVQWLMNTC |
| P9 | 9 | HAEGTFTSDVSSYLEGQAAKEFIAWLVKGRC |
| P10 | 10 | HADGSFSDEMNTILDNLAARDFINWLIQTKITDC |
| P11 | 11 | YAEGTFISDYSIAMDKIHQQDFVNWLLAQKGKKNDWKHNITQC |
| P12 | 12 | HGEGTFTSDLSKQMEEEAVRLFIEWLKNGGPSSGAPPPSC |
| P13 | 13 | SVSEIQLMHNLGKHLNSMERVEWLRKKLQDVHNFC |
| P14 | 14 | HSQGTFTSDYSKYLDSRRAQDFVQWLMNTC |

(continued)

| Physiologically Active Substance (Polypeptide) | SEQ ID NO | Amino Acid Sequence |
|---|---|---|
| P15 | 15 | GIVEQCCTSICSLEQLENYCN |
| P16 | 16 | FVNQHLCGSHLVEALYLVCGERGFFYTPKT |
| P17 | 17 | GIVEQCCTSICSLYQLENYCN |
| P18 | 18 | HGEGTFTSDLSKQMEEEAVRLFIEWLKNGGPSSGAPP PSK |
| P19 | 19 | HGEGTFTSDLSKQMEEEAVRLFIEWLKNGGPSSGAPP PSKKK |
| P20 | 20 | H(Aib)EGTFTSDLSKQMEEEAVRLFIEWLKNGGPSSGA PPPSK |
| P21 | 21 | H(Aib)EGTFTSDLSKQMEEEAVRLFIEWLKNGGPSSGA PPPSKKK |
| P22 | 22 | KCNTATCATQRLANFLVHSSNNFGAILSSTNVGSNTY |
| P23 | 23 | KCNTATCATQRLANFLVHSSNNFGAILSSTNVGSNTY K |
| P24 | 24 | KCNTATCATQRLANFLVHSSNNFGPILPPTNVGSNTY |
| P25 | 25 | KCNTATCATQRLANFLVHSSNNFGPILPPTNVGSNTY K |
| P26 | 26 | KCNTATCATQRLADFLRHSSPNFGAIPSSTNVGSRTY |
| P27 | 27 | KCNTATCATQRLADFLRHSSPNFGAIPSSTNVGSRTYK |
| P28 | 28 | KCNTATCATQRLADFLRHSSNNFGAIPSSTNVGSRTY |
| P29 | 29 | KCNTATCATQRLADFLRHSSNNFGAIPSSTNVGSRTY K |
| P30 | 30 | RCNTATCATQRLADFLRHSSNNFGAIPSSTNVGSKTY |
| P31 | 31 | RCNTATCATQRLADFLRHSSNNFGAIPSSTNVGSKTY K |
| P32 | 32 | H(Aib)QGTFTSDKSKYLDERAAQDFVQWLLDGGPSS GAPPPS |
| P33 | 33 | H(Aib)QGTFTSDKSKYLDERAAQDFVQWLMDGGPSS GAPPPS |

(continued)

| Physiologically Active Substance (Polypeptide) | SEQ ID NO | Amino Acid Sequence |
|---|---|---|
| P34 | 34 | H(Aib)QGTFTSDKSKYLDKIAAQDFVQWLIDGGPSSG APPPS |
| P35 | 35 | H(Aib)QGTFTSDKSWYLDKIAAQDFVQWLLGGGPSS GAPPPS |
| P36 | 36 | H(Aib)QGTFTSDKSWYLDERAAQDFVQWLMGGGPSS GAPPPS |
| P37 | 37 | H(Aib)QGTFTSDKSKWLDKIAAQDFVQWLIGGGPSSG APPPS |
| P38 | 38 | H(Aib)EGTFTSDLSKQMEEEAVRLFIEWLKNGGPSSGA PPPSC |
| P39 | 39 | h(Aib)EGTFTSDLSKQMEEEAVRLFIEWLKNGGPSSGA PPPSC |
| P40 | 40 | H(Aib)QGTFTSDYSKYLDEQAAKEFVQWLMNTK |
| P41 | 41 | H(Aib)QGTFTSDYSKYLDEKRAKEFVQWLMNTC |
| P42 | 42 | MFPTIPLSRLFDNAMLRAHRLHQLAFDTYQEFEEAYI PKEQKISFLQNPQTSLCFSESIPTPSNREETQQKSNLEL LRISLLLIQSWLEPVQFLRSVFANSLVYGASDSNVYDL LKDLEEGIQTLMGRLEDGSPRTGQIFKQTYSKFDTNS HNDDALLKNYGLLYCFRKDMDKVETFLRIVQCRSVE GSCGF |

<Embodiment: Preparation of a physiologically active substance combined with a biotin moiety, a fatty acid moiety, or a combination thereof>

[Preparation Example]

Using DMSO solution with 0.3% trimethylamine (TEA, Sigma) added as the reaction solvent, molar ratio mixtures of 1:X (1: 0.5~30) between the polypeptides of Table 6 and the biotin moieties of Tables 3 through 4 were reacted for at least 30 minutes each at room temperature. Thereafter, molar ratio mixtures of 1:Y (1: 0.5~20) between the polypeptide-biotin moiety mixtures and the fatty acid moieties of Table 5 were prepared and reacted for at least 90 minutes each at room temperature. The reactions were stopped by adding 1% Trifluoroacetic acid solution of the same volume as the volume of each mixture.

[Isolation, Purification and Confirmation]

The reaction products were isolated and purified using reverse phase high performance liquid chromatography. As the column, a SUPERSIL ODS-1 column (10x250mm, 5um, LB Science, South Korea) was used. The mobile phase condition was changed linearly while maintaining a flow rate of 4.7ml/min with 30-50% Solvent B (acetonitrile with 0.1% TFA added) and Solvent A (distilled water with 0.1% TFA added). Monitoring with a UV absorption spectrometer at 280nm, peaks detected between 10 minutes and 20 minutes were collected. The collected peaks were concentrated and purified using ultracentrifugal filters having an appropriate molecular weight cut-off, after volatilizing organic solvents and TFA under vacuum. The purity of the purified substances was confirmed using the HPLC analysis method. Analysis was carried out at a constant temperature near room temperature using a Gemini C18 column (4.6 x 250mm, 5um; Phenomenex, CA, USA). Analysis was carried out using the gradient elution method at a flow rate of 1mL/min using a mobile phase comprised of trifluoroacetic acid solution: acetonitrile mixture (at carrying mix ratios). UV absorbance was observed at 280nm.

<Embodiment: Polypeptide combined with a biotin moiety, a fatty acid moiety or a combination thereof>.

The substances stated above were used as the biotin moiety, fatty acid moiety and polypeptide. Methods known to the art or the method of the above embodiment was used for binding the polypeptide to the biotin moiety, fatty acid moiety or a combination thereof.

The polypeptides bound to a biotin moiety, fatty acid moiety or a combination thereof are as shown in Table 7 below. (Here, the molecular weights represent the measured molecular weights or the theoretical molecular weights).

[Table 7]

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 1 | 12 | HGEGTFTSDLSKQMEE EAVRLFIEWLKNGGPSS GAPPPSC | B1 | C40 | - | - | 4744.5 |
| 2 | 12 | HGEGTFTSDLSKQMEE EAVRLFIEWLKNGGPSS | B2 | C40 | - | - | 5167.1 |
| | | GAPPPSC | | | | | |
| 3 | 12 | HGEGTFTSDLSKQMEE EAVRLFIEWLKNGGPSS GAPPPSC | B3 | C40 | - | - | 5521.6 |
| 4 | 8 | H(Aib)QGTFTSDYSKYL DEQAAKEFVQWLMNT C | B1 | C30 | - | - | 3963.5 |
| 5 | 8 | H(Aib)QGTFTSDYSKYL DEQAAKEFVQWLMNT C | B2 | C30 | - | - | 4386.1 |
| 6 | 8 | H(Aib)QGTFTSDYSKYL DEQAAKEFVQWLMNT C | B3 | C30 | - | - | 4740.6 |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 7 | 13 | SVSEIQLMHNLGKHLN SMERVEWLRKKLQDV HNFC | B1 | C35 | - | - | 4675.4 |
| 8 | 13 | SVSEIQLMHNLGKHLN SMERVEWLRKKLQDV HNFC | B2 | C35 | - | - | 5098.0 |
| 9 | 13 | SVSEIQLMHNLGKHLN SMERVEWLRKKLQDV HNFC | B3 | C35 | - | - | 5452.5 |
| 10 | 5 | HGEGTFTSDLSKQMEE EAVRLFIEWLKNGGPSS GAPPPS | B4 | K27 | - | - | 5613.5 |
| 11 | 12 | HGEGTFTSDLSKQMEE | B5 | C40 | - | - | 5857.0 |
| | | EAVRLFIEWLKNGGPSS GAPPPSC | | | | | |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 12 | 15 | GIVEQCCTSICSLEQLENYCN (A chain) | B4 | B-chain K29 | - | - | 7200.9 |
| | 16 | FVNQHLCGSHLVEALYLVCGERGFFYTPKT (B chain) | | | - | - | |
| 13 | 15 | GIVEQCCTSICSLEQLENYCN (A chain) | B4 | F1/K29 of the B chain | - | - | 8627.8 |
| | 16 | FVNQHLCGSHLVEALYLVCGERGFFYTPKT (B chain) | | | - | - | |
| 14 | 12 | HGEGTFTSDLSKQMEEEAVRLFIEWLKNGGPSSGAPPPSC | B3 | C40 | F1 | K27 | 5759.4 |
| 15 | 12 | HGEGTFTSDLSKQMEEEAVRLFIEWLKNGGPSSGAPPPSC | B38 | K12, K27 | F2 | C40 | 5120.9 |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 16 | 12 | HGEGTFTSDLSKQMEE EAVRLFIEWLKNGGPSS GAPPPSC | B3 | C40 | F11 | K27 | 6237.2 |
| 17 | 12 | HGEGTFTSDLSKQMEE EAVRLFIEWLKNGGPSS GAPPPSC | B38 | K12, K27 | F12 | C40 | 5598.6 |
| 18 | 12 | HGEGTFTSDLSKQMEE EAVRLFIEWLKNGGPSS | B35 | C40 | - | - | 6208.4 |
| | | GAPPPSC | | | | | |
| 19 | 12 | HGEGTFTSDLSKQMEE EAVRLFIEWLKNGGPSS GAPPPSC | B36 | C40 | - | - | 6208.4 |
| 20 | 18 | HGEGTFTSDLSKQMEE EAVRLFIEWLKNGGPSS GAPPPSK | B37 | K40 | - | - | 5565.4 |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 21 | 19 | HGEGTFTSDLSKQMEE EAVRLFIEWLKNGGPSS GAPPPSKKK | B | K39, K40, K41 | - | - | 5251.1 |
| 22 | 19 | HGEGTFTSDLSKQMEE EAVRLFIEWLKNGGPSS GAPPPSKKK | B | K39, K40, K41 | F11 | K27 | 6241.6 |
| 23 | 19 | HGEGTFTSDLSKQMEE EAVRLFIEWLKNGGPSS GAPPPSKKK | B | K39, K40, K41 | F11 | K12 | 6241.6 |
| 24 | 20 | H(Aib)EGTFTSDLSKQM EEEAVRLFIEWLKNGG PSSGAPPPSK | B37 | K40 | - | - | 5278.1 |
| 25 | 21 | H(Aib)EGTFTSDLSKQM EEEAVRLFIEWLKNGG PSSGAPPPSKKK | B | K39, K40, K41 | - | - | 5279.0 |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 26 | 21 | H(Aib)EGTFTSDLSKQM EEEAVRLFIEWLKNGG PSSGAPPPSKKK | B | K39, K40, K41 | F11 | K27 | 6241.6 |
| 27 | 21 | H(Aib)EGTFTSDLSKQM | B | K39, K40, | F11 | K12 | 5759.0 |
| | | EEEAVRLFIEWLKNGG PSSGAPPPSKKK | | K41 | | | |
| 28 | 8 | H(Aib)QGTFTSDYSKYL DEQAAKEFVQWLMNT C | B2 | K12 | C30 | F6 | 5259.0 |
| 29 | 8 | H(Aib)QGTFTSDYSKYL DEQAAKEFVQWLMNT C | B5 | C30 | K12 | F5 | 4800.0 |
| 30 | 22 | KCNTATCATQRLANFL VHSSNNFGAILSSTNV GSNTY | B38 | K1 | - | - | 4129.6 |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 31 | 22 | KCNTATCATQRLANFL VHSSNNFGAILSSTNV GSNTY | B39 | K1 | - | - | 5842.7 |
| 32 | 23 | KCNTATCATQRLANFL VHSSNNFGAILSSTNV GSNTYK | B2 | K38 | F11 | K1 | 5627.5 |
| 33 | 24 | KCNTATCATQRLANFL VHSSNNFGAILSSTNV GSNTY | B38 | K1 | - | - | 4175.7 |
| 34 | 24 | KCNTATCATQRLANFL VHSSNNFGPILPPTNVG SNTY | B39 | K1 | - | - | 5888.8 |
| 35 | 25 | KCNTATCATQRLANFL VHSSNNFGPILPPTNVG SNTYK | B2 | K38 | F11 | K1 | 5673.6 |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 36 | 26 | KCNTATCATQRLADFL RHSSPNFGAIPSSTNVG SRTY | B38 | K1 | - | - | 4196.7 |
| 37 | 26 | KCNTATCATQRLADFL RHSSPNFGAIPSSTNVG SRTY | B39 | K1 | - | - | 5909.8 |
| 38 | 27 | KCNTATCATQRLADFL RHSSPNFGAIPSSTNVG SRTYK | B2 | K38 | F11 | K1 | 5694.5 |
| 39 | 28 | KCNTATCATQRLADFL RHSSNNFGAIPSSTNVG SRTY | B38 | K1 | - | - | 4213.7 |
| 40 | 28 | KCNTATCATQRLADFL RHSSNNFGAIPSSTNVG SRTY | B39 | K1 | - | - | 5926.8 |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 41 | 29 | KCNTATCATQRLADFL RHSSNNFGAIPSSTNVG SRTYK | B2 | K38 | F11 | K1 | 5711.5 |
| 42 | 30 | RCNTATCATQRLADFL RHSSNNFGAIPSSTNVG SKTY | B38 | K35 | - | - | 4213.7 |
| 43 | 30 | RCNTATCATQRLADFL RHSSNNFGAIPSSTNVG SKTY | B39 | K35 | - | - | 5926.8 |
| 44 | 31 | RCNTATCATQRLADFL RHSSNNFGAIPSSTNVG SKTYK | B2 | K35 | F11 | K38 | 5711.5 |
| 45 | 32 | H(Aib)QGTFTSDKSKYL DERAAQDFVQWLLDG GPSSGAPPS | B38 | K12 | - | - | 6112.9 |
| 46 | 33 | H(Aib)QGTFTSDKSKYL DERAAQDFVQWLMDG GPSSGAPPS | B39 | K12 | - | - | 6131.0 |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 47 | 34 | H(Aib)QGTFTSDKSKYL DKIAAQDFVQWLIDGG PSSGAPPPS | B38 | K10 | - | - | 6069.0 |
| 48 | 35 | H(Aib)QGTFTSDKSWY LDKIAAQDFVQWLLG GGPSSGAPPPS | B39 | K10 | - | - | 6069.0 |
| 49 | 36 | H(Aib)QGTFTSDKSWY LDERAAQDFVQWLMG GGPSSGAPPPS | B3 | K12 | F11 | K40 | 6142.0 |
| 50 | 37 | H(Aib)QGTFTSDKSKW LDKIAAQDFVQWLIGG GPSSGAPPPS | B3 | K12 | F12 | C40 | 6220.2 |
| 51 | 20 | H(Aib)EGTFTSDLSKQM EEEAVRLFIEWLKNGG PSSGAPPPSK | B37 | K40 | F11 | K27 | 6309.3 |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 52 | 20 | H(Aib)EGTFTSDLSKQM EEEAVRLFIEWLKNGG PSSGAPPPSK | B38 | K12, K27 | F11 | K40 | 5511.3 |
| 53 | 38 | H(Aib)EGTFTSDLSKQM EEEAVRLFIEWLKNGG PSSGAPPPSC | B38 | K12, K27 | F12 | C40 | 5626.5 |
| 54 | 39 | H(Aib)EGTFTSDLSKQM EEEAVRLFIEWLKNGG PSSGAPPPSC | B38 | K12, K27 | F12 | C40 | 5597.4 |
| 55 | 8 | H(Aib)QGTFTSDYSKYL DEQAAKEFVQWLMNT C | B38 | K12 | F12 | C30 | 4591.3 |
| 56 | 40 | H(Aib)QGTFTSDYSKYL DEQAAKEFVQWLMNT K | B38 | K12 | F11 | K30 | 4475.1 |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 57 | 41 | H(Aib)QGTFTSDYSKYL DEKRAKEFVQWLMNT C | B38 | K12 | F12 | C30 | 4647.6 |
| 58 | 8 | H(Aib)QGTFTSDYSKYL DEQAAKEFVQWLMNT C | B1 | C30 | F16 | K12 | 4679.4 |
| 59 | 8 | H(Aib)QGTFTSDYSKYL DEQAAKEFVQWLMNT C | B38 | K12 | F14 | C30 | 4619.4 |
| 60 | 22 | KCNTATCATQRLANFL VHSSNNFGAILSSTNV GSNTY | B38 | K1 | F11 | K1 | 4845.5 |
| 61 | 24 | KCNTATCATQRLANFL VHSSNNFGPILPPTNVG SNTY | B38 | K1 | F11 | K1 | 4891.6 |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 62 | 26 | KCNTATCATQRLADFL RHSSPNFGAIPSSTNVG SRTY | B38 | K1 | F11 | K1 | 4912.6 |
| 63 | 28 | KCNTATCATQRLADFL RHSSNNFGAIPSSTNVG SRTY | B38 | K1 | F11 | K1 | 4929.6 |
| 64 | 30 | RCNTATCATQRLADFL RHSSNNFGAIPSSTNVG SKTY | B38 | K35 | F11 | K1 | 4929.6 |
| 65 | 15 | GIVEQCCTSICSLEQLE NYCN (A chain) | B38 | B-chain K29 | - | - | 6000.3 |
| 66 | 16 | FVNQHLCGSHLVEALY LVCGERGFFYTPKT (B chain) | B38<br>B38 | B-chain K29<br>B-chain K29 | -<br>F11 | -<br>B chain F1 | 6000.3<br>6716.2 |
| | 15 | GIVEQCCTSICSLEQLE NYCN (A chain) | | | | | |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 67 | 16 | FVNQHLCGSHLVEALY LVCGERGFFYTPKT (B chain) | B38<br>B38 | B-chain K29<br>K13, K26, K27 | F11<br>- | B chain F1<br>- | 6716.2 |
| | 13 | SVSEIQLMHNLGKHLN SMERVEWLRKKLQDV HNF | | | | | 4796.7 |
| 68 | 42 | MFPTIPLSRLFDNAML RAHRLHQLAFDTYQEF EEAYIPKEQKISFLQNP QTSLCFSESIPTPSNREE | B38 | Lys random | - | - | |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| | | TQQKSNLELLRISLLLI<br>QSWLEPVQFLRSVFAN<br>SLVYGASDSNVYDLLK<br>DLEEGIQTLMGRLEDG<br>SPRTGQIFKQTYSKFDT<br>NSHNDDALLKNYGLL<br>YCFRKDMDKVETFLRI<br>VQCRSVEGSCGF | | | | | |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 69 | 42 | MFPTIPLSRLFDNAML RAHRLHQLAFDTYQEF EEAYIPKEQKISFLQNP QTSLCFSESIPTPSNREE TQQKSNLELLRISLLLI QSWLEPVQFLRSVFAN SLVYGASDSNVYDLLK DLEEGIQTLMGRLEDG SPRTGQIFKQTYSKFDT NSHNDDALLKNYGLL YCFRKDMDKVETFLRI VQCRSVEGSCGF | B38 | Lys random | F16 | Lys random | |
| 70 | 5 | HGEGTFTSDLSKQMEE EAVRLFIEWLKNGGPSS GAPPPS | B38 | K12, K27 | - | - | |

(continued)

| Conjugate SEQ ID NO | (Polypeptide) | | Biotin Moiety | | Fatty Acid Moiety | | Molecular Weight (g/mol) |
|---|---|---|---|---|---|---|---|
| | Order No. | Order | No. | Combination Location | No. | Combination Location | |
| 71 | 12 | HGEGTFTSDLSKQMEE EAVRLFIEWLKNGGPSS GAPPPSC | - | - | F1 | C40 | |
| 72 | 12 | HGEGTFTSDLSKQMEE EAVRLFIEWLKNGGPSS GAPPPSC | - | - | F12 | C40 | |
| (B in the table above refers to native biotin.) | | | | | | | |

<Embodiment. Formulation of Physiologically Active Substances Combined with Biotin Moieties>

<Formulation>

The conjugates, which are polypeptides (physiologically active substances) bound to a biotin moiety, fatty acid moiety or a combination thereof prepared in accordance with the above example, were formulated by dissolving in Hanks Balanced Salt Solution (HBSS) with specific compositions of excipients.

Formulation Embodiment 1. Preparation of dosage forms containing conjugate 3, one bile acid and propyl gallate, and measurement of Caco-2 cell membrane permeability of bioactive substances in the dosage form

Conjugate 3, which is a physiologically active substance (polypeptide) bound to a biotin moiety, was formulated by dissolving in Hanks Balanced Salt Solution (HBSS) with the compositions of excipients shown in Table 8, and the Caco-2 cell membrane permeation rate was measured.

First, to form a Caco-2 cell monolayer, $1.5 \times 10^5$ cells were dispensed per well in a 12-transwell plate, and cultured for 3 to 4 weeks under 37° C $CO_2$ conditions. For the first week, the culture medium was changed once every 2 days, and thereafter, culturing was performed changing the culture medium at 3-day intervals. Cells between 3 and 4 weeks after seeding were used for the experiment. To verify formation of a cell monolayer, the TEER value and Lucifer yellow values were measured, using only cell monolayers where the TEER value was $300\Omega \cdot cm^2$ or greater and the measured value of Lucifer yellow permeability was within 3%. The transwells to be used in the experiments were washed with transport medium (HBSS) then cultured for 1 hour in an incubator at 37°C $CO_2$, after which 200uL each of the formulation comprising the prepared agent and excipient were added to the apical side, treating the basolateral side with 1mL transport medium not containing the agent. This was followed by incubation for 2 hours in an incubator at 37°C $CO_2$. 2 hours later, samples of 1mL each were taken from the basolateral side, and the permeability coefficient (Papp value) was measured using the enzyme-linked immunoassay (ELISA) method. The permeability coefficient (Papp value) was calculated as follows, and the results of analysis are as shown in Table 8.

$$[Papp(10^{-6}, cm/s) = (dC_r/d_t) \text{ x } V_r / (A \text{ x } C_0)\ ]$$

(* $dC_r$- concentration of permeated sample, dt- drug treatment time, $V_r$- basolateral volume, A - transwell area, $C_0$- initially applied drug concentration)

[Table 8]

| Test Substance | | Excipient | | | | Weight ratio (pharmaceutically active substance: excipient) | Transmission Coefficient (Permeability Coefficient) (Papp) |
|---|---|---|---|---|---|---|---|
| Conjugate | Volume (ug/mL) | Type | Volume (ug/mL) | Excipient volume (ug/mL) | Excipient weight ratio (bile acid: PG) | | |
| Zygote 3 | 27.6 | - | - | - | - | - | 0.6 |
| | 27.6 | sDC | 41.5 | 41.5 | - | 1:1.5 | 2.1 |
| | 27.6 | sDC | 124.4 | 124.4 | - | 1:4.5 | 13.3 |
| | 27.6 | sDC | 41.5 | 62.7 | 2:1 | 1:2.3 | 1.9 |
| | | PG | 21.2 | | | | |
| | 27.6 | sCA | 43.1 | 43.1 | - | 1:1.6 | 0.7 |
| | 27.6 | sCA | 430.6 | 430.6 | - | 1:15.6 | 3.0 |
| | 27.6 | sCA | 43.1 | 64.3 | 2:1 | 1:2.3 | 0.9 |
| | | PG | 21.2 | | | | |
| | 27.6 | sGC | 48.8 | 48.8 | - | 1:1.8 | 1 |

(continued)

| Test Substance | | Excipient | | | | Weight ratio (pharmaceutically active substance: excipient) | Transmission Coefficient (Permeability Coefficient) (Papp) |
|---|---|---|---|---|---|---|---|
| Conjugate | Volume (ug/mL) | Type | Volume (ug/mL) | Excipient volume (ug/mL) | Excipient weight ratio (bile acid: PG) | | |
| | 27.6 | sGC | 1462.8 | 1462.8 | - | 1:53 | 10.3 |
| | 27.6 | sGC | 48.8 | 70 | 2.3:1 | 1:2.5 | 0.9 |
| | | PG | 21.2 | | | | |
| | 27.6 | sTC | 53.8 | 53.8 | - | 1:1.9 | 3.8 |
| | 27.6 | sTC | 1613.1 | 1613.1 | - | 1:58.4 | 11.4 |
| | 27.6 | sTC | 53.8 | 75 | 2.5:1 | 1:2.7 | 1.1 |
| | | PG | 21.2 | | | | |
| | 27.6 | sCDC | 41.5 | 41.5 | - | 1:1.5 | 7.2 |
| | 27.6 | sCDC | 41.5 | 62.7 | 2:1 | 1:2.3 | 0.9 |
| | 27.6 | PG | 21.2 | | | | |
| | 27.6 | sUDC | 41.5 | 41.5 | - | 1:1.5 | 6.0 |
| | 27.6 | sUDC | 414.6 | 414.6 | - | 1:15 | 6.3 |
| | 27.6 | sUDC | 41.5 | 62.7 | 2:1 | 1:2.3 | 1.6 |
| | | PG | 21.2 | | | | |
| (*PG: Propyl gallate, sCDC: Sodium chenodeoxycholate, sDC: Sodium deoxycholate, sCA: Sodium cholate, sUDC: Sodium ursodeoxycholate, sGC: Sodium glycocholate hydrate, sTC: Sodium taurocholate) | | | | | | | |

Formulation Embodiment 2. Preparation of formulation comprising a conjugate (3, 17, 52, 70) and one bile acid, and measurement of Caco-2 cell membrane permeation rate of physiologically active substance in formulation

Physiologically active substances bound to a biotin moiety, fatty acid moiety or a combination thereof, prepared in accordance with the above example, were formulated by dissolving in Hanks Balanced Salt Solution (HBSS) with the compositions of excipients shown in Table 9, and the Caco-2 cell membrane permeation rate was measured.

First, to form a Caco-2 cell monolayer, $1.5 \times 10^5$ cells were dispensed per well in a 12-transwell plate, and cultured for 3 to 4 weeks under 37°C $CO_2$ conditions. For the first week, the culture medium was changed once every 2 days, and thereafter, culturing was performed changing the culture medium at 3-day intervals. Cells between 3 and 4 weeks after seeding were used for the experiment. To verify formation of a cell monolayer, the TEER value and Lucifer yellow values were measured, using only cell monolayers where the TEER value was $300\Omega \cdot cm^2$ or greater and the measured value of Lucifer yellow permeability was within 3%. The transwells to be used in the experiments were washed with transport medium (HBSS) then cultured for 1 hour in an incubator at 37°C $CO_2$, after which 200uL each of the formulation comprising the prepared agent and excipient were added to the apical side, treating the basolateral side with 1mL transport medium not containing the agent. This was followed by incubation for 2 hours in an incubator at 37°C $CO_2$. 2 hours later, samples of 1mL each were taken from the basolateral side, and the permeability coefficient (Papp value) was measured using the enzyme-linked immunoassay (ELISA) method. The permeability coefficient (Papp value) was calculated as follows, and the results of analysis are as shown in Table 9.

$$[\ Papp(10^{-6}, cm/s) = (dC_r/d_t) \times V_r / (A \times C_0)\ ]$$

(* $dC_r$- concentration of permeated sample, dt- drug treatment time, $V_r$- basolateral volume, A - transwell area, $C_0$- initially applied drug concentration)

[Table 9]

| Test Substance | | Excipient | | Weight Ratio (Pharmaceutically Active Substance: Excipient) | Transmission Coefficient (Permeability Coefficient) (Papp, fold) |
|---|---|---|---|---|---|
| Test Substance | Volume (ug/mL) | Type | Volume (ug/mL) | | |
| Polypeptide SEQ ID | 214.5 | - | - | - | 1 |
| NO: 5 | | | | | |
| Polypeptide SEQ ID NO: 5 | 214.5 | sCDC | 41.5 | 5.2:1 | 2 |
| Polypeptide SEQ ID NO: 5 | 214.5 | sUDC | 41.5 | 5.2:1 | 1 |
| Conjugate 3 | 27.6 | - | - | - | 64 |
| Conjugate 3 | 27.6 | sCDC | 41.5 | 1:1.5 | 358 |
| Conjugate 3 | 27.6 | sUDC | 41.5 | 1:1.5 | 301 |
| Conjugate 17 | 280.0 | - | - | - | 155 |
| Conjugate 17 | 280.0 | sCDC | 41.5 | 6.7:1 | 272 |
| Conjugate 52 | 275.6 | - | - | - | 133 |
| Conjugate 52 | 275.6 | sCDC | 41.5 | 6.6:1 | 225 |
| Conjugate 70 | 232.0 | - | - | - | 110 |
| Conjugate 70 | 232.0 | sCDC | 41.5 | 5.6:1 | 147 |
| (PG: Propyl gallate, sCDC: Sodium chenodeoxycholate, sDC: Sodium deoxycholate, sUDC: Sodium ursodeoxycholate) | | | | | |

Formulation Embodiment 3. Preparation of formulation comprising a conjugate (68, 69), one bile acid (sodium chenodeoxycholate) and propyl gallate, and measurement of Caco-2 cell membrane permeation rate of physiologically active substance in formulation

Physiologically active substances bound to a biotin moiety, fatty acid moiety or a combination thereof, prepared in accordance with the above example, were formulated by dissolving in Hanks Balanced Salt Solution (HBSS) with the compositions of excipients shown in Table 11, and the Caco-2 cell membrane permeation rate was measured.

First, to form a Caco-2 cell monolayer, $7 \times 10^4$ cells were dispensed per well in a 96-transwell plate, and cultured in a $CO_2$ incubator under 37°C temperature conditions. 24 hours later, the culture fluid was removed from each well and washed with HBSS, followed by addition of 100uL of the prepared drug and the drug comprising excipient, and culturing in a $CO_2$ incubator at 37°C. After 8 minutes, each well was washed with PBS and treated with 100uL 10% formalin, followed by reacting at room temperature. After 10 minutes, each well was washed with PBS and treated with 100uL 0.1% TRITON X-100, followed by reacting at room temperature. After 10 minutes, each well was washed with PBS and blocked for 1 hour using 1% BSA. This was followed by treatment with HRP Anti-Growth Hormone antibody (1:1000). After 1 hour, each well was washed with PBST, and Ultra TMB substrate solution was added. After 10 minutes, each well was treated with 2N HCL stop solution, and absorbance was measured at 450nM to calculate the intracellular

accumulation of each substance.

The results are relative to the polypeptide of SEQ ID NO 42 as 100%. The results of measurement are as shown in Table 10 and FIG. 1.

[Table 10]

| Test Substance | | Excipient | | | | Weight Ratio (Pharmaceutically Active Substances: Excipients) | % |
|---|---|---|---|---|---|---|---|
| Test Substance | Volume (ug/mL) | Type | Volume (ug/mL) | Excipient volume (ug/mL) | Excipient weight ratio (bile acid:PG) | | |
| Polypeptide SEQ ID NO: 42 | 0.66 | - | - | - | - | - | 100 |
| Conjugate 68 | 0.69 | - | - | - | - | - | 110 |
| Conjugate 69 | 0.74 | - | - | - | - | - | 707 |
| Polypeptide SEQ ID NO: 42 | 0.66 | sCDC | 41.5 | 43.6 | 19.8:1 | 1:66.1 | 743 (*P*<0.05) |
| | | PG | 2.1 | | | | |
| Conjugate 68 | 0.69 | sCDC | 41.5 | 43.6 | 19.8:1 | 1:63.2 | 1533 (*P*<0.0 |
| | | PG | 2.1 | | | | 1) |
| Conjugate 69 | 0.74 | sCDC | 41.5 | 43.6 | 19.8:1 | 1:58.9 | 2011 (*P*<0.001) |
| | | PG | 2.1 | | | | |
| (PG: Propyl gallate, sCDC: Sodium chenodeoxycholate) | | | | | | | |

Formulation Embodiment 4. Preparation of formulation comprising conjugate (65, 66), one bile acid (sodium chenodeoxycholate) and propyl gallate, and measurement of blood glucose regulating ability of physiologically active substance in formulation

Physiologically active substances bound to a biotin moiety, fatty acid moiety or a combination thereof, prepared in accordance with the above example, were formulated by dissolving in a vehicle (0.02% polysorbate 80 in 10mM PBS (pH 7.4)) with the compositions of excipients shown in Table 11 and Table 12, then orally administered to mice, then their blood glucose regulating ability was measured through a glucose tolerance test.

The results of measurement are as shown in FIG. 2. At this time, as Control 1, a polypeptide wherein the proteins are joined through disulfide bonds between the 6th and 11th cysteine of SEQ ID NO 15; the 7th cysteine of SEQ ID NO 15 and the 7th cysteine of SEQ ID NO 16; and the 20th cysteine of SEQ ID NO 15 and the 19th cysteine of SEQ ID NO 16 dissolved in the vehicle, was used. Further, as Control 2, the same polypeptide dissolved in a formulation comprising sodium chenodeoxycholate and propyl gallate was used. As Control 3, Conjugate 65, a physiologically active substance bound to a biotin moiety, dissolved in a phosphate buffer solution not comprising bile acid and propyl gallate was used. The hypoglycemic effects were compared from 0 to 120 minutes following the glucose tolerance tests of Controls 1, 2 and 3. In the results, it was found, as shown in FIG. 2, that Conjugate 65 in the formulation comprising one bile acid and propyl gallate had a superior hypoglycemic effect.

The results of measurement are as shown in FIG. 3. As the Control, a polypeptide wherein the proteins are joined through disulfide bonds between the 6th and 11th cysteine of SEQ ID NO 15; the 7th cysteine of SEQ ID NO 15 and the 7th cysteine of SEQ ID NO 16; and the 20th cysteine of SEQ ID NO 15 and the 19th cysteine of SEQ ID NO 16 was used. In the measurement results, as shown in FIG. 3, the blood glucose level of the Control was lower than the untreated group. Further, it was found that blood glucose was substantially lower (especially after 20 to 40 minutes) than that of the Control following administration of Conjugate 65 and Conjugate 66.

[Table 11]

| Division | Test Substance | | Excipient | | | | Weight Ratio (Physiologically active substances: Excipients) |
|---|---|---|---|---|---|---|---|
| | Test Substance | Dose (mg/kg) | sCDC (mg/kg) | PG (mg/kg) | Excipient dose (mg/kg) | Excipient weight ratio (bile acid:PG) | |
| Vehicle 1 | Untreated Group 1 | | | | | | |
| Vehicle 2 | Untreated Group 2 | - | 68 | 34 | 102 | 2:1 | - |
| 1 | Control Group 1 | 1.4 | - | - | - | - | - |
| 2 | Control Group 2 | 1.4 | 68 | 34 | 102 | 2:1 | 1:70.4 |
| 3 | Control Group 3 Triconjugate 65 | 1.5 | - | - | - | - | - |
| 4 | Conjugate 65 | 1.5 | 68 | 34 | 102 | 2:1 | 1:68 |

[Table 12]

| Division | Test Substance | | Excipient | | | | Weight Ratio (Pharmaceutically Active Substance: Excipient) |
|---|---|---|---|---|---|---|---|
| | Test Substance | Administration dose (mg/kg) | sCDC (mg/kg) | PG (mg/kg) | Excipient dose (mg/kg) | Excipient weight ratio (bile acid:PG) | |
| Vehicle | Untreated Group | - | 68 | 34 | 102 | 2:1 | - |
| 1 | Control Group | 5.8 | 68 | 34 | 102 | 2:1 | 1:17.6 |
| 2 | Conjugate 65 | 6.0 | 68 | 34 | 102 | 2:1 | 1:17 |
| 3 | Conjugate 66 | 6.7 | 68 | 34 | 102 | 2:1 | 1:15.2 |
| (PG: Propyl gallate, sCDC: Sodium chenodeoxycholate) | | | | | | | |

Formulation Embodiment 5. Preparation of formulation comprising conjugate (33, 36, 39, 42, 61 through 64), one bile acid (sodium chenodeoxycholate) and propyl gallate, and measurement of the weight reduction and feed intake reduction effect of physiologically active substance in the formulation

Physiologically active substances bound to a biotin moiety, fatty acid moiety or a combination thereof, prepared in accordance with the above example, were formulated by dissolving in a vehicle (0.02% polysorbate 80 in 10mM PBS (pH 7.4)) with the compositions of excipients shown in Table 13, then administered orally to mice. Weight reduction and feed intake reduction were measured over 24 hours. The measurement results are shown in Table 14 and FIG. 4.

[Table 13]

| Division | Test Substance | | Excipient | | | | Weight Ratio (Physiologically Active Substance : Excipient) |
|---|---|---|---|---|---|---|---|
| | Test Substance | Administration Dose (mg/kg) | sCDC (mg/kg) | PG (mg/kg) | Excipient dose (mg/kg) | Excipient Weight Ratio (Bile Acid:PG) | |
| Vehicle | - | - | 68 | 34 | 102 | 2:1 | - |
| 1 | Polypeptide SEQ ID NO: 24 | 3.9 | 68 | 34 | 102 | 2:1 | 1:25.8 |
| 2 | Conjugate 33 | 4.2 | 68 | 34 | 102 | 2:1 | 1:24.4 |
| 3 | Conjugate 61 | 4.9 | 68 | 34 | 102 | 2:1 | 1:20.9 |
| 4 | Polypeptide SEQ ID NO: 26 | 4.0 | 68 | 34 | 102 | 2:1 | 1:25.7 |
| 5 | Conjugate 36 | 4.2 | 68 | 34 | 102 | 2:1 | 1:24.3 |
| 6 | Conjugate 62 | 4.9 | 68 | 34 | 102 | 2:1 | 1:20.8 |
| 7 | Polypeptide SEQ ID NO: 28 | 4.0 | 68 | 34 | 102 | 2:1 | 1:25.6 |
| 8 | Conjugate 39 | 4.2 | 68 | 34 | 102 | 2:1 | 1:24.2 |
| 9 | Conjugate 63 | 4.9 | 68 | 34 | 102 | 2:1 | 1:20.7 |
| 10 | Polypeptide SEQ ID NO: 30 | 4.0 | 68 | 34 | 102 | 2:1 | 1:25.6 |
| 11 | Conjugate 42 | 4.2 | 68 | 34 | 102 | 2:1 | 1:24.2 |
| 12 | Conjugate 64 | 4.9 | 68 | 34 | 102 | 2:1 | 1:20.7 |
| (PG: Propyl gallate, sCDC: Sodium chenodeoxycholate) | | | | | | | |

[Table 14]

| Item | Weight loss compared to untreated group (%) |
|---|---|
| Polypeptide SEQ ID NO: 24 | -2.63±1.06 |
| Polypeptide SEQ ID NO: 26 | -3.13±1.05 |
| Polypeptide SEQ ID NO: 28 | -1.11±0.84 |
| Polypeptide SEQ ID NO: 30 | -1.17±0.76 |
| Conjugate 33 | -4.07±1.30 |
| Conjugate 36 | -4.53±0.86 |

(continued)

| Item | Weight loss compared to untreated group (%) |
|---|---|
| Conjugate 39 | -1.99±0.94 |
| Conjugate 42 | -2.60±0.62 |
| Conjugate 61 | -2.96±4.22 |
| Conjugate 62 | -3.32±1.31 |
| Conjugate 63 | -2.41±0.78 |
| Conjugate 64 | -3.38±1.26 |

Formulation Embodiment 6. Preparation of formulation comprising Conjugate 3, one bile acid and propyl gallate, and measurementof intestinal absorption of physiologically active substance in formulation

Conjugate 3, a physiologically active substance bound to a biotin moiety, was formulated by dissolving in a vehicle (saline or 0.5% CMC in saline) with the compositions of excipients shown in Table 15, then administered to the duodenum of experimental rats (SD rat). Pharmaceutical behavior was compared. The results are as shown in Table 15 below.

[Table 15]

| Test Substance | | Excipient | | | | | | Weight Ratio | BA |
|---|---|---|---|---|---|---|---|---|---|
| Test substance | Dose (mg/kg) | Bile Acids | | PG (mg/kg) | Excipient dose (mg/kg) | Excipient Weight Ratio (Bile acid: PG) | | (Pharmaceutically Active Substance: Excipient) | (%) |
| | | Type | Dose (mg/kg) | | | | | | |
| Conjugate 3 | 0.55 | sCDC | 17 | 0 | 17 | - | | 1:30.9 | 1.0 |
| | 0.55 | sCDC | 34 | 0 | 34 | - | | 1:61.8 | 1.7 |
| | 0.55 | sCDC | 68 | 0 | 68 | - | | 1:123.6 | 0.4 |
| | 0.55 | sCDC | 17 | 8.5 | 25.5 | 2:1 | | 1:46.4 | 3.4 |
| | 0.55 | sCDC | 34 | 17 | 51 | 2:1 | | 1:92.7 | 7.1 |
| | 0.55 | sCDC | 68 | 34 | 102 | 2:1 | | 1:185.5 | 7.8 |
| | 0.55 | sDC | 16 | 0 | 16 | - | | 1:29.1 | 0.1 |
| | 0.55 | sDC | 32 | 0 | 32 | - | | 1 :58.2 | 0.2 |
| | 0.55 | sDC | 65 | 0 | 65 | - | | 1:118.2 | 0.3 |
| | 0.55 | sDC | 16 | 8.5 | 24.5 | 1.9:1 | | 1:44.5 | 7.8 |
| | 0.55 | sDC | 32 | 17 | 49 | 1.9:1 | | 1:89.1 | 13.1 |
| | 0.55 | sDC | 65 | 34 | 99 | 1.9:1 | | 1:180 | 16.3 |
| | 0.55 | sCA | 35 | 0 | 35 | - | | 1:63.6 | 0.1 |
| | 0.55 | sCA | 71 | 0 | 71 | - | | 1:129.1 | 0.5 |
| | 0.55 | sCA | 18 | 8.5 | 26.5 | 2.1:1 | | 1:48.2 | 1.0 |
| | 0.55 | sCA | 35 | 17 | 52 | 2.1:1 | | 1:94.5 | 3.1 |
| | 0.55 | sCA | 71 | 34 | 105 | 2.1:1 | | 1:190.9 | 12.4 |
| | 0.55 | sUDC | 17 | 0 | 17 | - | | 1 :30.9 | 0.1 |
| | 0.55 | sUDC | 34 | 0 | 34 | - | | 1:61.8 | 0.2 |
| | 0.55 | sUDC | 68 | 0 | 68 | - | | 1:123.6 | 0.7 |

(continued)

| Test Substance | | Excipient | | | | | Weight Ratio | BA |
|---|---|---|---|---|---|---|---|---|
| Test substance | Dose (mg/kg) | Bile Acids | | PG (mg/kg) | Excipient dose (mg/kg) | Excipient Weight Ratio (Bile acid: PG) | (Pharmaceutically Active Substance: Excipient) | (%) |
| | | Type | Dose (mg/kg) | | | | | |
| | 0.55 | sUDC | 17 | 8.5 | 25.5 | 2:1 | 1:46.4 | 0.3 |
| | 0.55 | sUDC | 34 | 17 | 51 | 2:1 | 1:92.7 | 3.9 |
| | 0.55 | sUDC | 68 | 34 | 102 | 2:1 | 1:185.5 | 6.9 |
| | 0.55 | sGC | 21 | 0 | 21 | - | 1:38.2 | 0.1 |
| | 0.55 | sGC | 42 | 0 | 42 | - | 1 :76.4 | 0.1 |
| | 0.55 | sGC | 83 | 0 | 83 | - | 1:150.9 | 0.2 |
| | 0.55 | sGC | 21 | 8.5 | 29.5 | 2.5:1 | 1:53.6 | 0.1 |
| | 0.55 | sGC | 42 | 17 | 59 | 2.5:1 | 1:107.3 | 0.3 |
| | 0.55 | sGC | 83 | 34 | 117 | 2.4:1 | 1:212.7 | 4.4 |
| | 0.55 | sTC | 22 | 0 | 22 | - | 1:40 | 0.4 |
| | 0.55 | sTC | 44 | 0 | 44 | - | 1:80 | 0.1 |
| | 0.55 | sTC | 88 | 0 | 88 | - | 1:160 | 0.8 |
| | 0.55 | sTC | 44 | 17 | 61 | 2.6:1 | 1:110.9 | 1.5 |
| | 0.55 | sTC | 88 | 34 | 122 | 2.6:1 | 1:221.8 | 0.9 |
| (PG: Propyl gallate, sCDC: Sodium chenodeoxycholate, sDC: Sodium deoxycholate, sCA: Sodium cholate, sUDC: Sodium ursodeoxycholate, sGC: Sodium glycocholate hydrate, sTC: Sodium taurocholate) | | | | | | | | |

Formulation Embodiment 7. Preparation of formulations comprising conjugate (14, 16, 17, 18, 19), one bile acid and propyl gallate, and measurement of intestinal absorption of physiologically active substance in formulation

Physiologically active substances bound to a biotin moiety, fatty acid moiety or combination thereof prepared in accordance with the above example were formulated by dissolving in a vehicle (0.02% polysorbate 80 in saline) with the compositions of excipients shown in Table 16, then administered to the duodenum of experimental rats (SD rat). Pharmaceutical behavior was compared. The results are as shown in Table 16 below.

[Table 16]

| Test Substance | | Excipient | | | | Weight Ratio | $AUC_{last}$ | BA |
|---|---|---|---|---|---|---|---|---|
| Test Substance | Administration Dose (mg/kg) | sCDC (mg/kg) | PG (mg /kg) | Excipient Dose (mg/kg) | Excipient Weight Ratio (sCDC: PG) | (Physiologically Active Substance: Excipient) | (hr*ng/ mL) | (%) |
| Conjugate 14 | 1.7 | 68 | 34 | 102 | 2:1 | 1:60 | 196.0 + 50 | - |
| Conjugate 16 | 1.9 | 68 | 34 | 102 | 2:1 | 1:53.7 | 12976.4 ± 7009 | 17.4 |
| Conjugatee 17 | 1.7 | 68 | 34 | 102 | 2:1 | 1:60 | 15506.1 ± 3709 | 5.2 |
| Conjugate 18 | 1.9 | 68 | 34 | 102 | 2:1 | 1:53.7 | 6383.1 ± 1593 | - |

(continued)

| Test Substance | | Excipient | | | | Weight Ratio | $AUC_{last}$ | BA |
|---|---|---|---|---|---|---|---|---|
| Test Substance | Administration Dose (mg/kg) | sCDC (mg/kg) | PG (mg/kg) | Excipient Dose (mg/kg) | Excipient Weight Ratio (sCDC: PG) | (Physiologically Active Substance: Excipient) | (hr*ng/mL) | (%) |
| Conjugate 19 | 1.9 | 68 | 34 | 102 | 2:1 | 1:53.7 | 1273.4 ± 368 | - |
| (PG: Propyl gallate, sCDC: Sodium chenodeoxycholate) | | | | | | | | |

Formulation Embodiment 8. Preparation of formulation comprising Conjugate 17, one bile acid (sodium chenodeoxycholate) and propyl gallate, and measurement of intestinal absorption of physiologically active substance in formulation

The biotin moiety, fatty acid moiety, or a combination thereof prepared according to the above example was dissolved in a vehicle with the composition of the excipients shown in Table 17. At this time, the vehicle was formulated by properly mixing polysorbate 80, propylene glycol, CMC, saline or phosphate buffer. After administration of the formulation to the duodenum of SD rats, pharmacological behaviors were compared. The results are as shown in Table 17 below.

[Table 17]

| Test Substance | | Excipient | | | | | Weight Ratio (Physiologically Active Substance : Excipient) | BA (%) |
|---|---|---|---|---|---|---|---|---|
| Test Substance | Administration Dose (mg/kg) | Bile Acids | | PG (mg/kg) | Excipient Dose (mg/kg) | Excipient Weight Ratio (Bile Acid: PG) | | |
| | | Type | Dose (mg/kg) | | | | | |
| Conjugate 17 | 1.7 | sCDC | 34 | - | 34 | | 1:20 | 0.9 |
| | 1.7 | sCDC | 68 | - | 68 | - | 1:40 | 0.4 |
| | 1.7 | sUDC | 68 | - | 68 | - | 1:40 | 0.8 |
| | 1.7 | sUDC | 102 | - | 102 | - | 1:60 | 1.1 |
| | 1.7 | sCDC+ sUDC | 34+68 | - | 102 | - | 1:60 | 2.3 |
| | 1.7 | sCDC +sUD C | 34+102 | - | 136 | - | 1:80 | 2.0 |
| | 1.7 | - | - | 68 | 68 | - | 1:40 | 0.6 |
| | 1.7 | sCDC | 17 | 34 | 51 | 1:2 | 1:30 | 10. 6 |
| | 1.7 | sCDC | 17 | 68 | 85 | 1:4 | 1:50 | 2.4 |
| | 1.7 | sCDC | 34 | 68 | 102 | 1:2 | 1:60 | 6.3 |
| | 1.7 | sCDC | 34 | 68 | 102 | 1:2 | 1:60 | 4.0 |
| | 1.7 | sCDC | 68 | 34 | 102 | 2:1 | 1:60 | 5.2 |
| | 1.7 | sUDC | 34 | 34 | 68 | 1:1 | 1:40 | 7.0 |
| | 1.7 | sUDC | 34 | 68 | 102 | 1:2 | 1:60 | 4.1 |
| | 1.7 | sUDC | 68 | 17 | 85 | 4:1 | 1:50 | 6.3 |
| | 1.7 | sDC | 17 | 34 | 51 | 1:2 | 1:30 | 2.9 |
| | 1.7 | sDC | 34 | 68 | 102 | 1:2 | 1:60 | 6.4 |
| | 1.7 | sDC | 68 | 34 | 102 | 2:1 | 1:60 | 3.9 |
| | 1.7 | sCDC+ sUDC | 34+68 | 17 | 119 | 6:1 | 1:70 | 3.2 |
| | 1.7 | sCDC+ sUDC | 34+68 | 34 | 136 | 3:1 | 1:80 | 5.9 |
| | 1.7 | sCDC +sUD C | 34+68 | 68 | 170 | 1.5:1 | 1:100 | 7.4 |
| (PG: Propyl gallate, sCDC: Sodium chenodeoxycholate, sDC: Sodium deoxycholate, sUDC: Sodium ursodeoxycholate) | | | | | | | | |

**A) Formulation Embodiment 9. Preparation of formulation comprising Conjugate 67, one bile acid (sodium chenodeoxycholate) and propyl gallate, and measurement of intestinal absorption of physiologically active substance in formulation**

B) Conjugate 67, a physiologically active substance bound to a biotin moiety, was formulated by dissolving in a vehicle (0.02% polysorbate 80 in 10mM PBS (pH 7.4)) with the compositions of excipients shown in Table 18, then administered to the duodenum of experimental rats (SD rat). The concentration of the physiologically active substance was measured at $T_{max}$, and the results are represented as $C_{max}$. These results were compared against results obtained by administering SEQ ID NO 6, a physiologically active substance not bound to a biotin moiety, using the same formulation. The results are as shown in Table 18 below.

[Table 18]

| Test Substance | | Excipient | | | | Weight Ratio (Physiologically Active Substance : Excipient) | $C_{max}$ (ng/mL) |
|---|---|---|---|---|---|---|---|
| Test Substance | Administration Dose (mg/kg) | sCDC (mg/kg) | PG (mg/kg) | Excipient Volume (mg/kg) | Excipient Weight Ratio | | |
| Polypeptide SEQ ID NO: 6 | 5.4 | 68 | 34 | 102 | 2:1 | 1:18.9 | 30.0 |
| Conjugate 67 | 6.2 | 68 | 34 | 102 | 2:1 | 1:16.4 | 198.1 |
| (PG: Propyl gallate, sCDC: Sodium chenodeoxycholate) | | | | | | | |

Formulation Embodiment 10. Preparation of solid formulation comprising Conjugate 52, one bile acid and propyl gallate, and measurement of oral absorption rate

Solid formulations were prepared using Conjugate 52, a physiologically active substance bound to a biotin moiety, with the compositions of excipients shown in Table 19. These were administered orally to Beagle Dogs, and pharmaceutical behavior was compared.

The solid formulations were prepared by mixing the physiologically active substance with bile acid, propyl gallate and typical excipients used for manufacturing solid formulations (Mannitol, Crosspovidone, Stearate, and the like), then preparing into granules using the dry granulation method, and preparing as tablets using a tableting machine. These were then enterically coated using a coating machine. The tablets were prepared as immediate release and extended release tablets by adjusting the amounts of binder and disintegrant. The immediate release tablets eluted at least 80% of the physiologically active substance within 60 minutes under elution conditions (pH 6.8, 50rpm, 37C), and the extended release tablets eluted at least 80% of the physiologically active substance within 360 minutes under elution conditions (pH 6.8, 50rpm, 37C).

[Table 19]

| Type | Test Substance | | Excipient | | | | | | Weight Ratio (Physiologically Active Substance: Excipient) | BA (%) | CV (%) |
|---|---|---|---|---|---|---|---|---|---|---|---|
| | Test Substance | Dose (mg/tab) | Bile Acids | | PG (mg/kg) | Dose (mg/tab) | Excipient Weight Ratio (Bile Acid:PG) | | | | |
| | | | Type | Dose (mg/tab) | | | | | | | |
| Extended Release | Conjugate 52 | 10 | sCDC | 100 | 200 | 300 | 1:2 | | 1:30 | 0.54 | 186 |
| Immediate Release | | 10 | sCDC | 100 | 200 | 300 | 1:2 | | 1:30 | 0.92 | 68 |
| Extended Release | | 10 | CDC | 100 | 200 | 300 | 1:2 | | 1:30 | 2.18 | 107 |
| Immediate Release | | 10 | CDC | 100 | 200 | 300 | 1:2 | | 1:30 | 4.23 | 84 |
| Extended Release | | 10 | sCDC | 100 | 200 | 500 | 1:1 | | 1:50 | 6.81 | 94 |
| | | | sUDC | 200 | | | | | | | |
| Immediate Release | | 10 | sCDC | 100 | 200 | 500 | 1:1 | | 1:50 | 10.0 8 | 117 |
| | | | sUDC | 200 | | | | | | | |
| Extended Release | | 10 | CDC | 100 | 200 | 500 | 1:1 | | 1:50 | 2.79 | 175 |
| | | | UDC | 200 | | | | | | | |
| Immediate Release | | 10 | CDC | 100 | 200 | 500 | 1:1 | | 1:50 | 5.21 | 119 |
| | | | UDC | 200 | | | | | | | |

A) (PG: Propyl gallate, sCDC: Sodium Chenodeoxycholate, sDC: Sodium deoxycholate, sCA: Sodium cholate, sUDC: Sodium ursodeoxycholate, sGC: Sodium glycocholate, sTC: Sodium taurocholate)

**B) Formulation Embodiment 11. Preparation of formulation comprising Conjugate 52, one bile acid (sodium chenodeoxycholate) and propyl gallate, and measurement of blood glucose regulating ability of physiologically active substance in formulation**

C) Physiologically active substances bound to a biotin moiety, fatty acid moiety or combination thereof prepared in accordance with the above example were formulated by dissolving in a vehicle (0.02% polysorbate 80 in 10mM PBS (pH 7.4)) with the compositions of excipients shown in Table 20, then orally administered to mice. Their blood glucose regulating ability was measured through glucose tolerance tests.

D) The results of measurement were as shown in FIG. 5. As the Control, Conjugate 52 dissolved at two different doses in phosphate buffer solution not comprising bile acid and propyl gallate was used, and the hypoglycemic effects from 0 minutes to 120 minutes after the glucose tolerance tests were compared. As a result, as shown in FIG. 5, a dose-dependent hypoglycemic effect was found compared to the Control after administration of Conjugate 52 in the formulation comprising one bile acid and propyl gallate.

[Table 20]

| Item | Test Substance | | Excipient | | | | Weight Ratio (Physiologically Active Substance: Excipient) |
|---|---|---|---|---|---|---|---|
| | Test Substance | Administration Dose (mg/kg) | sCDC (mg/kg) | PG (mg/kg) | Excipient Dose (mg/kg) | Excipient Weight Ratio (Bile acid: PG) | |
| Vehicle 1 | Untreated Group 1 | - | - | - | - | - | - |
| Vehicle 2 | Untreated Group 2 | - | 68 | 34 | 102 | 2:1 | - |
| 1 | Control Group Conjugate 52 | 5.51 | - | - | - | - | - |
| 2 | Conjugate 52 | 5.51 | 68 | 34 | 102 | 2:1 | 1:18.5 |

Formulation Embodiment 12. Evaluation of the antidiabetic effect of physiologically active substance in formulations comprising Conjugate 3, one bile acid (sodium chenodeoxycholate) and propyl gallate

Physiologically active substances bound to a biotin moiety prepared in accordance with the above example were formulated by dissolving in a vehicle with the compositions of excipients shown in Table 21. These were orally administered to mice twice daily over 8 weeks, and changes in glycated hemoglobin were measured.

The measurement results are as shown in Table 21. Conjugate 3 dissolved in two different formulations was used as the test substance, and glycate hemoglobin testing was carried out after administering for 8 weeks. In the results, it was confirmed, as shown in Table 21, that the glycated hemoglobin reduction effect of Conjugate 3 in a formulation comprising one bile acid and propyl gallate was superior to that of a formulation comprising Labrasol and Poloxamer 188.

[Table 21]

| Type | Test Substance | | Excipient | | | | ΔGlycated Hemoglobin (%) |
|---|---|---|---|---|---|---|---|
| | Test Substance | Administration Dose (mg/kg) | Labrazol (mg/kg) | Poloxamer 188 (mg/kg) | sCDC (mg/kg) | PG (mg/kg) | |
| Vehicle 1 | Untreated Group 1 | - | 100 | 0.4 | - | - | +2.02 |
| Vehicle 2 | Untreated Group 2 | - | - | - | 68 | 34 | +2.38 |

(continued)

| Type | Test Substance | | Excipient | | | | ΔGlycated Hemoglobin (%) |
|---|---|---|---|---|---|---|---|
| | Test Substance | Administration Dose (mg/kg) | Labrazol (mg/kg) | Poloxamer 188 (mg/kg) | sCDC (mg/kg) | PG (mg/kg) | |
| 1 | Conjugate 3 | 0.55 | 100 | 0.4 | - | - | -1.40 |
| 2 | Conjugate 3 | 0.55 | - | - | 68 | 34 | -2.22 |

Formulation Embodiment 13. Evaluation of the antidiabetic effect of physiologically active substance in formulations comprising Conjugate 3, one bile acid (sodium chenodeoxycholate) and propyl gallate

Conjugate 3, a physiologically active substance bound to a biotin moiety, was formulated by dissolving in a vehicle with the compositions of excipients shown in Table 22. These were administered to the duodenum of experimental beagles, and pharmaceutical behavior was compared. The results are as shown in Table 22.

[Table 22]

| Test Substance | | Excipient | | | | Bioavailability (%) |
|---|---|---|---|---|---|---|
| Test Substance | Administration Dose (mg/kg) | Labrazol (mg/kg) | Poloxamer 188 (mg/kg) | sCDC (mg/kg) | PG (mg/kg) | |
| Conjugate 3 | 0.25 | 100 | 0.4 | - | - | 0.32±0.06 |
| Conjugate 3 | 0.25 | - | - | 68 | 34 | 1.61±0.81 |

Formulation Embodiment 14. Evaluation of the antidiabetic effect of physiologically active substance in formulation comprising Conjugate 56, one bile acid (sodium chenodeoxycholate) and propyl gallate

Physiologically active substances bound to a biotin moiety prepared in accordance with the above example were formulated by dissolving in a vehicle with the compositions of excipients shown in Table 23. These were orally administered to mice once daily over 3 weeks, and changes in glycated hemoglobin were measured.

The results of measurement were as shown in Table 23. Results of glycated hemoglobin tests after three weeks of administration confirmed that oral administration of Conjugate 56 in a formulation comprising one bile acid and propyl gallate exhibited a glycated hemoglobin reduction effect.

[Table 23]

| Item | Test Substance | | Excipient | | ΔGlycated Hemoglobin (%) |
|---|---|---|---|---|---|
| | Test Substance | Administration Dose (mg/kg) | sCDC (mg/kg) | PG (mg/kg) | |
| Vehicle | Untreated Group | - | 68 | 34 | + 0.20±0.48 |
| 1 | Conjugate 56 | 1.343 | 68 | 34 | - 0.47±0.34 |
| 2 | Conjugate 56 | 4.475 | 68 | 34 | - 0.98±0.67 |

<Embodiment: Pharmaceutical formulations comprising pharmaceutically active substances bound to biotin moieties and excipients≥

Measurement of solubility of each formulation

The solubility of physiologically active substance bound to biotin moieties prepared in accordance with the

above example in formulations was measured. Conjugate 3 was dissolved in a vehicle (saline) or excipient shown in the table below at a concentration of 2.2 mg/mL, then the solution was filtered and the concentration of Conjugate 3 in the solution was measured using an HPLC. As shown in Table 24 below, the solubility of Conjugate 3 in saline was 0.081 mg/mL, and it can be seen that in the following combinations of excipients, the solubility of Conjugate 3 was increased by 12 to 27 times.

[Table 24]

| Test Substance | | Excipient | | Solubility (mg/mL) |
|---|---|---|---|---|
| Conjugate | Test Concentration | Type | Volume | |
| Conjugate 3 | 2.2 mg/mL | - | - | 0.081 |
| Conjugate 3 | 2.2 mg/mL | sCDC | 27 mg/mL | 2.2 |
| | | PG | 14 mg/mL | |
| Conjugate 3 | 2.2 mg/mL | sDC | 26 mg/mL | 2.2 |
| | | PG | 13 mg/mL | |
| Conjugate 3 | 2.2 mg/mL | sCA | 28 mg/mL | 1.4 |
| | | PG | 14 mg/mL | |
| Conjugate 3 | 2.2 mg/mL | sGC | 33 mg/mL | 1.0 |
| | | PG | 17 mg/mL | |
| Conjugate 3 | 2.2 mg/mL | sTC | 35 mg/mL | 1.0 |
| | | PG | 18 mg/mL | |
| Conjugate 3 | 2.2 mg/mL | sUDC | 27 mg/mL | 2.2 |
| | | PG | 14 mg/mL | |

Those skilled in the art will recognize, or be able to ascertain using no more than routine experimentation, many equivalents to the specific embodiments of the invention described herein. Such equivalents are intended to be included in the scope of the following claims. The above description of the present application is for illustration, and those of ordinary skill in the art to which the present application pertains will understand that it can be easily modified into other specific forms without changing the technical idea or essential features of the present application. Therefore, it should be understood that the embodiments described above are illustrative in all respects and non-limiting. For example, each component described as a single type may be implemented in a distributed manner, and likewise components described as distributed may be implemented in a combined form. All changes or modifications derived from the meaning and scope of the claims to be described below, and equivalents thereof, may be construed as being included in the scope of the present invention.

[Commercial Applicability]

The oral formulations of the present invention can efficiently increase absorption in the body, and thus can be usefully used in the pharmaceutical field.

## Claims

1. An oral pharmaceutical formulation comprising (i) a physiologically active substance conjugate bound to a biotin moiety, a fatty acid moiety, or a combination thereof, and (ii) an excipient.

2. The oral pharmaceutical formulation of Claim 1,

    where, in the (i) physiologically active substance conjugate bound to a biotin moiety, a fatty acid moiety, or a combination thereof,
    the physiologically active substance is selected from the group comprising: glucagon (Glugacon), GLP-1 (Glucagon-like peptide-1), GLP-2 (Glucagon-like peptide-2), GIP (glucose-dependent insulinotropic polypeptide), exendin-4, insulin, parathyroid hormone, interferon, erythropoietin, calcitonin, amylin, serotonin, rituximab, trastuzumab, uricase, tissue plasminogen activator, thymoglobin, vaccine, heparin or heparin analog, antithrombin III, filgrastim, pramlintide acetate, exenatide, eptifibatide, antivenin, IgG, IgM, HGH, thyroxine, blood clotting factors VII and VIII, glycolipids acting as therapeutic agents, and derivatives thereof.

**3.** The oral pharmaceutical formulation of Claim 1,
where, in the (i) physiologically active substance conjugate bound to a biotin moiety, a fatty acid moiety, or a combination thereof, the physiologically active substance is one selected from the group comprising polypeptides comprised of the amino acid sequences represented by SEQ ID NOs: 1 through 14 and SEQ ID NOs: 18 through 42, and derivatives thereof.

**4.** The oral pharmaceutical formulation of Claim 1,
where, in the (i) physiologically active substance conjugate bound to a biotin moiety, a fatty acid moiety, or a combination thereof, the physiologically active substance is a polypeptide comprised of the amino acid sequences represented by SEQ ID NOs: 15 and 16 or a derivative thereof; or a polypeptide comprised of the amino acid sequences represented by SEQ ID NOs: 17 and 16 or a derivative thereof.

**5.** The oral pharmaceutical formulation of Claim 1,

where, in the (i) physiologically active substance conjugate bound to a biotin moiety, a fatty acid moiety, or a combination thereof,
the biotin moiety is represented by General Formula A below:

[Formula A]

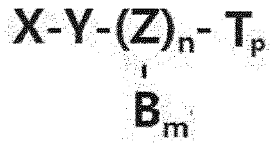

Where, in the above general formula A,

X is a functional group capable of binding to a physiologically active substance;
Y is a spacer;
Z is a binding unit;
B is represented by the following Chemical Formula A-1;

[Chemical Formula A-1]

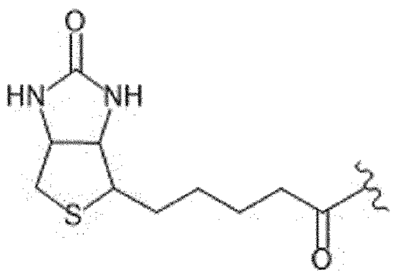

Z is connected to 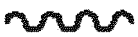 of Chemical Formula A-1;
T is a terminal group;
m is an integer of 1 to 10;
n is 0 or an integer of 1 to 10, and when n=0, Y is directly bonded to B or T;
p is an integer of 0 or 1.

6. The oral pharmaceutical formulation of Claim 5, wherein X is selected from the group comprising: maleimide, succinimide, N-hydroxysuccinimide, succinimidyl succinate, succinimidyl glutarate, succinimidyl methyl ester, succinimidyl pentyl ester, succinimidyl carbonate, p-nitrophenyl carbonate, aldehyde, amine, thiol, oxyamine, iodoacetamide, aminooxyl, hydrazide, hydroxy, propionate, pyridyl, alkyl halide, vinyl sulfone, carboxyl, hydrazide, halogen acetamide, $C_{2-5}$ alkynyl, $C_{6-20}$ aryldisulfide, $C_{5-20}$ heteroaryldisulfide, isocyanate, thioester, iminoester, and derivatives thereof.

7. The oral pharmaceutical formulation of Claim 5,

wherein Y is absent, or is a substituted or unsubstituted linear or branched $C_{1-50}$ alkylene, substituted or unsubstituted linear or branched $C_{1-50}$ heteroalkylene, substituted or unsubstituted $C_{6-50}$ arylene, or substituted or unsubstituted $C_{6-50}$ heteroarylene,
and if substituted, comprises at least one selected from the group comprising =O, -C(O)$NH_2$, - OH, -COOH, -SH, =NH and -$NH_2$.

8. The oral pharmaceutical formulation of Claim 5, wherein Y comprises -C(O)-($OCH_2CH_2$)$_u$-NH- as a repeating unit, where u is an integer of 1 to 20.

9. The oral pharmaceutical formulation of Claim 5, wherein Y comprises glutamic acid, glutamine, glycine, isoleucine, or lysine as a component.

10. The oral pharmaceutical formulation of Claim 5,

wherein Z is any one of the following, each of whichmay be independently selected:

A) forms an amino acid or a derivative thereof together with X or separately from X;
B) is a substituted or unsubstituted linear or branched $C_{1-50}$ heteroalkyene,

where, if substituted, comprises at least one selected from the group comprising =O, -C(O)$NH_2$, -OH, -COOH, -SH, =NH, and -$NH_2$.

11. The oral pharmaceutical formulation of Claim 5, wherein T is selected fromthe group comprising: amine, $C_{1-8}$ alkyl, $C_{1-8}$ alkenyl, halo, hydroxy, thiol, sulfonic acid, carboxyl, phenyl, benzyl, aldehyde, azide, cyanate, isocyanate, thiocyanate, isothiocyanate, nitrile and phosphonic acid.

12. The oral pharmaceutical formulation of Claim 1,

where, in the (i) physiologically active substance conjugate bound to a biotin moiety, a fatty acid moiety, or a combination thereof,
the biotin moiety is selected from the group comprising:

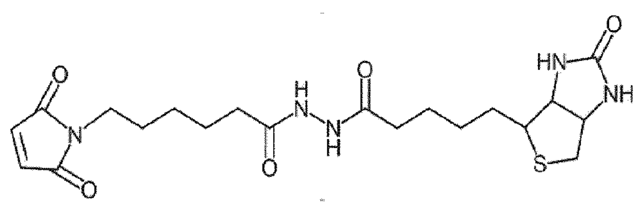
,

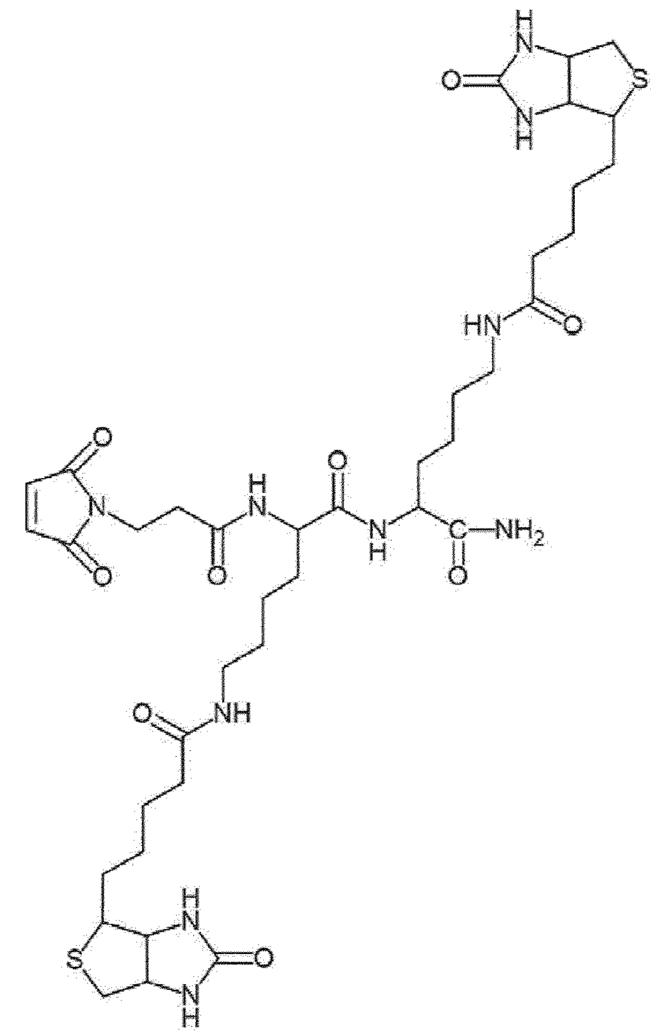

,

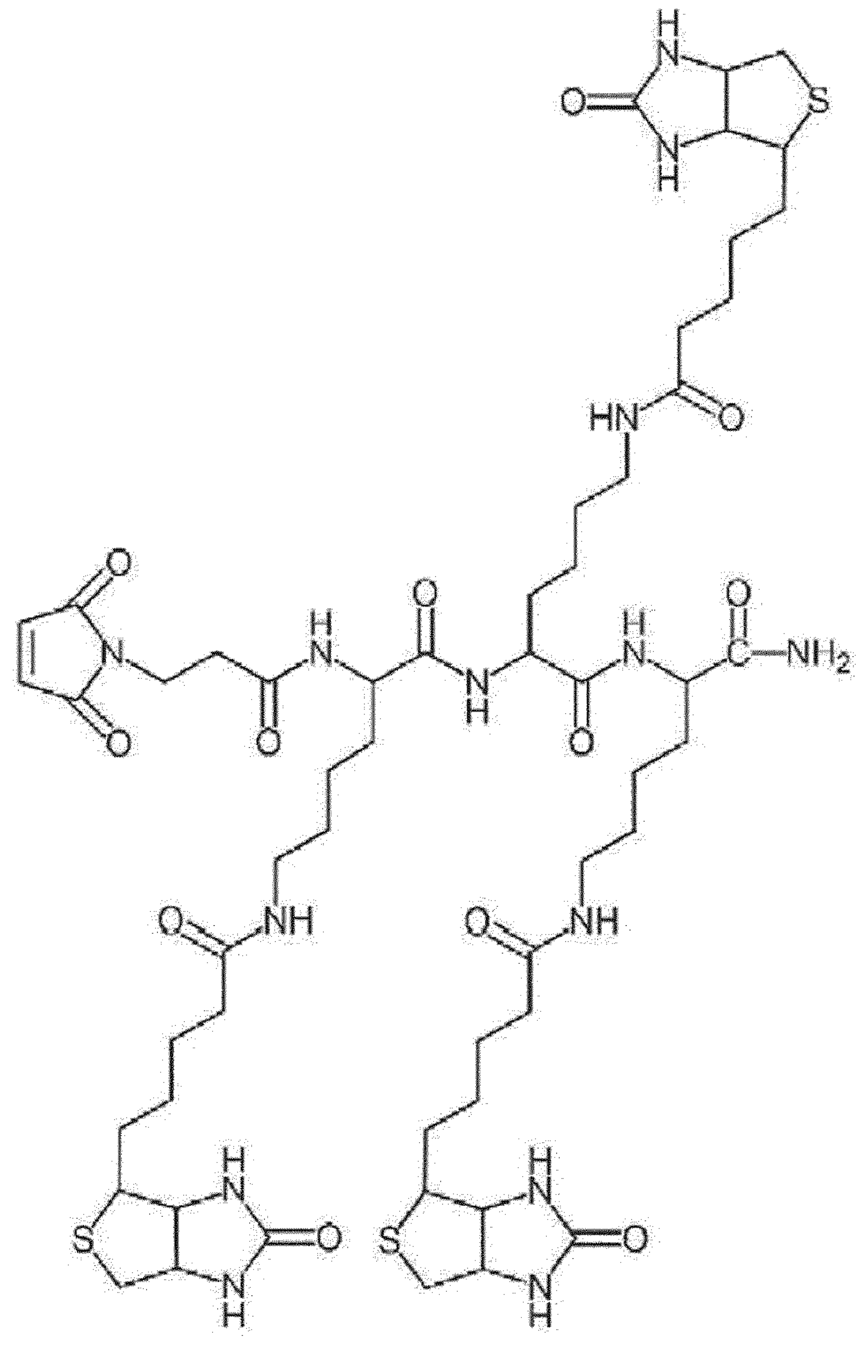

,

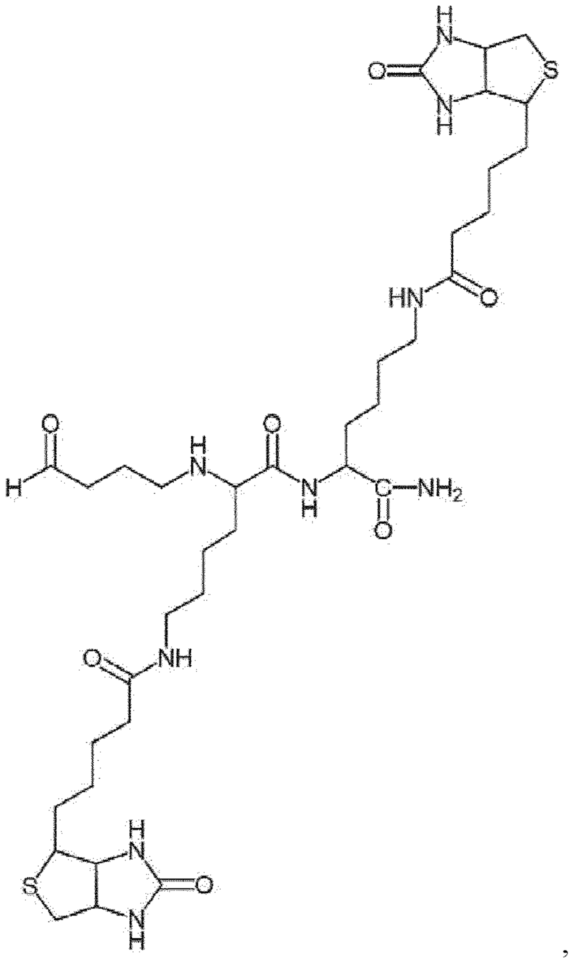

,

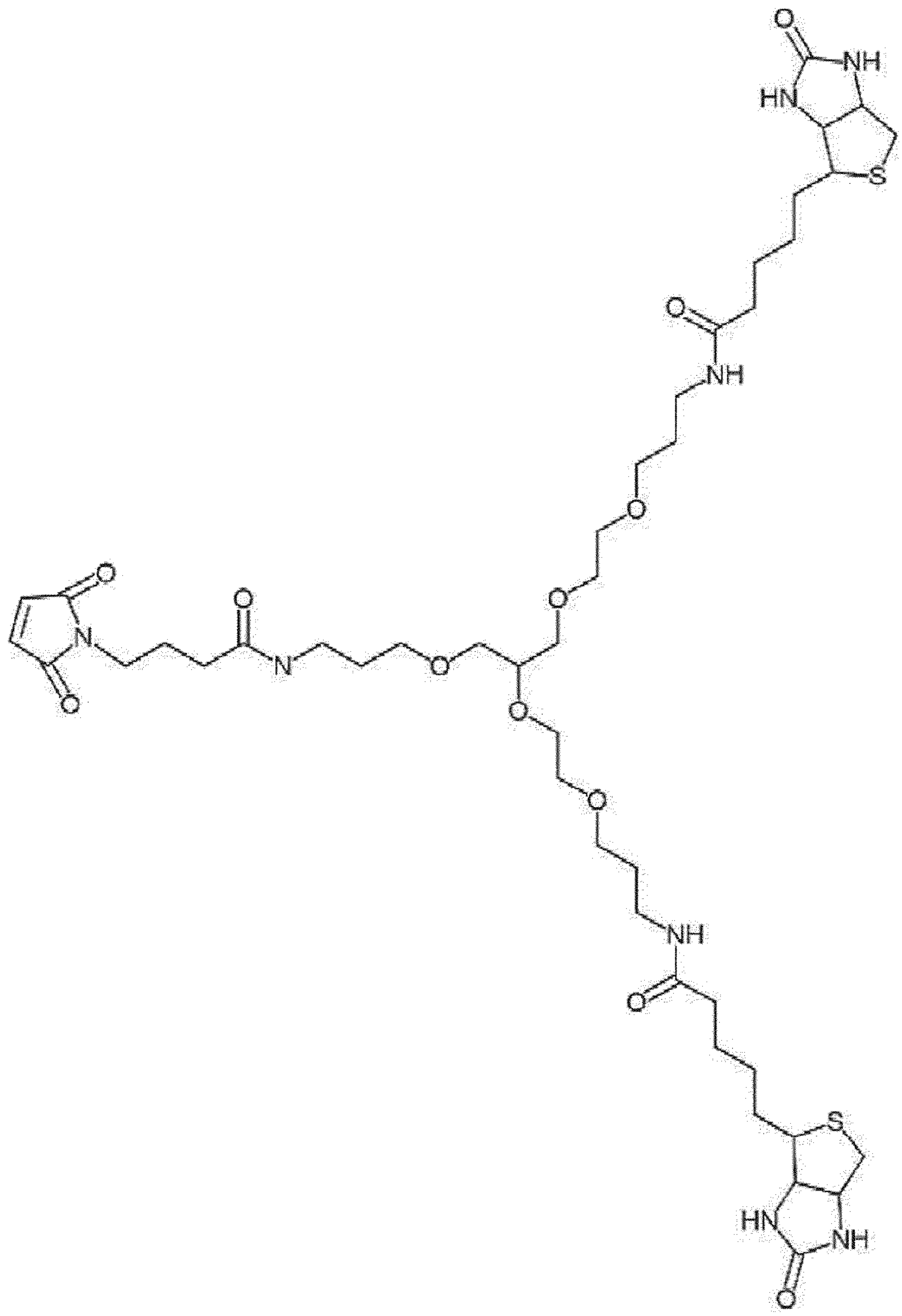

,

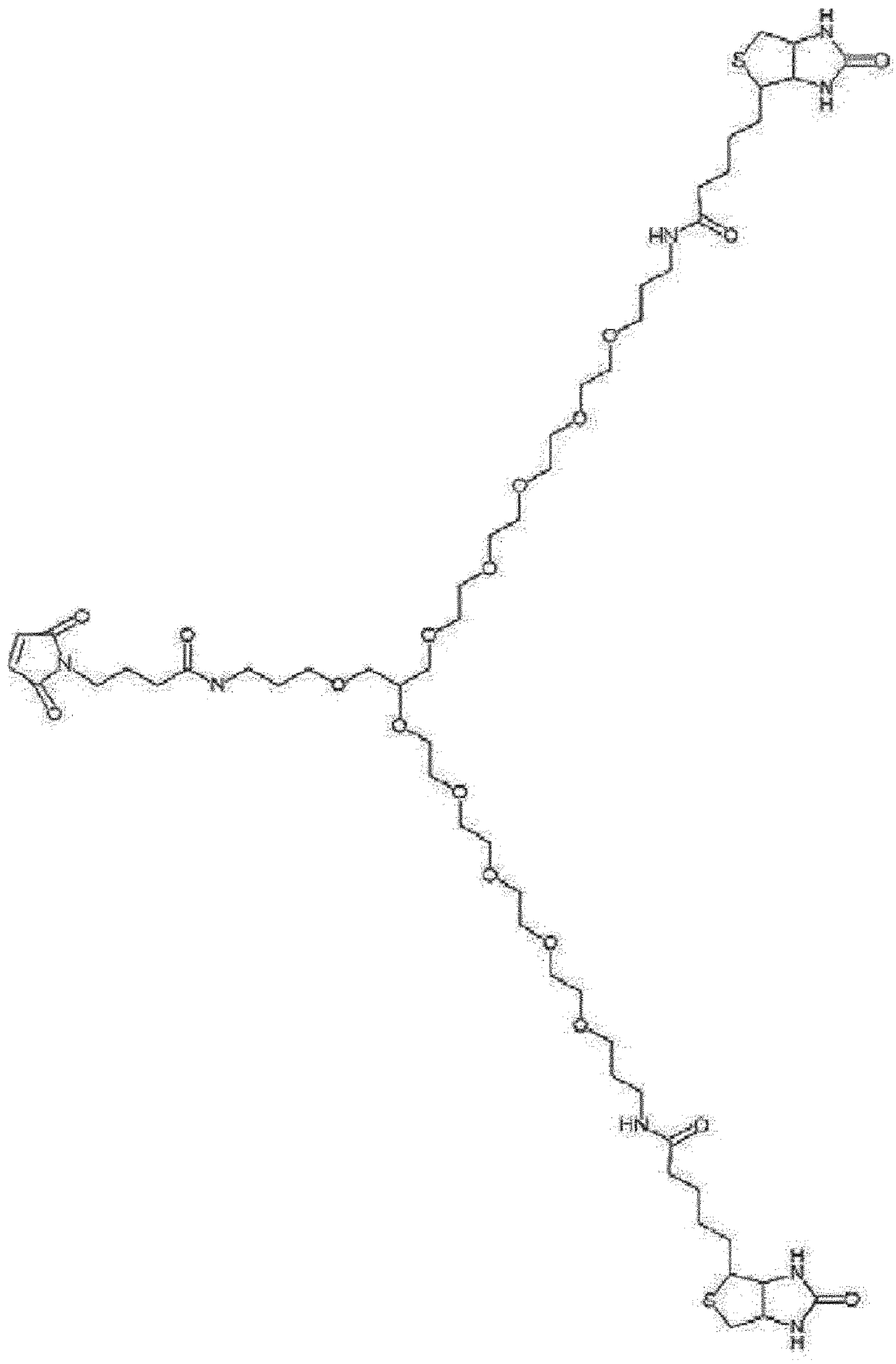

,

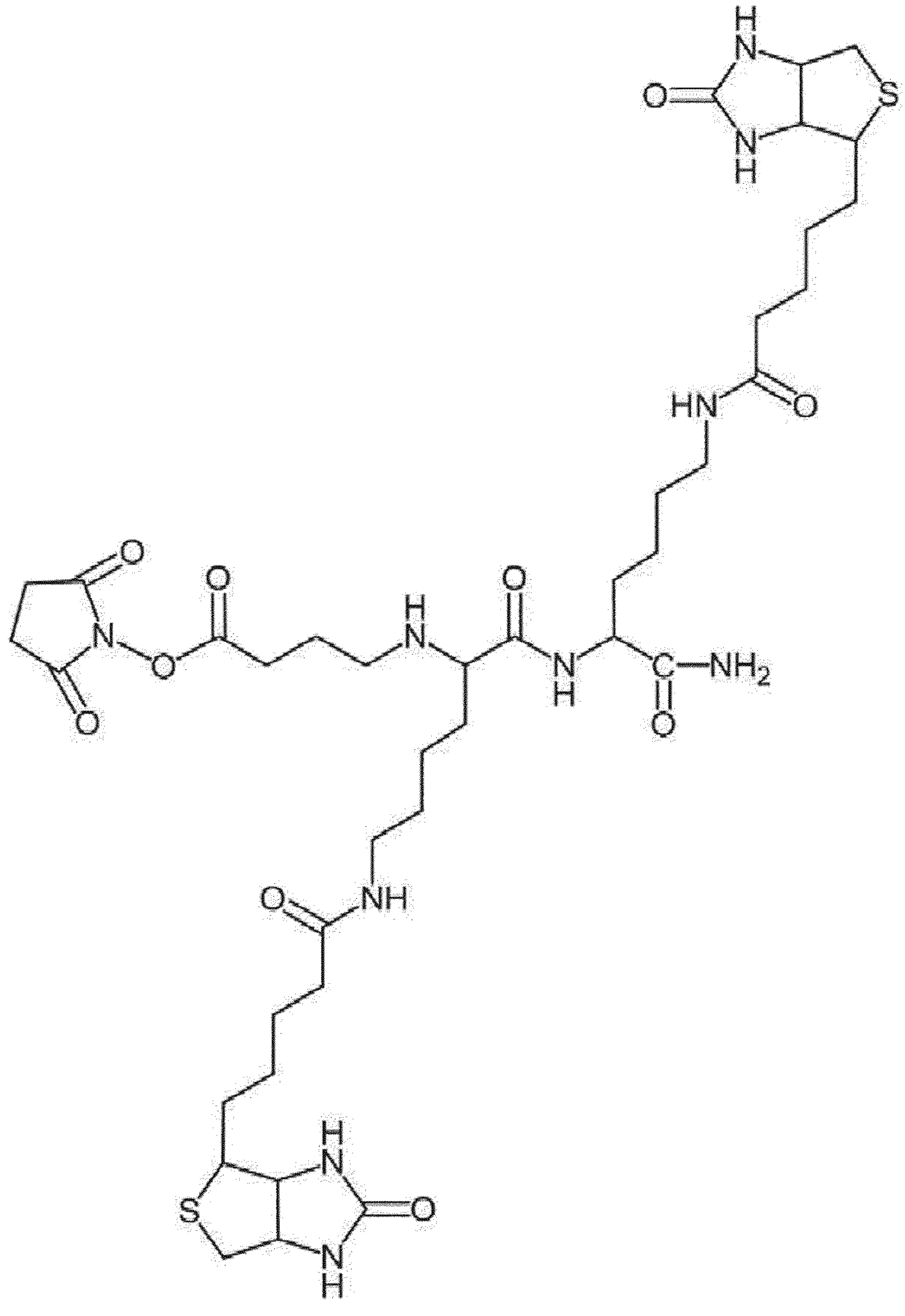

,

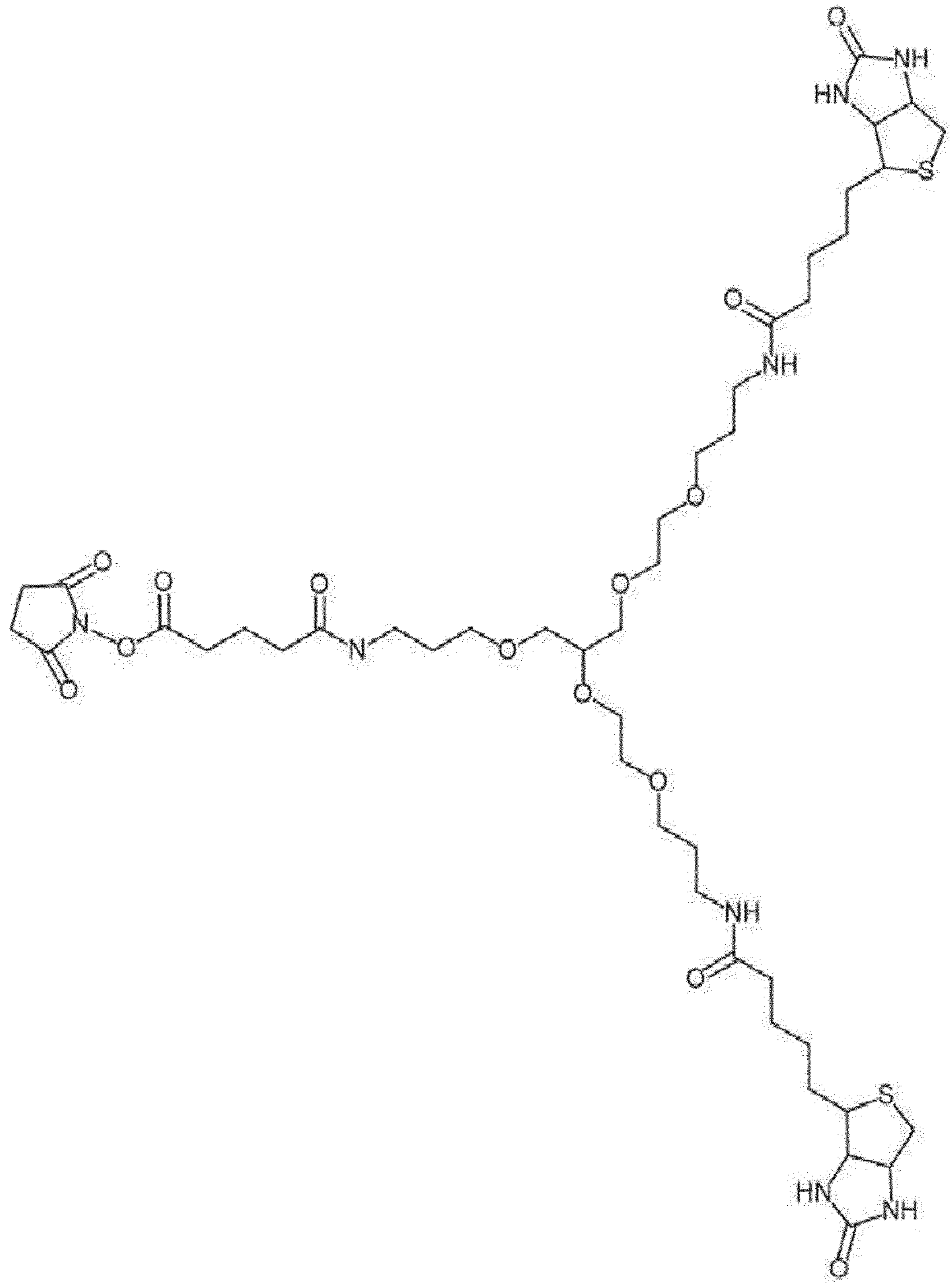

,

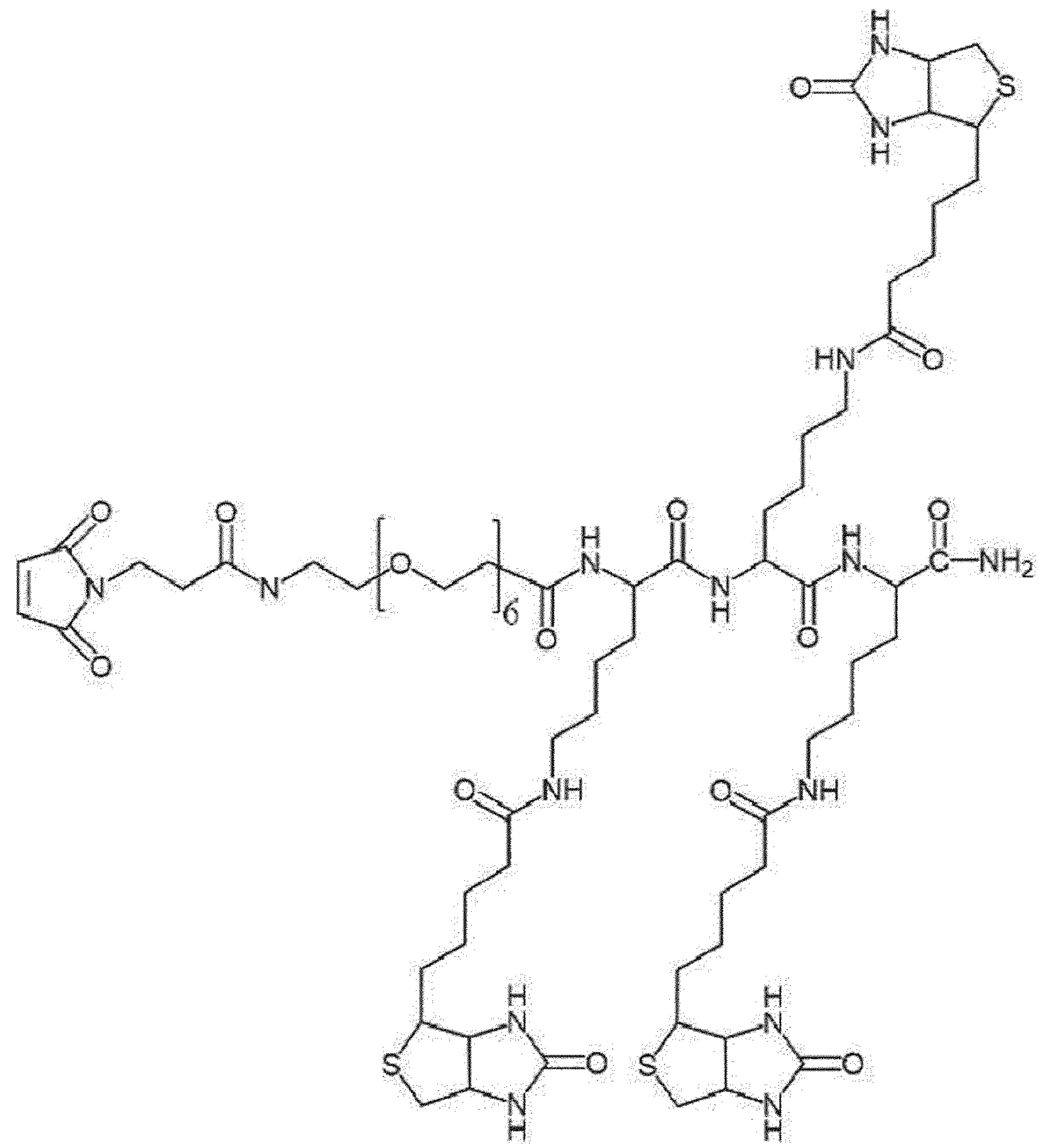

,

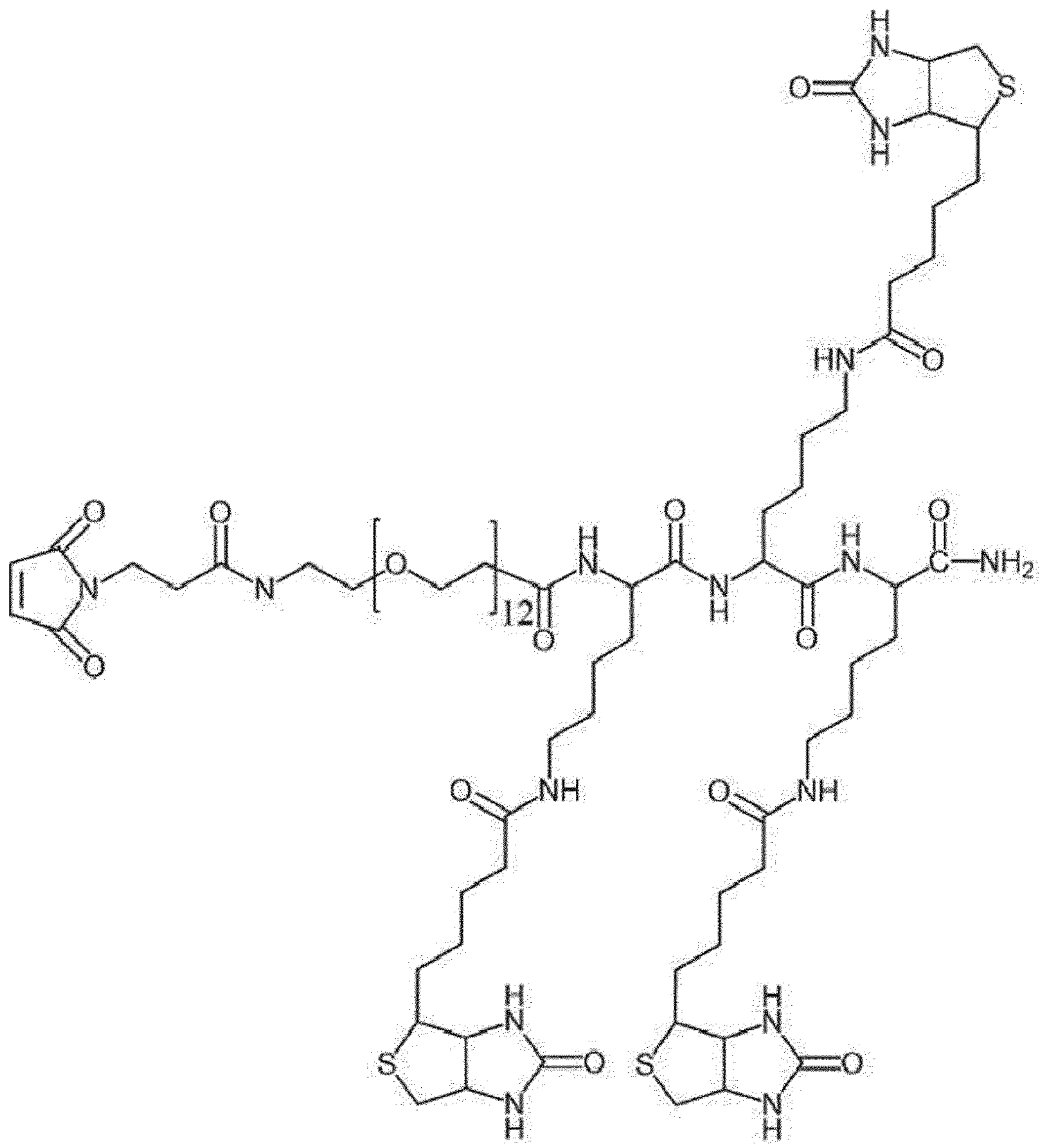

,

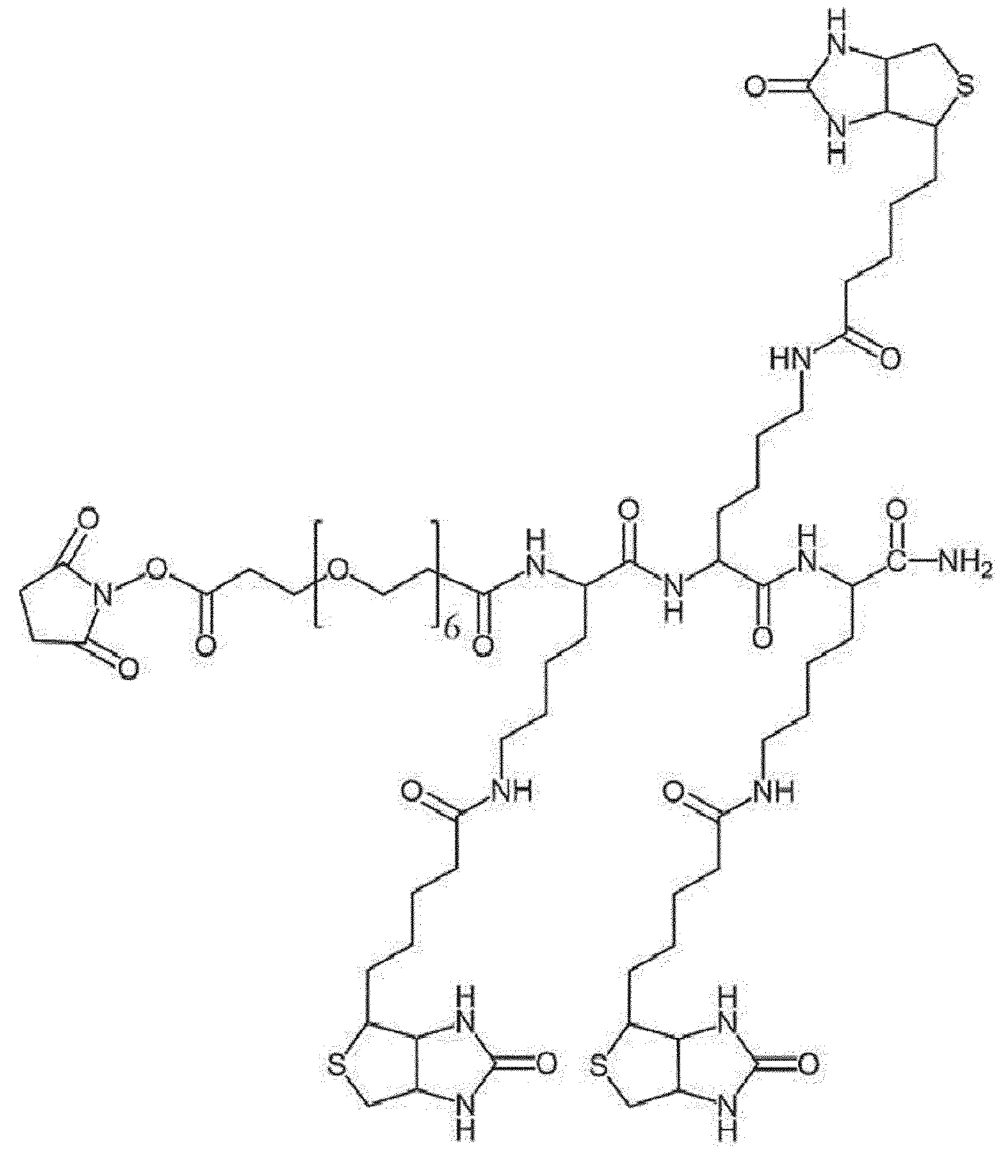

,

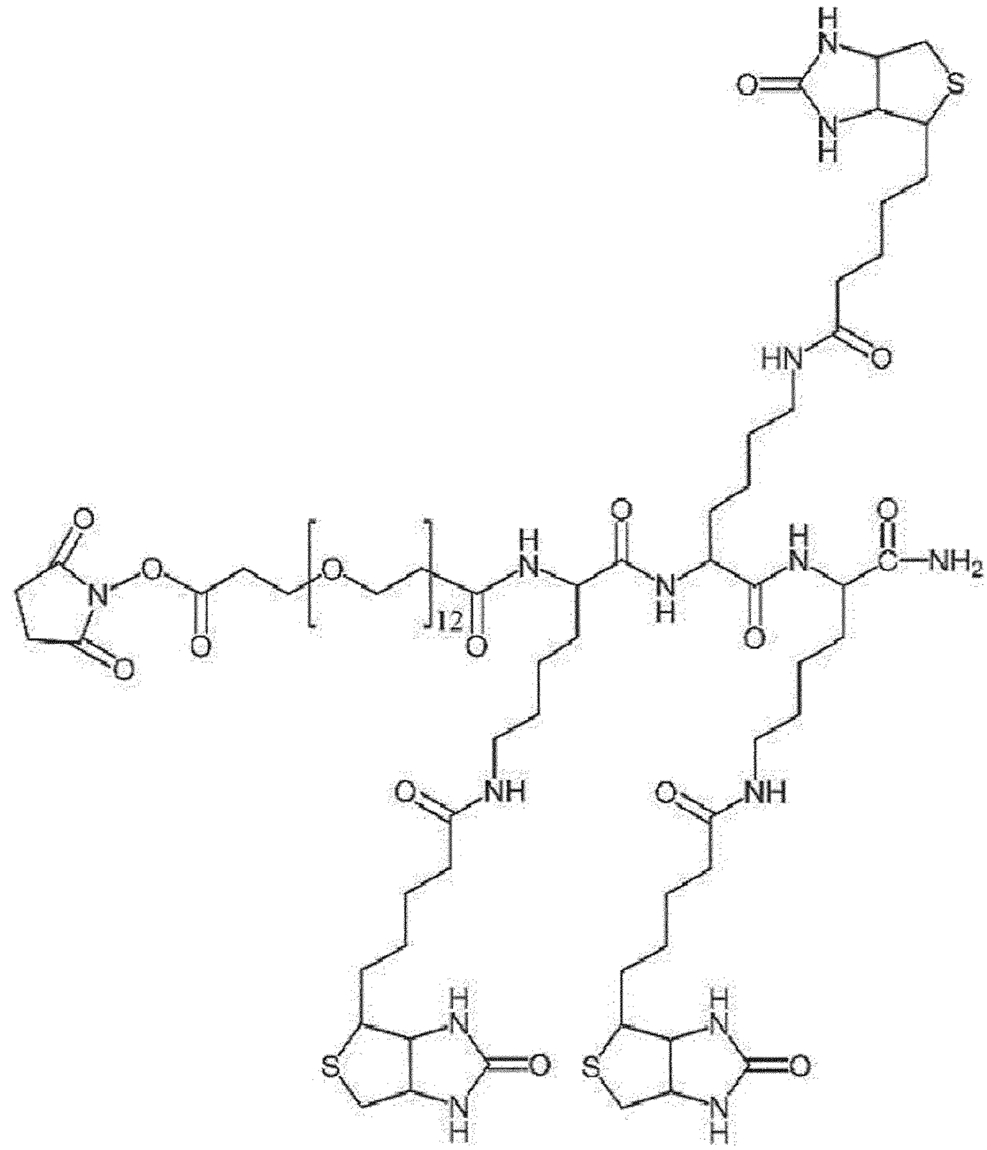

,

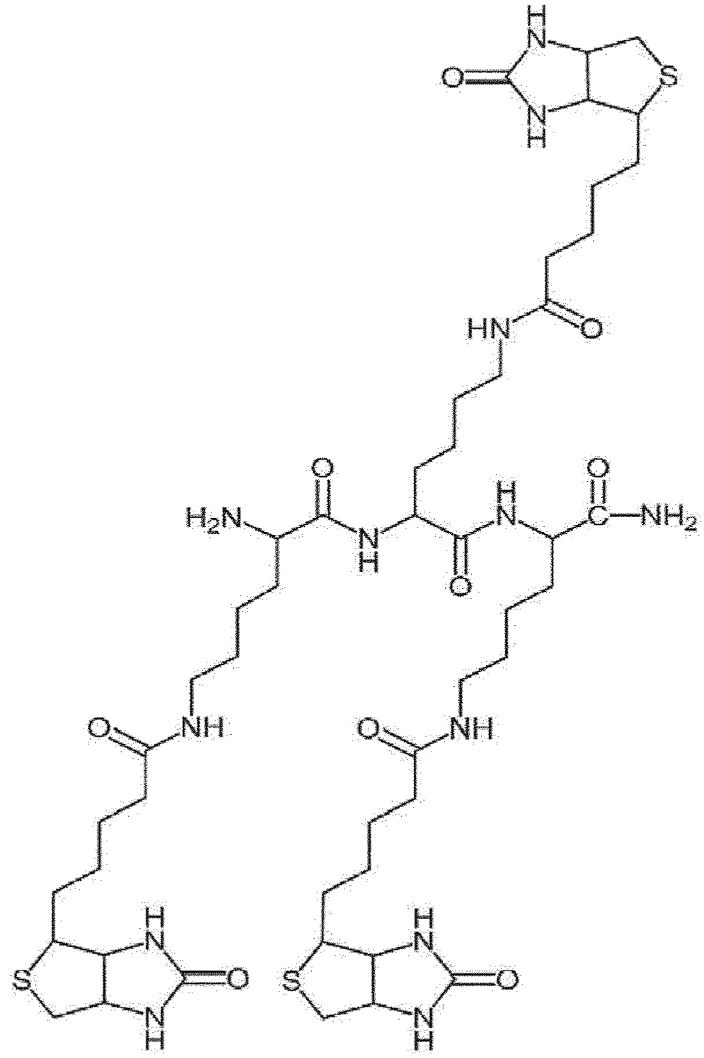

,

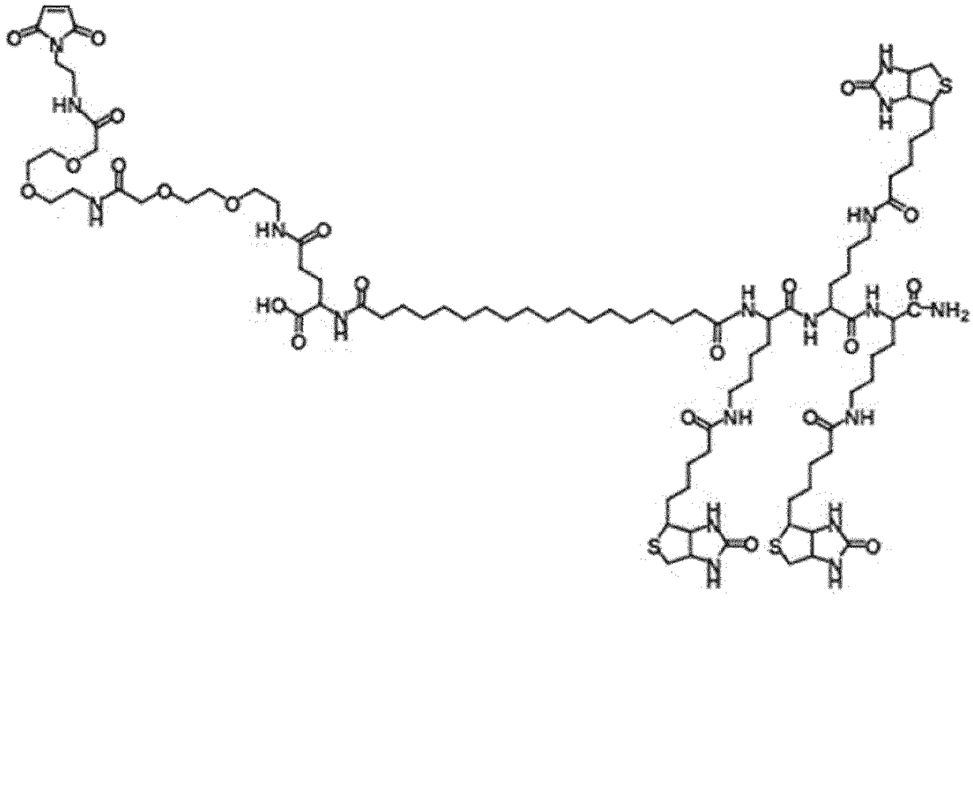

,

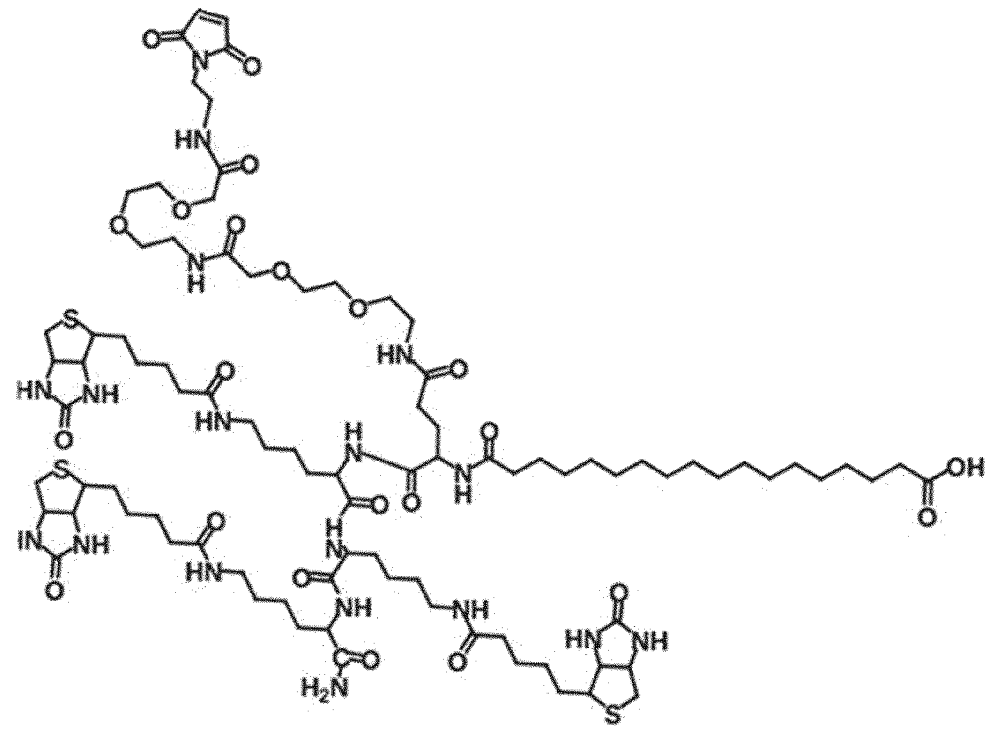

,

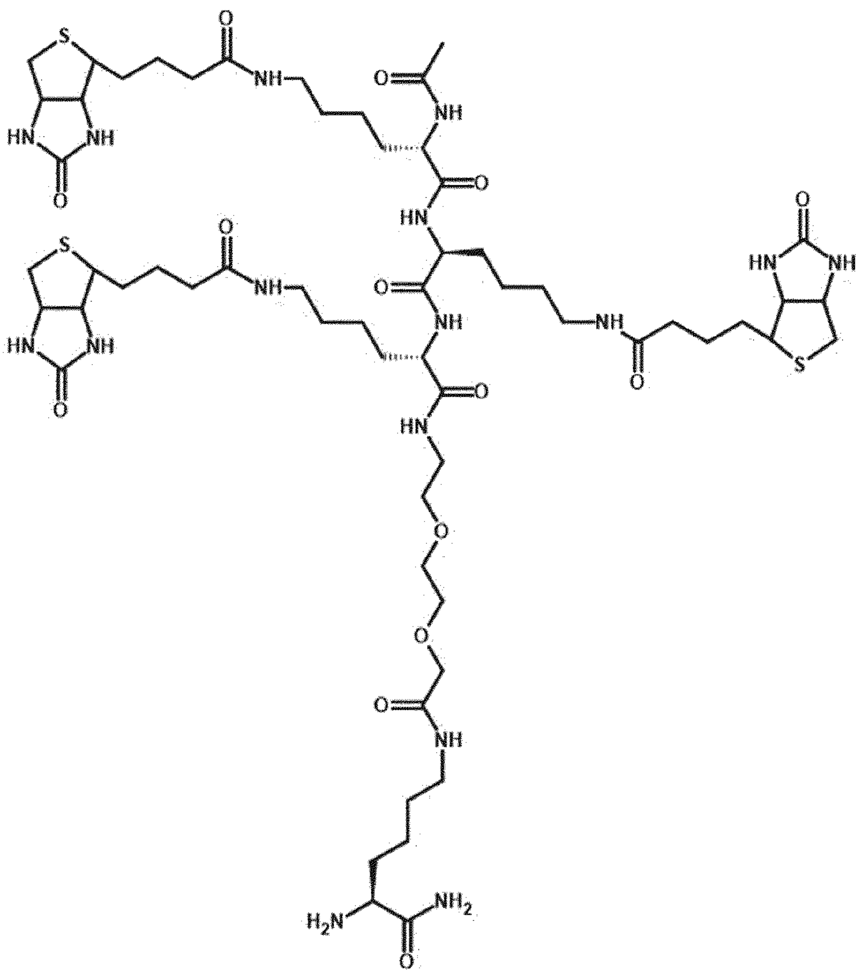

The oral pharmaceutical formulation selected from and

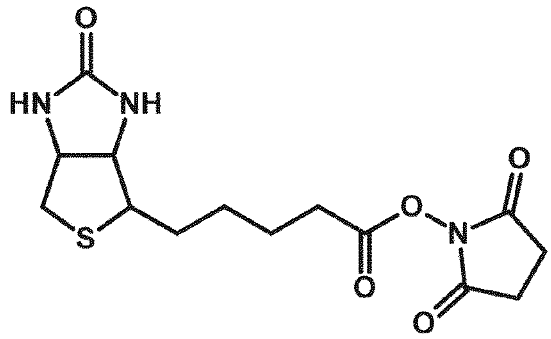

.

**13.** The oral pharmaceutical formulation of Claim 1,

where, in the (i) physiologically active substance conjugate bound to a biotin moiety, a fatty acid moiety, or a combination thereof,
the fatty acid moiety is represented by General Formula B below:

[General Formula B] **X'-Y'-W**

where, in the above formula,

X' is a functional group capable of binding to a the physiologically active substance;
Y' is a spacer; and
W is a fatty acid.

**14.** The physiologically active substance conjugate of Claim 13,
wherein X' is selected from the group comprising maleimide, succinimide, N-hydroxysuccinimide, succinimidyl succinate, succinimidyl glutarate, succinimidyl methyl ester, succinimidyl pentyl ester, succinimidyl carbonate, p-nitrophenyl carbonate, aldehyde, amine, thiol, oxyamine, iodoacetamide, aminooxyl, hydrazide, hydroxy, propionate, pyridyl, alkyl halide, vinyl sulfone, carboxyl, hydrazide, halogen acetamide, $C_{2-5}$ alkynyl, $C_{6-20}$ aryldisulfide, $C_{5-20}$ heteroaryldisulfide, isocyanate, thioester, iminoester, tetrafluorophenyl ester, nitrophenyl carbonate, nitrophenyl and derivatives thereof.

**15.** The physiologically active substance conjugate of Claim 13,
wherein Y' is a direct bond, or the structure of Y includes at least one of the group comprising substituted or unsubstituted $C_{1-50}$ linear alkylene, substituted or unsubstituted $C_{1-50}$ non-linear alkylene, substituted or unsubstituted $C_{1-50}$ linear heteroalkylene, substituted or unsubstituted $C_{1-50}$ nonlinear heteroalkylene, substituted or unsubstituted $C_{1-50}$ arylene, substituted or unsubstituted $C_{1-50}$ heteroarylene, -O-, -C(O), -C(O)NR-, -C(O)O-, -S-, -NR- or -NOR-, wherein R is hydrogen, or unsubstituted $C_{1-50}$ alkyl, substituted or unsubstituted $C_{1-50}$ aryl, or an ethylene glycol repeatingunit ($-(CH_2CH_2O)_n-$, where n is an integer of at least 1 but not more than 20).

**16.** The physiologically active substance conjugate of Claim 13,
Wherein Y' comprises $-C(O)-(OCH_2CH_2)_u-NH-$ as a repeating unit, where u is an integer of 1 to 20.

**17.** The physiologically active substance conjugate of Claim 13,
wherein Y' comprises glutamic acid, glutamine, glycine, isoleucine, or lysine as a component.

**18.** The oral pharmaceutical formulation of Claim 1,

where, in the (i) physiologically active substance conjugate bound to a biotin moiety, a fatty acid moiety, or a combination thereof,
the fatty acid moiety is the oral pharmaceutical formulation selected from the group comprising:

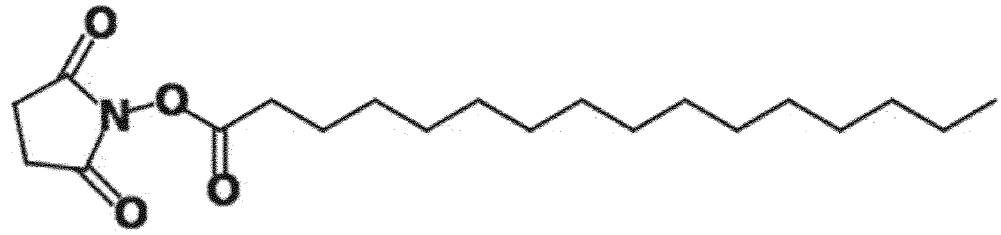

,

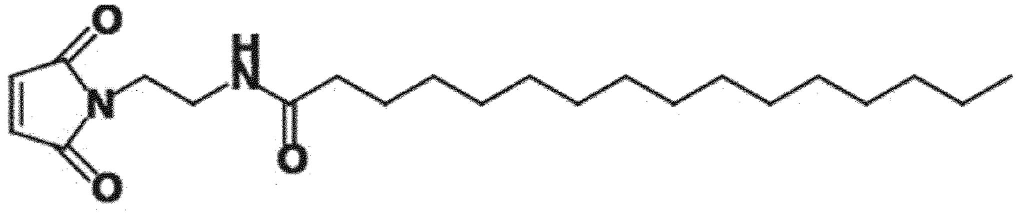

,

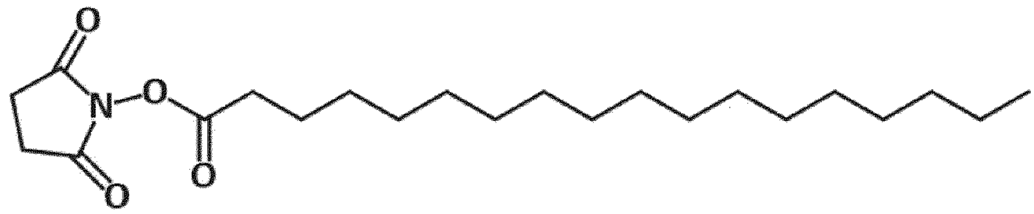

,

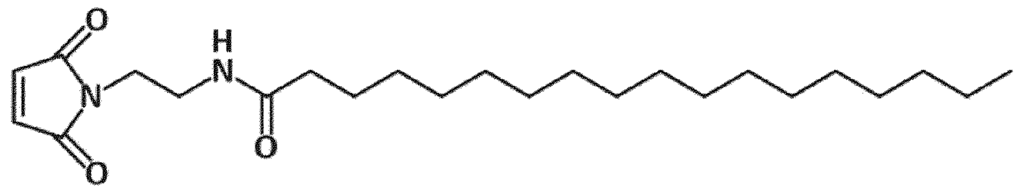

,

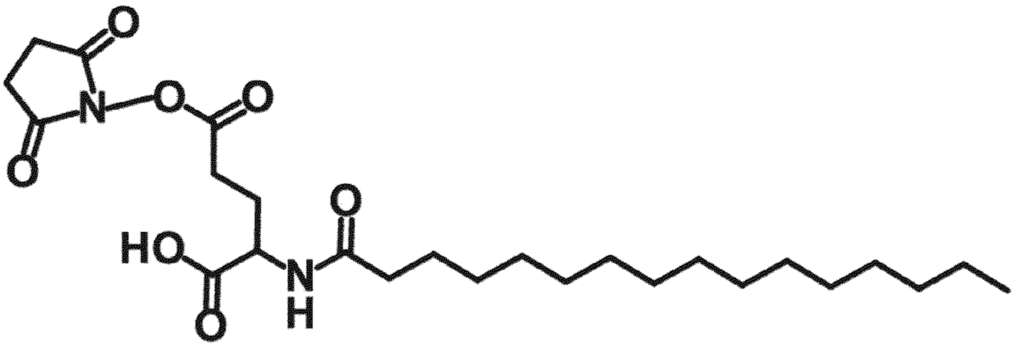

,

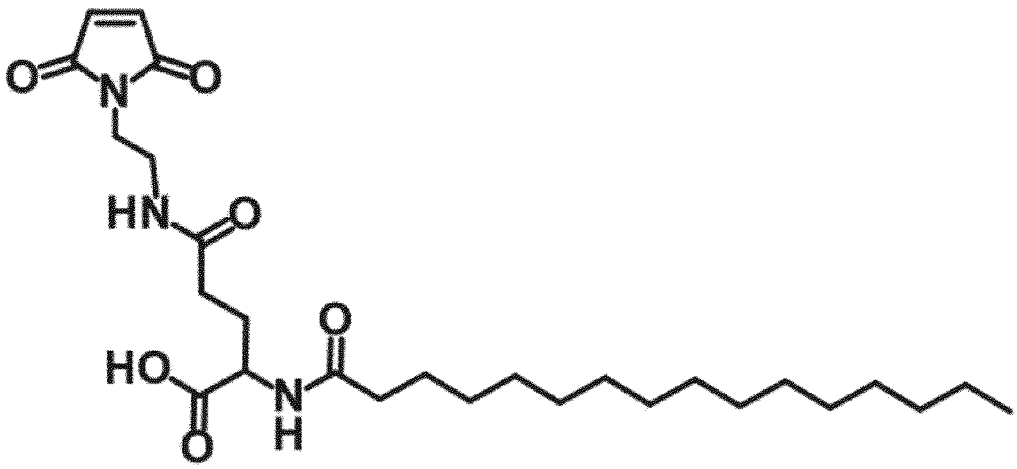

,

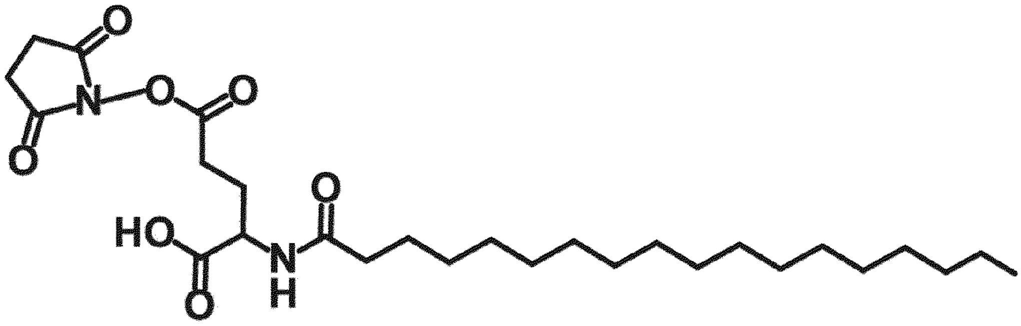

,

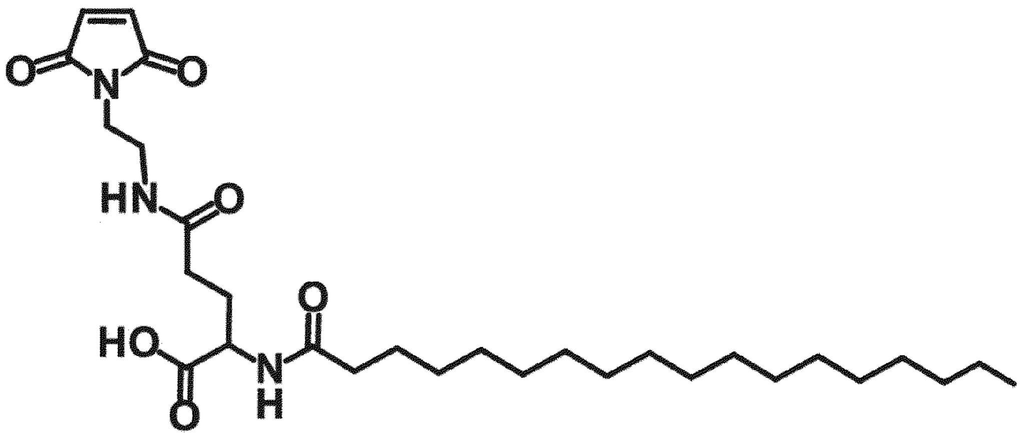

,

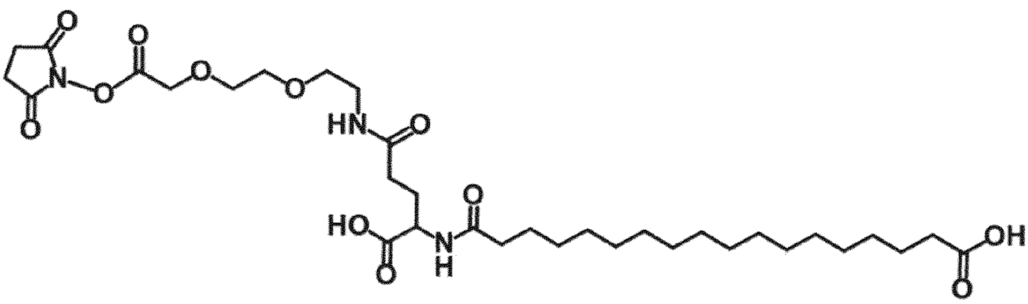

,

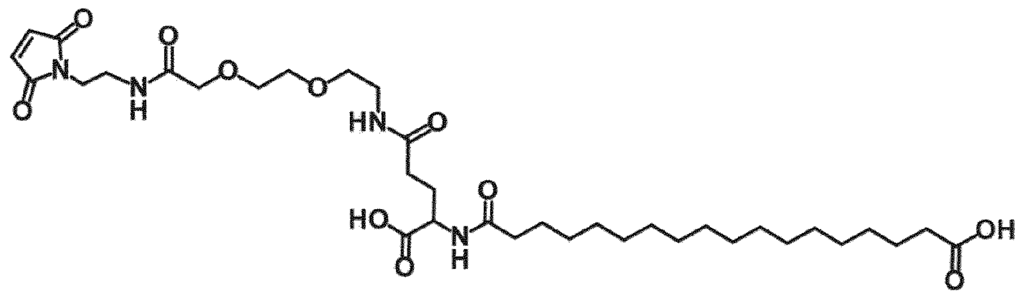

,

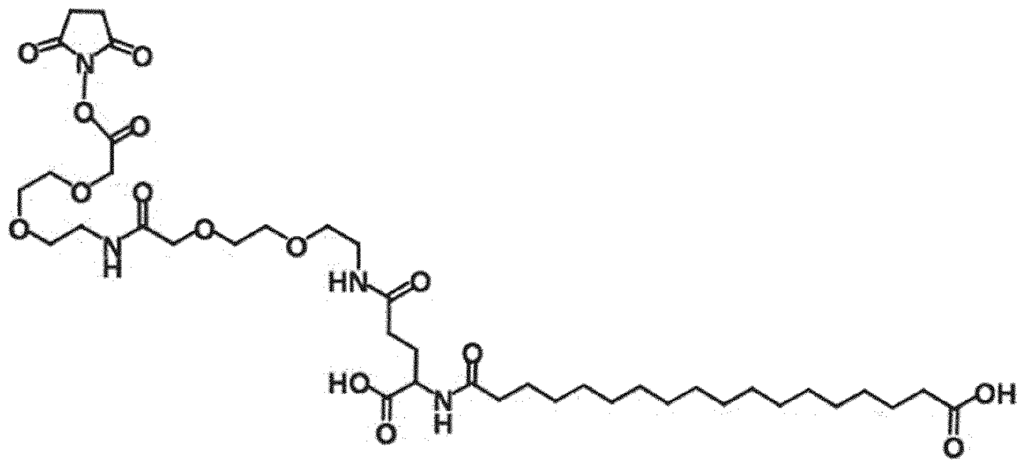

,

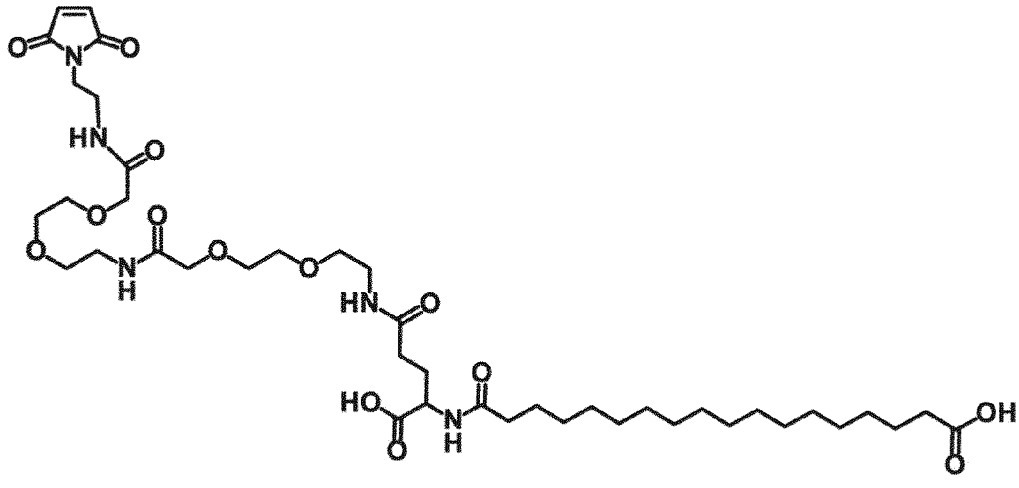

,

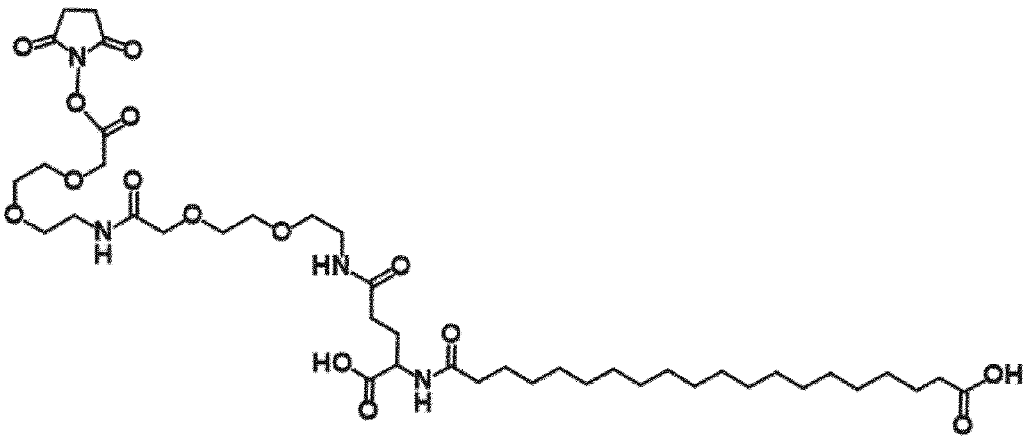

,

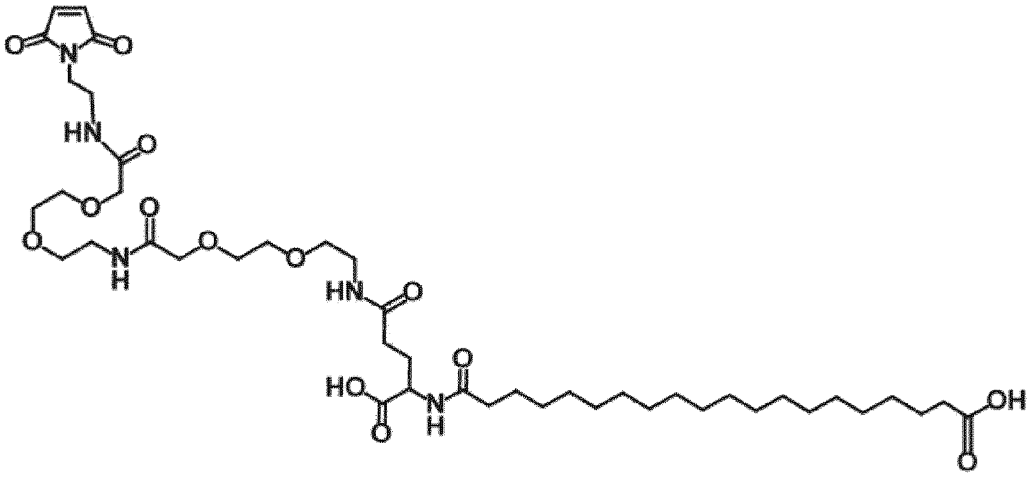

,

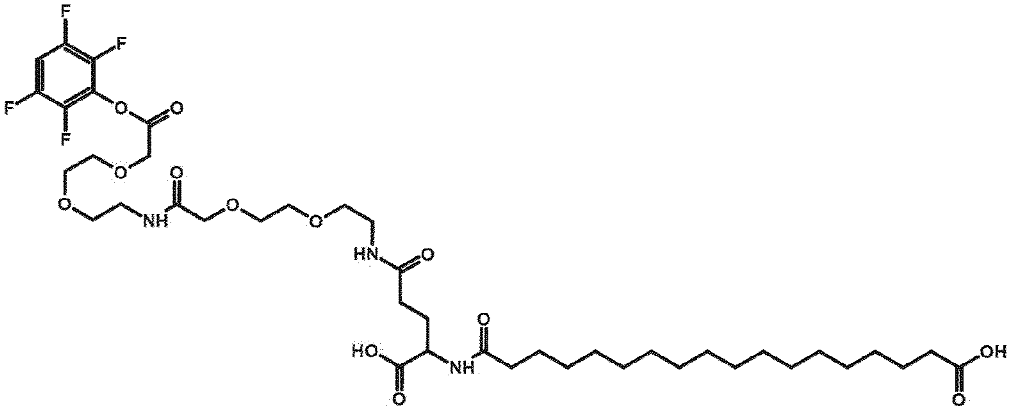

and

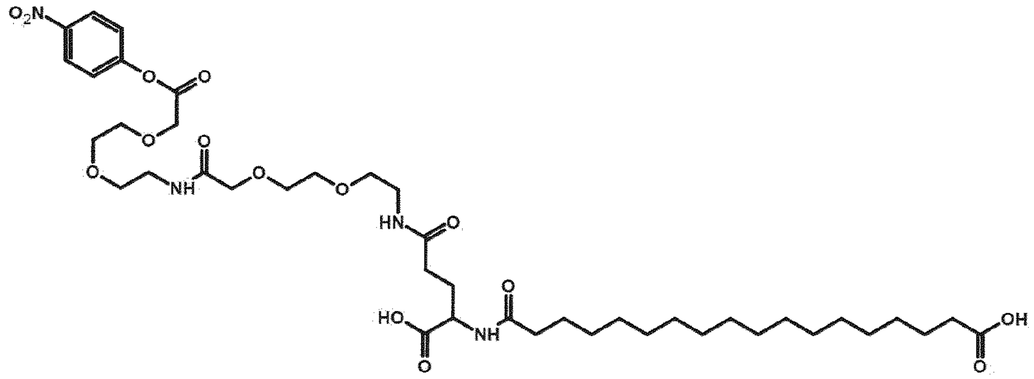

.

19. The oral pharmaceutical formulation of Claim 1, wherein the excipient comprises a bile acid, a derivative thereof, or a pharmaceutically acceptable salt thereof.

20. The oral pharmaceutical formulation of Claim 19, wherein the bile acid is at least one selected from the group comprising glycocholic acid, glycochenodeoxycholic acid, taurochenodeoxycholic acid, taurocholic acid, deoxycholic acid, cholic acid, chenodeoxycholic acid, ursodeoxycholic acid, and lithocholic acid.

21. The oral pharmaceutical formulation of Claim 19, wherein the bile acid is one selected from the group comprising chenodeoxycholic acid, deoxycholic acid, cholic acid, glycocholic acid, taurocholic acid and ursodioxycholic acid.

22. The oral pharmaceutical formulation of Claim 19, wherein the excipient further comprises at least one selected from the group comprising alpha-tocopherol, malic acid, fumaric acid, ascorbic acid, butylated hydroxyanisole, butylated hydroxy toluene, sodium phosphate, calcium phosphate, potassium phosphate, galactose, glucose, maltose, gallic acid, propyl gallate, and pharmaceutically acceptable salts thereof.

23. The oral pharmaceutical formulation of Claim 19, wherein the excipient further comprises gallic acid, propyl gallate or a pharmaceutically acceptable salt thereof.

24. The oral pharmaceutical formulation of Claim 1, wherein the weight ratio of (i) the physiologically active substance bound to the biotin moiety and (ii) the excipient is 1: 0.01 to 1000.

25. The oral pharmaceutical formulation of Claim 1, wherein the weight ratio of (i) the physiologically active substance bound to the biotin moiety and (ii) the excipient is 1: 0.1 to 500.

26. The oral pharmaceutical formulation of Claim 1,

wherein the excipient comprises gallic acid, propyl gallate or a pharmaceutically acceptable salt thereof, and the weight ratio of bile acid or a pharmaceutically acceptable salt thereof and propyl gallate or a pharmaceutically acceptable salt thereof is 1: 0.01 to 8.

27. The oral pharmaceutical formulation of Claim 1,

wherein the excipient comprises gallic acid, propyl gallate or a pharmaceutically acceptable salt thereof;
and [comprises] 1 to 1000mg bile acid or a pharmaceutically acceptable salt thereof;
and 1 to 1000mg propyl gallate or a pharmaceutically acceptable salt thereof.

[FIG. 1]

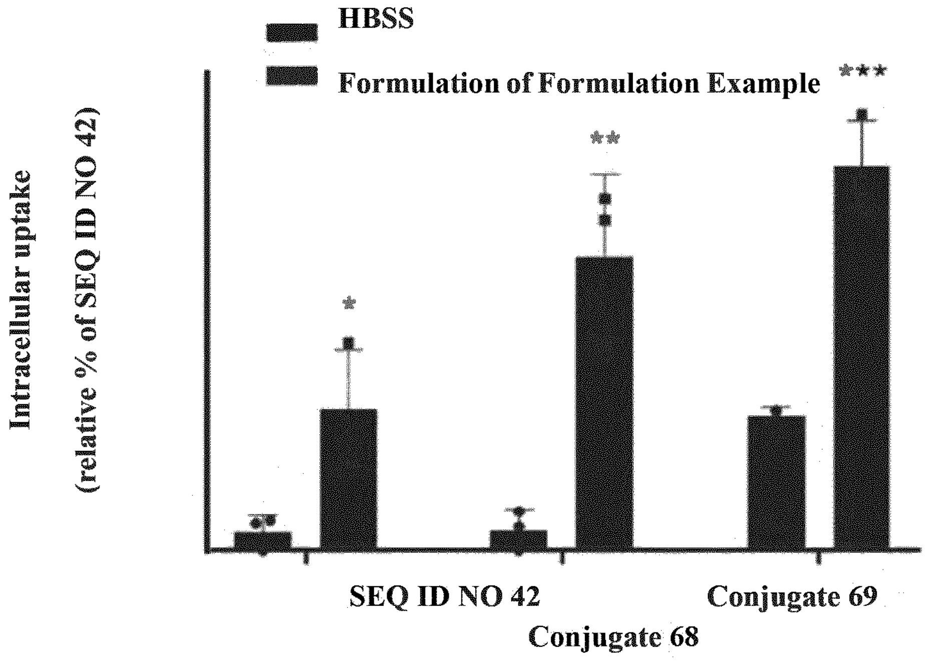
HBSS
Formulation of Formulation Example
Intracellular uptake
(relative % of SEQ ID NO 42)
*
**
***
SEQ ID NO 42
Conjugate 68
Conjugate 69

[FIG. 2]

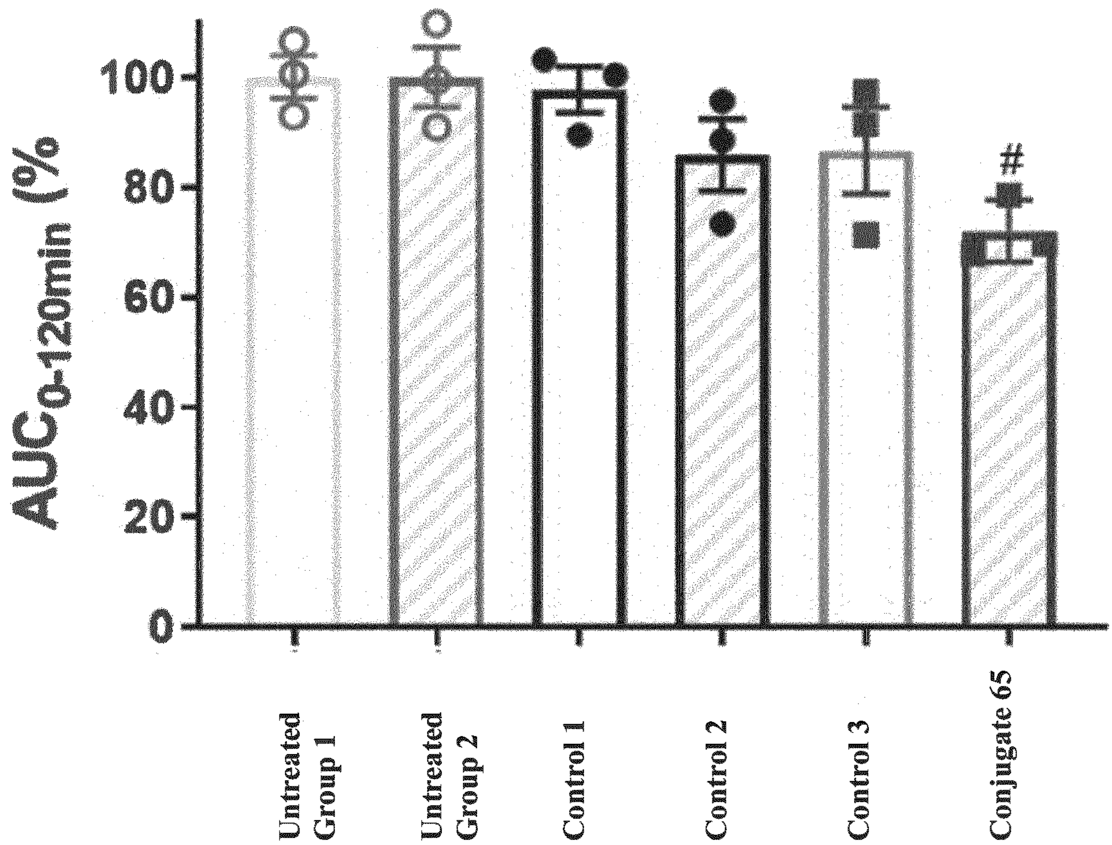
$AUC_{0-120min}$ (%
100
80
60
40
20
0
#
Untreated Group 1
Untreated Group 2
Control 1
Control 2
Control 3
Conjugate 65

[FIG. 3]

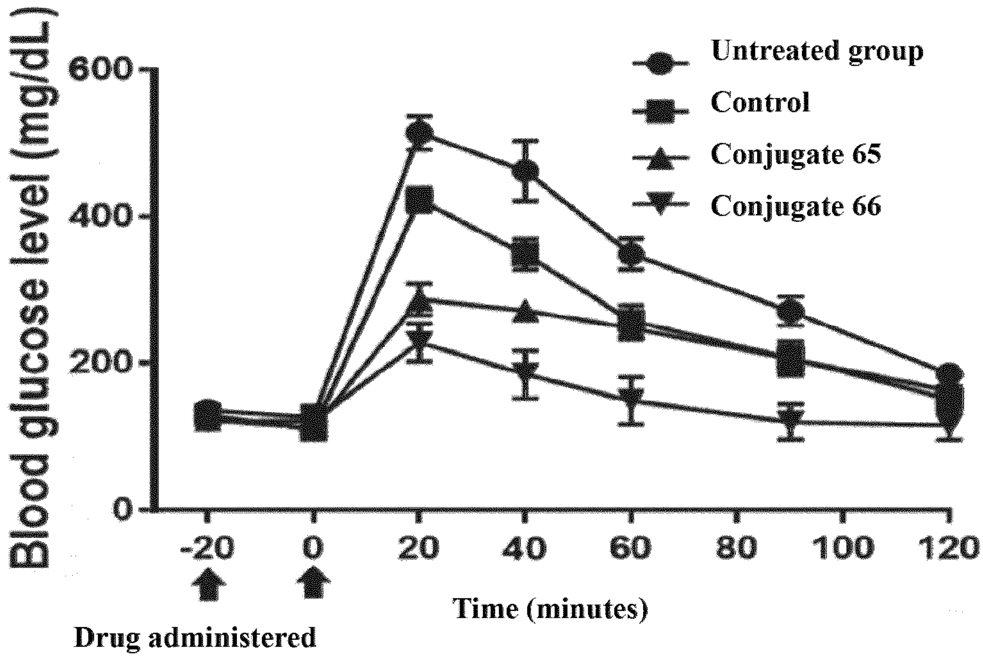


[FIG. 4]

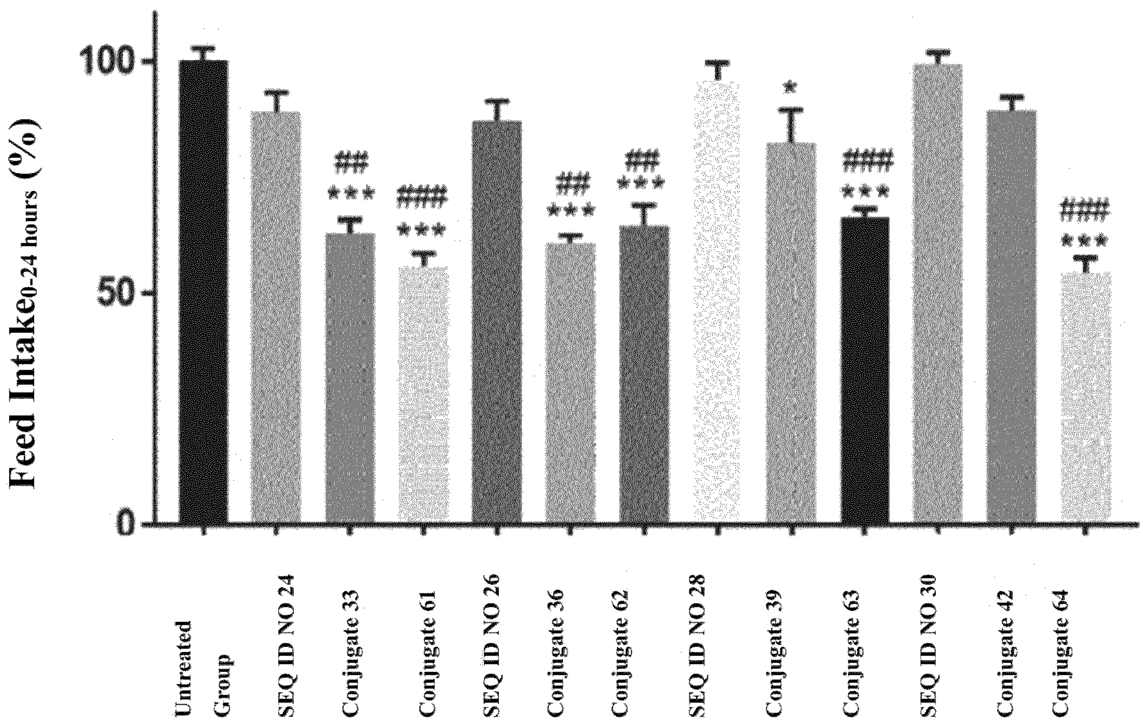


*p vs. untreated group, #p vs. cyclic peptide (SEQ ID NOs: 24, 26, 28, 30)

[FIG. 5]

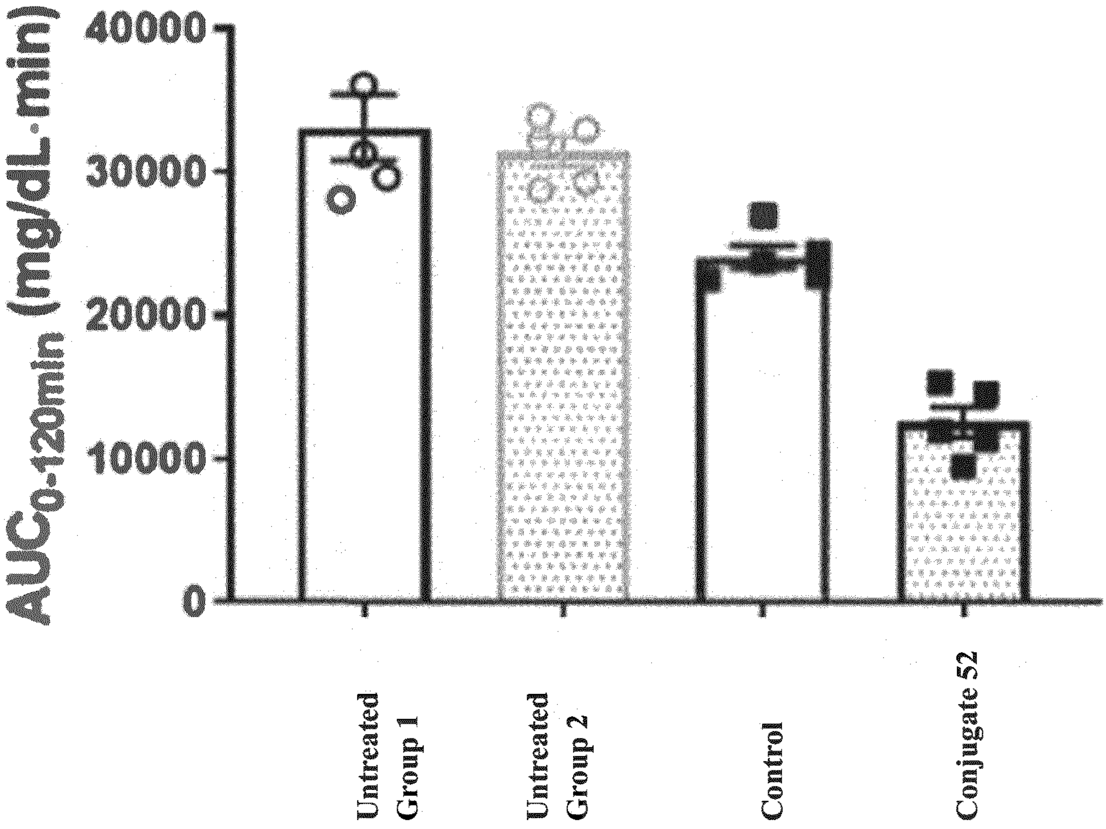
AUC0-120min (mg/dL·min)
40000
30000
20000
10000
0
Untreated Group 1
Untreated Group 2
Control
Conjugate 52

**INTERNATIONAL SEARCH REPORT**

International application No. **PCT/KR2021/017802**

**A. CLASSIFICATION OF SUBJECT MATTER**

**A61K 47/54**(2017.01)i; **A61K 47/28**(2006.01)i; **A61K 47/14**(2006.01)i; **A61K 38/16**(2006.01)i; **A61K 9/00**(2006.01)i; **A61P 3/10**(2006.01)i

According to International Patent Classification (IPC) or to both national classification and IPC

**B. FIELDS SEARCHED**

Minimum documentation searched (classification system followed by classification symbols)

A61K 47/54(2017.01); A61K 38/21(2006.01); A61K 38/28(2006.01); A61K 47/42(2006.01); A61K 47/68(2017.01); A61K 9/127(2006.01); C07K 14/605(2006.01); C07K 14/62(2006.01); C07K 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), PubChem, Google & keywords: 비오틴 모이어티 (biotin moiety), 지방산 모이어티 (fatty acid moiety), 생리활성 물질 (bioactive material), 부형제 (excipient)

**C. DOCUMENTS CONSIDERED TO BE RELEVANT**

| Category* | Citation of document, with indication, where appropriate, of the relevant passages | Relevant to claim No. |
|---|---|---|
| X<br>Y | KR 10-2010-0109896 A (SDG, INC.) 11 October 2010 (2010-10-11)<br>See paragraphs [0081], [0082], [0120], [0122], [0137], [0235], [0236] and [0238]; and claims 1-5, 9, 17 and 18. | 1,2,5-12,19,22-27<br>4 |
| Y | KR 10-2018-0039726 A (THE CALIFORNIA INSTITUTE FOR BIOMEDICAL RESEARCH) 18 April 2018 (2018-04-18)<br>See claims 1, 44, 45, 48 and 55. | 4 |
| X<br>Y | KR 10-2011-0004366 A (NOVO NORDISK A/S) 13 January 2011 (2011-01-13)<br>See abstract; paragraphs [0288], [0303], [0358], [0359], [0366], [0403], [0730] and [0731]; and claims 1, 9 and 10. | 1,2,13-16,18-27<br>4 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 March 2022** | **18 March 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2021/017802**

**C. DOCUMENTS CONSIDERED TO BE RELEVANT**

| Category* | Citation of document, with indication, where appropriate, of the relevant passages | Relevant to claim No. |
|---|---|---|
| X | US 2003-0199451 A1 (MOGENSEN, J. P. et al.) 23 October 2003 (2003-10-23) See paragraphs [0037], [0783], [0870], [0883], [1058] and [1162]; and claims 1, 69, 70 and 72. | 1,2,13-15,17-27 |
| Y | | 3 |
| Y | KR 10-2008-0064840 A (BIOCOMPATIBLES UK LIMITED) 09 July 2008 (2008-07-09) See claims 1, 17, 18, 20, 21 and 35. | 3 |
| X | KR 10-2008-0042045 A (SDG, INC.) 14 May 2008 (2008-05-14) See paragraphs [0131], [0155], [0251]-[0253], [0255] and [0273]; and claims 1, 9-11, 38 and 45. | 1,2,5-12,19,22-27 |

Form PCT/ISA/210 (second sheet) (July 2019)

**INTERNATIONAL SEARCH REPORT**

International application No.
**PCT/KR2021/017802**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
**Information on patent family members**

International application No.
**PCT/KR2021/017802**

| Patent document cited in search report | Publication date (day/month/year) | Patent family member(s) | Publication date (day/month/year) |
|---|---|---|---|
| KR 10-2010-0109896 A | 11 October 2010 | AU 2008-304269 A1 | 02 April 2009 |
| | | AU 2008-304269 B2 | 09 July 2015 |
| | | AU 2011-230564 A1 | 11 October 2012 |
| | | AU 2011-230564 B2 | 09 July 2015 |
| | | AU 2015-238887 A1 | 29 October 2015 |
| | | AU 2015-238887 B2 | 06 July 2017 |
| | | AU 2015-238890 A1 | 29 October 2015 |
| | | AU 2015-238890 B2 | 03 August 2017 |
| | | AU 2017-239513 A1 | 26 October 2017 |
| | | BR PI0817478 A2 | 08 September 2015 |
| | | CA 2704712 A1 | 02 April 2009 |
| | | CA 2704712 C | 31 May 2016 |
| | | CA 2794540 A1 | 29 September 2011 |
| | | CA 2794540 C | 04 December 2018 |
| | | CN 101917971 A | 15 December 2010 |
| | | CN 104666246 A | 03 June 2015 |
| | | EP 2205217 A1 | 14 July 2010 |
| | | EP 2205217 A4 | 23 January 2013 |
| | | EP 2205217 B1 | 13 December 2017 |
| | | EP 2410991 A1 | 01 February 2012 |
| | | EP 2410991 A4 | 07 October 2015 |
| | | EP 2552202 A1 | 06 February 2013 |
| | | EP 2552202 A4 | 17 February 2016 |
| | | EP 3360540 A1 | 15 August 2018 |
| | | ES 2661325 T3 | 28 March 2018 |
| | | HK 1211205 A1 | 20 May 2016 |
| | | HU E037002 T2 | 28 August 2018 |
| | | JP 2010-540559 A | 24 December 2010 |
| | | JP 2015-044883 A | 12 March 2015 |
| | | JP 6066982 B2 | 25 January 2017 |
| | | KR 10-1747433 B1 | 14 June 2017 |
| | | KR 10-2016-0005130 A | 13 January 2016 |
| | | LT 2205217 T | 26 March 2018 |
| | | MX 2010003396 A | 12 November 2010 |
| | | MX 337138 B | 02 February 2016 |
| | | NO 2205217 T3 | 12 May 2018 |
| | | PL 2205217 T3 | 30 May 2018 |
| | | PT 2205217 T | 15 March 2018 |
| | | US 10568835 B2 | 25 February 2020 |
| | | US 10751418 B2 | 25 August 2020 |
| | | US 2009-0087479 A1 | 02 April 2009 |
| | | US 2010-0080773 A1 | 01 April 2010 |
| | | US 2010-0247625 A1 | 30 September 2010 |
| | | US 2010-0310599 A1 | 09 December 2010 |
| | | US 2013-0183270 A1 | 18 July 2013 |
| | | US 2014-0243430 A1 | 28 August 2014 |
| | | US 2015-0125518 A1 | 07 May 2015 |
| | | US 2020-0405638 A1 | 31 December 2020 |
| | | US 8846053 B2 | 30 September 2014 |
| | | US 8962015 B2 | 24 February 2015 |

Form PCT/ISA/210 (patent family annex) (July 2019)

**INTERNATIONAL SEARCH REPORT**
**Information on patent family members**

International application No.
**PCT/KR2021/017802**

| Patent document cited in search report | Publication date (day/month/year) | Patent family member(s) | Publication date (day/month/year) |
|---|---|---|---|
| | | US 9145453 B2 | 29 September 2015 |
| | | US 9943602 B2 | 17 April 2018 |
| | | WO 2009-042945 A1 | 02 April 2009 |
| | | WO 2010-111271 A1 | 30 September 2010 |
| | | WO 2011-119953 A1 | 29 September 2011 |
| | | ZA 201002177 B | 29 June 2011 |
| KR 10-2018-0039726 A | 18 April 2018 | AU 2018-315889 A1 | 22 March 2018 |
| | | CA 2996716 A1 | 09 March 2017 |
| | | CN 108348581 A | 31 July 2018 |
| | | EP 3344278 A2 | 11 July 2018 |
| | | EP 3344278 A4 | 23 January 2019 |
| | | HK 1258125 A1 | 08 November 2019 |
| | | JP 2018-537399 A | 20 December 2018 |
| | | JP 6951825 B2 | 20 October 2021 |
| | | US 10501546 B2 | 10 December 2019 |
| | | US 2019-0023794 A1 | 24 January 2019 |
| | | US 2020-0115458 A1 | 16 April 2020 |
| | | WO 2017-041001 A2 | 09 March 2017 |
| | | WO 2017-041001 A3 | 04 May 2017 |
| KR 10-2011-0004366 A | 13 January 2011 | AU 2009-226910 A1 | 24 September 2009 |
| | | AU 2009-226910 B2 | 06 February 2014 |
| | | BR PI200910348 A2 | 11 August 2020 |
| | | BR PI200910348 B1 | 29 June 2021 |
| | | CA 2009738 A1 | 24 September 2009 |
| | | CA 2718738 C | 07 May 2019 |
| | | CN 102037008 A | 27 April 2011 |
| | | CN 102037008 B | 31 August 2016 |
| | | CN 102227213 A | 26 October 2011 |
| | | CN 102245633 A | 16 November 2011 |
| | | DK 2254906 T3 | 23 January 2017 |
| | | DK 2910570 T3 | 30 January 2017 |
| | | EP 2254906 A1 | 01 December 2010 |
| | | EP 2254906 B1 | 05 October 2016 |
| | | EP 2370059 A1 | 05 October 2011 |
| | | EP 2376531 A1 | 19 October 2011 |
| | | EP 2910569 A1 | 26 August 2015 |
| | | EP 2910569 B1 | 05 October 2016 |
| | | EP 2910570 A1 | 26 August 2015 |
| | | EP 2910570 B1 | 12 October 2016 |
| | | EP 2910571 A1 | 26 August 2015 |
| | | EP 2910571 B1 | 05 October 2016 |
| | | ES 2609288 T3 | 19 April 2017 |
| | | ES 2611007 T3 | 04 May 2017 |
| | | HU E032284 T2 | 28 September 2017 |
| | | HU E032287 T2 | 28 September 2017 |
| | | IL 250548 A | 31 March 2019 |
| | | IL 250549 A | 31 December 2018 |
| | | JP 2011-515358 A | 19 May 2011 |
| | | JP 2012-510438 A | 10 May 2012 |
| | | JP 2012-511506 A | 24 May 2012 |

Form PCT/ISA/210 (patent family annex) (July 2019)

**INTERNATIONAL SEARCH REPORT**
**Information on patent family members**

International application No.
**PCT/KR2021/017802**

| Patent document cited in search report | Publication date (day/month/year) | Patent family member(s) | Publication date (day/month/year) |
|---|---|---|---|
| | | JP 2015-147781 A | 20 August 2015 |
| | | JP 2015-147782 A | 20 August 2015 |
| | | JP 2015-155421 A | 27 August 2015 |
| | | JP 5749155 B2 | 15 July 2015 |
| | | KR 10-1755434 B1 | 10 July 2017 |
| | | KR 10-1755529 B1 | 07 July 2017 |
| | | KR 10-2016-0073431 A | 24 June 2016 |
| | | KR 10-2016-0124929 A | 28 October 2016 |
| | | MX 2010009850 A | 30 September 2010 |
| | | PL 2254906 T3 | 28 April 2017 |
| | | PL 2910570 T3 | 30 June 2017 |
| | | PT 2254906 T | 03 January 2017 |
| | | PT 2910570 T | 24 January 2017 |
| | | RU 2010141481 A | 27 April 2012 |
| | | RU 2571857 C2 | 20 December 2015 |
| | | US 10259856 B2 | 16 April 2019 |
| | | US 2011-0105720 A1 | 05 May 2011 |
| | | US 2011-0293714 A1 | 01 December 2011 |
| | | US 2011-0294729 A1 | 01 December 2011 |
| | | US 2014-0073564 A1 | 13 March 2014 |
| | | US 2015-0210747 A1 | 30 July 2015 |
| | | US 2015-0210748 A1 | 30 July 2015 |
| | | US 2019-0112348 A1 | 18 April 2019 |
| | | US 8691759 B2 | 08 April 2014 |
| | | US 9045560 B2 | 02 June 2015 |
| | | US 9688737 B2 | 27 June 2017 |
| | | WO 2009-115469 A1 | 24 September 2009 |
| | | WO 2010-060667 A1 | 03 June 2010 |
| | | WO 2010-066636 A1 | 17 June 2010 |
| | | ZA 201006126 B | 30 November 2011 |
| US 2003-0199451 A1 | 23 October 2003 | AU 2831801 A | 07 August 2001 |
| | | BR 0107902 A | 05 November 2002 |
| | | CA 2396372 A1 | 02 August 2001 |
| | | CN 1396904 A | 12 February 2003 |
| | | EP 1254102 A1 | 06 November 2002 |
| | | HU 0204247 A2 | 28 April 2003 |
| | | HU 0204247 A3 | 28 October 2003 |
| | | JP 2003-520839 A | 08 July 2003 |
| | | KR 10-2002-0090211 A | 30 November 2002 |
| | | MX PA02007295 A | 29 November 2002 |
| | | NO 20023567 L | 25 September 2002 |
| | | PL 357017 A1 | 12 July 2004 |
| | | RU 2002123050 A | 10 January 2004 |
| | | US 2001-0041709 A1 | 15 November 2001 |
| | | US 6569901 B2 | 27 May 2003 |
| | | US 7202213 B2 | 10 April 2007 |
| | | WO 01-55086 A1 | 02 August 2001 |
| | | ZA 200204800 B | 26 March 2003 |
| KR 10-2008-0064840 A | 09 July 2008 | AT 448247 T | 15 November 2009 |
| | | AU 2006-299134 A1 | 12 April 2007 |

Form PCT/ISA/210 (patent family annex) (July 2019)

**INTERNATIONAL SEARCH REPORT**
**Information on patent family members**

International application No.
**PCT/KR2021/017802**

| Patent document cited in search report | Publication date (day/month/year) | Patent family member(s) | Publication date (day/month/year) |
|---|---|---|---|
| | | AU 2006-299134 B2 | 23 February 2012 |
| | | BR PI0616107 A2 | 07 June 2011 |
| | | CA 2619053 A1 | 12 April 2007 |
| | | CN 101273058 A | 24 September 2008 |
| | | CY 1109778 T1 | 10 September 2014 |
| | | DK 1767545 T3 | 15 March 2010 |
| | | EA 013796 B1 | 30 June 2010 |
| | | EA 200800699 A1 | 30 October 2008 |
| | | EP 1767545 A1 | 28 March 2007 |
| | | EP 1767545 B1 | 11 November 2009 |
| | | EP 1926748 A1 | 04 June 2008 |
| | | EP 1926748 B1 | 29 August 2012 |
| | | EP 2045265 A1 | 08 April 2009 |
| | | EP 2045265 B1 | 21 November 2012 |
| | | EP 2174952 A2 | 14 April 2010 |
| | | EP 2174952 A3 | 17 November 2010 |
| | | EP 2261245 A1 | 15 December 2010 |
| | | ES 2336575 T3 | 14 April 2010 |
| | | ES 2394218 T3 | 23 January 2013 |
| | | ES 2397289 T3 | 06 March 2013 |
| | | HR P20100062 T1 | 31 March 2010 |
| | | JP 2009-508505 A | 05 March 2009 |
| | | JP 2013-099352 A | 23 May 2013 |
| | | JP 5222729 B2 | 26 June 2013 |
| | | PL 1767545 T3 | 30 April 2010 |
| | | PT 1767545 E | 05 February 2010 |
| | | RS 51319 B | 31 December 2010 |
| | | SG 165414 A1 | 28 October 2010 |
| | | SI 1767545 T1 | 31 March 2010 |
| | | US 2011-0130329 A1 | 02 June 2011 |
| | | US 2012-0238497 A1 | 20 September 2012 |
| | | US 8431533 B2 | 30 April 2013 |
| | | US 8853159 B2 | 07 October 2014 |
| | | WO 2007-039140 A1 | 12 April 2007 |
| | | ZA 200803488 B | 28 October 2009 |
| KR 10-2008-0042045 A | 14 May 2008 | AU 2006-249479 A1 | 30 November 2006 |
| | | AU 2006-249480 A1 | 30 November 2006 |
| | | AU 3999699 A | 06 December 1999 |
| | | CA 2609376 A1 | 30 November 2006 |
| | | CA 2609402 A1 | 30 November 2006 |
| | | CA 2609402 C | 21 October 2014 |
| | | CA 2864366 A1 | 30 November 2006 |
| | | CA 2864366 C | 05 April 2016 |
| | | CN 101237854 A | 06 August 2008 |
| | | CN 101237854 B | 27 March 2013 |
| | | CN 101237855 A | 06 August 2008 |
| | | DK 1883394 T3 | 25 June 2018 |
| | | DK 3391876 T3 | 14 June 2021 |
| | | EP 1087753 A1 | 04 April 2001 |
| | | EP 1087753 A4 | 30 June 2004 |

Form PCT/ISA/210 (patent family annex) (July 2019)

**INTERNATIONAL SEARCH REPORT**
**Information on patent family members**

International application No.
**PCT/KR2021/017802**

| Patent document cited in search report | Publication date (day/month/year) | Patent family member(s) | Publication date (day/month/year) |
|---|---|---|---|
| | | EP 1883394 A2 | 06 February 2008 |
| | | EP 1883394 A4 | 21 August 2013 |
| | | EP 1883394 B1 | 28 March 2018 |
| | | EP 1883395 A2 | 06 February 2008 |
| | | EP 1883395 A4 | 04 July 2012 |
| | | EP 3391876 A1 | 24 October 2018 |
| | | EP 3391876 B1 | 10 March 2021 |
| | | ES 2675042 T3 | 05 July 2018 |
| | | HU E039023 T2 | 28 December 2018 |
| | | JP 2002-515417 A | 28 May 2002 |
| | | JP 2008-542270 A | 27 November 2008 |
| | | JP 2009-507761 A | 26 February 2009 |
| | | JP 5414270 B2 | 12 February 2014 |
| | | KR 10-1389226 B1 | 25 April 2014 |
| | | KR 10-2008-0043742 A | 19 May 2008 |
| | | LT 1883394 T | 11 June 2018 |
| | | PL 1883394 T3 | 28 September 2018 |
| | | PT 1883394 T | 06 June 2018 |
| | | US 10004686 B2 | 26 June 2018 |
| | | US 10463616 B2 | 05 November 2019 |
| | | US 2006-0222697 A1 | 05 October 2006 |
| | | US 2006-0222698 A1 | 05 October 2006 |
| | | US 2007-0104777 A1 | 10 May 2007 |
| | | US 2007-0218117 A1 | 20 September 2007 |
| | | US 2010-0129428 A1 | 27 May 2010 |
| | | US 2010-0209492 A1 | 19 August 2010 |
| | | US 2011-0135725 A1 | 09 June 2011 |
| | | US 2011-0135727 A1 | 09 June 2011 |
| | | US 2013-0267462 A1 | 10 October 2013 |
| | | US 2015-0031608 A1 | 29 January 2015 |
| | | US 2019-0054021 A1 | 21 February 2019 |
| | | US 2020-0261363 A1 | 20 August 2020 |
| | | US 7169410 B1 | 30 January 2007 |
| | | US 7858116 B2 | 28 December 2010 |
| | | US 7871641 B2 | 18 January 2011 |
| | | US 8257735 B2 | 04 September 2012 |
| | | US 8303983 B2 | 06 November 2012 |
| | | US 9034372 B2 | 19 May 2015 |
| | | WO 2006-127360 A2 | 30 November 2006 |
| | | WO 2006-127360 A3 | 10 January 2008 |
| | | WO 2006-127361 A2 | 30 November 2006 |
| | | WO 2006-127361 A3 | 24 May 2007 |
| | | WO 99-59545 A1 | 25 November 1999 |
| | | WO 99-59545 A9 | 27 April 2000 |
| | | ZA 200710055 B | 25 March 2009 |
| | | ZA 200710056 B | 25 March 2009 |

Form PCT/ISA/210 (patent family annex) (July 2019)